# EUROPEAN PATENT SPECIFICATION

(11) **EP 3 110 970 B1**
(45) Date of publication and mention of the grant of the patent: **02.12.2020**
(21) Application number: 15708041.7
(22) Date of filing: 27.02.2015
(51) Int. Cl.: G01N 33/48, G01N 33/53, C12Q 1/6886

(54) **BIOMARKERS FOR ENDOMETRIOSIS**
BIOMARKER FÜR ENDOMETRIOSE
MARQUEURS BIOLOGIQUES DE L'ENDOMÉTRIOSE

(30) Priority: 27.02.2014 GB 201403489
(43) Date of publication of application: 04.01.2017
(73) Proprietor: Queen Mary University of London, London E1 4NS (GB)
(72) Inventor: COTTER, Finbar, London EC1 2AD (GB); DEGUARA, Christine, London E1 2AD (GB); DAVIS, Colin, London E1 2AD (GB)
(74) Representative: Bassil, Nicholas Charles
(86) International application number: PCT/GB2015/050584
(87) International publication number: WO 2015/128671

(56) References cited:
- EP-A1- 2 405 266
- EP-A1- 2 857 521
- WO-A1-2010/021843
- WO-A1-2012/013955
- WO-A1-2012/142330
- WEN-TAO WANG ET AL: "Circulating MicroRNAs Identified in a Genome-Wide Serum MicroRNA Expression Analysis as Noninvasive Biomarkers for Endometriosis", JOURNAL OF CLINICAL ENDOCRINOLOGY & METABOLISM, vol. 98, no. 1, 1 January 2013 (2013-01-01), pages 281-289, XP055127854, ISSN: 0021-972X, DOI: 10.1210/jc.2012-2415
- PIOTR LAUDANSKI ET AL: "MicroRNAs expression profiling of eutopic proliferative endometrium in women with ovarian endometriosis", REPRODUCTIVE BIOLOGY AND ENDOCRINOLOGY, BIOMED CENTRAL LTD, GB, vol. 11, no. 1, 15 August 2013 (2013-08-15), page 78, XP021160152, ISSN: 1477-7827, DOI: 10.1186/1477-7827-11-78
- E. MARIA C. OHLSSON TEAGUE ET AL: "MicroRNA-Regulated Pathways Associated with Endometriosis", MOLECULAR ENDOCRINOLOGY, vol. 23, no. 2, 1 February 2009 (2009-02-01), pages 265-275, XP055127849, ISSN: 0888-8809, DOI: 10.1210/me.2008-0387
- K. E. MAY ET AL: "Peripheral biomarkers of endometriosis: a systematic review", HUMAN REPRODUCTION UPDATE, vol. 16, no. 6, 1 November 2010 (2010-11-01), pages 651-674, XP055187792, ISSN: 1355-4786, DOI: 10.1093/humupd/dmq009

## Description

### TECHNICAL FIELD

This invention relates to biomarkers useful for the diagnosis and/or prognosis of endometriosis. The biomarkers are also useful for monitoring treatment of the disease and can also be used as targets for therapeutic intervention.

### BACKGROUND

Endometriosis is a common gynaecological condition in which uterine or uterine-like endometrial and/or stromal cells are found exterior to the uterus in various sites and tissues of the body. These sites are numerous and can vary from pelvic infiltrations (superficial or deeply infiltrating) to ovarian cysts (also known as endometriomas), vaginal, vulval, bowel and bladder infiltrations, skin and scar lesions and lesions in the lymphatic system, brain and lungs (though these are rarer). It can potentially be found in any site of the body and presents with non-specific signs and symptoms. Symptoms of endometriosis vary widely and can range from pain, *e*.*g*. pelvic pain, dysmenorrhea, dyspareunia, dyschesia, dysuria, dyschesia, fatigue and infertility causing numerous physical and psychosocial co-morbidities and severely limiting the quality of patient's life [1]. Endometriosis is a chronic and possibly hormonally responsive condition which recurs, potentially leaving scars and fibrosis in the tissue it affects.

Endometriosis is found almost exclusively in women, generally in their reproductive years. The average age at diagnosis is 25-29 years, but there is an estimated delay in diagnosis of disease quoted at around 6.7 years in one multicentre trial [2]. US studies report an average delay in diagnosis of 11.7 years and UK studies estimate 8 years of diagnostic delay [3]. The disease in itself affects around 5%-15% of women in their reproductive age varying in prevalence between populations. It is estimated to affect over 70 million women worldwide [4]. Diagnostic incidence rates have been quoted in 26.4% (95% CI: 20-32.7%) of African to 54.5% (95% CI: 44.2-64.7%) in South American countries [5]. Endometriosis is prevalent in 0.5-5% of fertile women and 25-40% in infertile women making it a leading cause of infertility [6,7]. In one Norwegian study the lifetime prevalence was documented at 2.2% [8].

There are no defined causative factors but risk factors associated with disease vary, and suggestions include age (rare before and after menarche and menopause respectively), social class and race [9] (increased incidence in higher social classes). In utero exposure to multiple pregnancies and diethylstilbestrol [10], infertility [11], low parity [11], oral contraceptive use and age of commencement or stopping of oral contraceptive pill [12], family history [13,14], smoking [15], diet [7], exercise [16], body mass index [17], dioxin [18], a history of immune disorders [19], association with non-Hodgkin's lymphoma [20], association with pigmentary traits [21], links with melanoma [22] and association with ovarian (serous, mucinous, endometroid, clear cell, other subtypes) and endometrial cancer have all been published. Most studies describing links to diet, exercise and familial inheritance show conflicting evidence and are by no means definitive.

Theories of causation vary with literature supporting or refuting each respectively. Theories include those of: *in situ* development, mullerianosis [23], accentuation by genital tract anomalies [24], genetic predisposition [25], coelomic metaplasia [26], induction [27], transplantation [28], retrograde menstruation [29], endometrial stem cells [30], physiological phenomenon [31], alterations in the endometrium [32], exogenous endometrial hormonal production [33], angiogenesis [34], the evasion of endometrial tissue from the immune system [35], cellular protection from apoptosis [36,37], the potential of endometrium to implant [38] and invade [39], and differences in peritoneal fluid [40].

The diagnosis of endometriosis is a combination of physician vigilance and knowledge, patient history, clinical symptoms/signs at examination and use of radiology (including trans-vaginal and trans-abdominal ultrasound to assess for endometriomas or macroscopic lesions). MRI and CT scanning are used for deep lesions and the most utilised serum marker is the non-specific CA125 which has poor correlation with presence or severity of disease. Biomarkers reported in literature range from the use of cytokines, non-cytokines, serum and endometrial biomarkers, the presence of nerve fibres within tissues [41], gene aberrations and miRNAs. However, none of them is currently used and validated in the clinical setting. Document D1 describes circulating microRNAs identified in a genome-wide serum microRNA expression analysis as non-invasive biomarkers for endometriosis. D2 describes microRNAs expression profiling of eutopic proliferative endometrium in women with ovarian endometriosis. D3 describes microRNA-regulated pathways associated with endometriosis.

There is the postulated use of proteomic analysis of endometrium and patient blood using 2-DIGE and SELDI-TOF MS technology [42,43,44,45]. No biomarkers have so far been identified from this. Peripheral blood studies using SELDI TOF MS report proteins altered in endometriosis [46,47,48,49,50], but none of them provides sufficient sensitivity and specificity for use in clinical settings.

The gold standard for diagnosis and treatment of endometrial peritoneal lesions is invasive surgical laparoscopy enabling the visualisation of lesions. Endometriosis is removed by diathermy or/and peritoneal and/or cyst stripping. This has substantial patient morbidity and possibly mortality.

An early non-invasive test would enable earlier diagnosis and treatment, prevent chronic effects of disease that include pain, scarring, psychological trauma and infertility; reduce surgical patient morbidity and mortality and enable the monitoring of the response of disease to therapeutics and management.

There is a need for new or improved *in vitro* tests with good specificity and sensitivity to enable non-invasive diagnosis of endometriosis. It is an object of the invention to provide further and improved biomarkers for the diagnosis of endometriosis e.g. using *in vitro* detection techniques.

### DISCLOSURE OF THE INVENTION

The present invention provides a method for providing a diagnostic indicator of whether a subject has endometriosis, comprising a step of determining the level of x different biomarkers in a sample from the subject, wherein x is 3 or more and wherein the x different biomarkers are hsa-miR-150 and ebv-miR-BART2-5p and one or more other biomarkers listed in Table 1.

In some embodiments, the one or more other biomarkers are selected from the group consisting of hsa-let-7f, hsa-let-7g, hsa-miR-1260, hsa-miR142-3p, hsa-miR-197, hsa-miR-215, hsa-miR-223, hsa-miR-30b, hsa-miR-320c, hsa-miR34a, hsa-miR-497, hsa-miR-630, hsa-miR-663 and hsa-miR-720.

In some embodiments, x is 5 or more.

In some embodiments, x is 10 or more.

In some embodiments, the method also includes a step of determining if a sample from the subject contains autoantibodies against CA125 and/or CA19-9.

In some embodiments, the method involves comparing levels of the biomarkers in the subject sample to levels in (i) a sample from a patient with endometriosis and/or (ii) a sample from a patient without endometriosis.

In some embodiments, the method involves analysing levels of the biomarkers in the sample with a classifier algorithm which uses the measured levels of to distinguish between patients with endometriosis and patients without endometriosis.

In some embodiments, the subject is (i) pre-symptomatic for endometriosis or (ii) already displaying clinical symptoms of endometriosis.

In some embodiments, the sample is a body fluid.

In some embodiments, the sample is cervical discharge or peritoneal fluid.

In some embodiments, the presence of antibodies is determined using an immunoassay.

In some embodiments, the immunoassay utilises an antigen comprising an amino acid sequence (i) having at least 90% sequence identity to an amino acid sequence disclosed in Table 1, and/or (ii) comprising at least one epitope from an amino acid sequence disclosed in Table 1.

In some embodiments, the immunoassay utilises a fusion polypeptide with a first region and a second region, wherein the first region can react with an auto-antibody in a sample and the second region can react with a substrate to immobilise the fusion polypeptide thereon.

In some embodiments, the subject sample is a human.

The disclosure is based on the identification of correlations between endometriosis and the increased or decreased levels of certain proteins and small non-coding miRNAs.

The inventors have identified miRNAs for which the expression profiles can be used to indicate that a subject has endometriosis. These miRNAs are present at significantly different levels in subjects with endometriosis and without endometriosis. Detection of the presence or absence of these miRNAs, and/or of changes in their levels over time can thus be used to indicate that a subject has endometriosis, or has the potential to develop endometriosis in the future. These miRNAs function as biomarkers of endometriosis.

The inventors have also identified antigens for which the level of auto-antibodies can be used to indicate that a subject has endometriosis. Auto-antibodies against these antigens are present at significantly different levels in subjects with endometriosis and without endometriosis. Detection of the presence or absence of these auto-antibodies, and/or of changes in their levels over time, can thus be used to indicate that a subject has endometriosis. The auto-antibodies and their antigens also function as biomarkers of endometriosis.

Detection of these biomarkers in a subject sample can be used to improve the diagnosis, prognosis and monitoring of endometriosis. Advantageously, the disclosure can be used to distinguish between endometriosis and other forms of intra-abdominal inflammation.

The inventors have identified 343 such biomarkers (Table 1) and the disclosure uses at least one of these to assist in the diagnosis of endometriosis by measuring level(s) of the biomarker(s). The biomarker can be a protein or a miRNA. A protein biomarker can be (i) auto-antibody which binds to an antigen in Table 1 and/or (ii) an antigen in Table 1, but is preferably the former.

Thus the disclosure provides a method for analysing a subject sample, comprising a step of determining the level of a Table 1 biomarker in the sample, wherein the level of the biomarker provides a diagnostic indicator of whether the subject has endometriosis.

Analysis of a single Table 1 biomarker can be performed, and detection of the auto-antibody/antigen or miRNA can provide a useful diagnostic indicator for endometriosis even without considering any of the other Table 1 biomarkers.

The sensitivity and specificity of diagnosis can be improved, however, by combining data for multiple biomarkers. It is thus preferred to analyse more than one Table 1 biomarker. Analysis of two or more different biomarkers (a "panel") can enhance the sensitivity and/or specificity of diagnosis compared to analysis of a single biomarker. Each different biomarker in a panel is shown in a different row in Table 1, e.g. measuring both auto-antibody which binds to an antigen listed in Table 1 and the antigen itself is measurement of a single biomarker rather than of a panel.

The inventors found that the combination of the information from protein and miRNA biomarkers provides an enhancement in diagnostic utility, as measured by sensitivity, specificity and/or area under the Receiver Operating Characteristic (ROC) curve. Thus, the disclosure preferably uses at least one protein biomarker from Table 1 and at least one miRNA biomarker from Table 1 to assist in the diagnosis of endometriosis.
Thus the disclosure provides a method for analysing a subject sample, comprising a step of determining the levels of x different biomarkers of Table 1, wherein the levels of the biomarkers provide a diagnostic indicator of whether the subject has endometriosis. The value of x is 2 or more e.g. 2, 3, 4, 5, 6, 7, 8, 9, 10, 11, 12, 13, 14, 15 or more (e.g. up to 343). These panels may include (i) any specific one of the 343 biomarkers in Table 1 in combination with (ii) any of the other 342 biomarkers in Table 1. Preferably the panel includes at least one protein biomarker (e.g. auto-antibodies or an antigen) from Table 1 and at least one miRNA biomarker from Table 1. Preferably the panel includes protein biomarkers only (e.g. auto-antibodies or antigens) from Table 1. Preferably the panel includes miRNA biomarkers only from Table 1.

Suitable panels are described below and panels of particular interest include those listed in Tables 11 to 15. Preferred panels have from 2 to 6 biomarkers, as using >6 of them adds little to sensitivity and specificity.

The Table 1 biomarkers can be used in combination with one or more of: (a) known biomarkers for endometriosis, which may be auto-antibodies, antigens or miRNAs; and/or (b) other information about the subject from whom a sample was taken e.g. age, genotype (genetic variations can affect auto-antibody profiles [51] and considerable progress on the elucidation of the genetics of endometriosis has been made [52]), weight, other clinically-relevant data or phenotypic information; and/or (c) other diagnostic tests or clinical indicators for endometriosis. Such combinations can enhance the sensitivity and/or specificity of diagnosis. Known endometriosis biomarkers of particular interest include, but are not limited to, auto-antibodies against CA125, CA19-9 and/or any of the antigens listed in Table 5.

Thus the disclosure provides a method for analysing a subject sample, comprising a step of determining:
(a) the level(s) of y Table 1 biomarker(s), wherein the levels of the biomarkers provide a diagnostic indicator of whether the subject has endometriosis; and also one or more of:
(b) if a sample from the subject contains a known biomarker selected from the group consisting of auto-antibodies against antigens such as CA125, CA19-9 and/or any of the antigens listed in Table 5 (and optionally, any other known biomarkers e.g. see above); wherein detection of the known biomarker provides a second diagnostic indicator of whether the subject has endometriosis;
(c) the subject's age and/or gender,
and combining the different diagnostic indicators (and optionally age and/or gender) to provide an aggregate diagnostic indicator of whether the subject has endometriosis.

The samples used in (a) and (b) may be the same or different.

The value of y is 1 or more e.g. 1, 2, 3, 4, 5, 6, 7, 8, 9, 10, 11, 12, 13, 14, 15 (e.g. up to 343). When *y* >1 the disclosure uses a panel of different Table 1 biomarkers. When y>1, preferably the panel includes at least one protein biomarker (e.g. auto-antibodies or an antigen) from Table 1 and at least one miRNA biomarker from Table 1.
The disclosure also provides, in a method for diagnosing if a subject has endometriosis, an improvement consisting of determining in a sample from the subject the level(s) of y biomarker(s) of Table 1, wherein the level(s) of the biomarker(s) provide a diagnostic indicator of whether the subject has endometriosis. The biomarker(s) of Table 1 can be used in combination with known endometriosis biomarkers, as discussed above.

The disclosure also provides a method for diagnosing a subject as having endometriosis, comprising steps of: (i) determining the levels of y biomarkers of Table 1 in a sample from the subject; and (ii) comparing the determination from step (i) to data obtained from samples from subjects without endometriosis and/or from subjects with endometriosis, wherein the comparison provides a diagnostic indicator of whether the subject has endometriosis. The comparison in step (ii) can use a classifier algorithm as discussed in more detail below. The biomarkers measured in step (i) can be used in combination with known endometriosis biomarkers, as discussed above.

The disclosure also provides a method for monitoring development of endometriosis in a subject, comprising steps of: (i) determining the levels of *z₁* biomarker(s) of Table 1 in a first sample from the subject taken at a first time; and (ii) determining the levels of *z₂* biomarker(s) of Table 1 in a second sample from the subject taken at a second time, wherein: (a) the second time is later than the first time; (b) one or more of the *z₂* biomarker(s) were present in the first sample; and (c) a change in the level(s) of the biomarker(s) in the second sample compared with the first sample indicates that endometriosis is in remission or is progressing. Thus the method monitors the biomarker(s) over time, with changing levels indicating whether the disease is getting better or worse.

The disease development can be either an improvement or a worsening of the disease, and this method may be used in various ways e.g. to monitor the natural progress of a disease, or to monitor the efficacy of a therapy being administered to the subject. Thus a subject may receive a therapeutic agent or may receive surgery for removing endometriosis before the first time, at the first time, or between the first time and the second time. Increased levels of antibodies against a particular auto-antigen may be due to "epitope spreading", in which additional antibodies or antibody classes are raised to antigens against which an antibody response has already been mounted [53].

The value of *z₁* is 1 or more e.g. 1, 2, 3, 4, 5, 6, 7, 8, 9, 10, 11, 12, 13, 14, 15 (e.g. up to 343). The value of *z₂* is 1 or more e.g. 1, 2, 3, 4, 5, 6, 7, 8, 9, 10, 11, 12, 13, 14, 15 (e.g. up to 343). The values of *z₁* and *z₂* may be the same or different. If they are different, it is usual that *z₁*>*z₂* as the later analysis (z₂) can focus on biomarkers which were already detected in the earlier analysis; as described herein, however, z₂ can be larger than z₁ e.g. if previous data have indicated that an expanded panel should be used; as described herein, *z₂*=*z₁ e.g.* so that, for convenience, the same panel can be used for both analyses. When *z₁*>1 or *z₂*>1, the biomarkers are different biomarkers. The z₁ and/or z₂ biomarker(s) can be used in combination with known endometriosis biomarkers, as discussed above.
The disclosure also provides a method for monitoring development of endometriosis in a subject, comprising steps of: (i) determining the level of at least *w₁* Table 1 biomarkers in a first sample taken at a first time from the subject; and (ii) determining the level of at least *w₂* Table 1 biomarkers in a second sample taken at a second time from the subject, wherein: (a) the second time is later than the first time; (b) at least one biomarker is common to both the *w₁* and *w₂* biomarkers; (c) the level of at least one biomarker common to both the *w₁* and *w₂* biomarkers is different in the first and second samples, thereby indicating that the endometriosis is progressing or regressing. Thus the method monitors the range of biomarkers over time, with a broadening in the number of detected biomarkers indicating that the disease is getting worse. As mentioned above, this method may be used to monitor disease development in various ways.

The value of *w₁* is 1 or more e.g. 1, 2, 3, 4, 5, 6, 7, 8, 9, 10, 11, 12, 13, 14, 15 (e.g. up to 343). The value of *w₂* is 2 or more e.g. 1, 2, 3, 4, 5, 6, 7, 8, 9, 10, 11, 12, 13, 14, 15 (e.g. up to 343). The values of *w₁* and *w₂* may be the same or different. If they are different, it is usual that *w₂*≥*w₁,* as the later analysis should focus on a biomarker panel that is at least as wide as the number already detected in the earlier analysis. There will usually be an overlap between the w₁ and w₂ biomarkers (including situations where they are the same, such that the same biomarkers are measured at two time points) but it is also possible for w₁ and w₂ to have no biomarkers in common. The w₁ and/or w₂ biomarker(s) can be used in combination with known endometriosis biomarkers, as discussed above.

Where the methods involve a first time and a second time, these times may differ by at least 1 day, 1 week, 1 month or 1 year. Samples may be taken regularly. The methods may involve measuring biomarkers in more than 2 samples taken at more than 2 time points i.e. there may be a 3rd sample, a 4th sample, a 5th sample, *etc.*

The disclosure also provides a diagnostic device for use in diagnosis of endometriosis, wherein the device permits determination of the level(s) of y Table 1 biomarkers. The value of y is defined above. The device may also permit determination of whether a sample contains one or more of the known endometriosis biomarkers mentioned above.

The disclosure also provides a kit comprising (i) a diagnostic device of the disclosure and (ii) instructions for using the device to detect *y* of the Table 1 biomarkers. The value of y is defined above. The kit is useful in the diagnosis of endometriosis.

The disclosure also provides a kit comprising reagents for measuring the levels of x different Table 1 biomarkers. The kit may also include reagents for determining whether a sample contains one or more of the known endometriosis biomarkers mentioned above. The value of x is defined above. The kit is useful in the diagnosis of endometriosis.
The disclosure also provides a kit comprising components for preparing a diagnostic device of the disclosure. For instance, the kit may comprise individual detection reagents for x different biomarkers, such that an array of those x biomarkers can be prepared.

The disclosure also provides a product comprising (i) one or more detection reagents which permit measurement of x different Table 1 biomarkers, and (ii) a sample from a subject.

The disclosure also provides a software product comprising (i) code that accesses data attributed to a sample, the data comprising measurement of y Table 1 biomarkers, and (ii) code that executes an algorithm for assessing the data to represent a level of y of the biomarkers in the sample. The software product may also comprise (iii) code that executes an algorithm for assessing the result of step (ii) to provide a diagnostic indicator of whether the subject has endometriosis. As discussed below, suitable algorithms for use in part (iii) include support vector machine algorithms, artificial neural networks, tree-based methods, genetic programming, *etc.* The algorithm can preferably classify the data of part (ii) to distinguish between subjects with endometriosis and subjects without based on measured biomarker levels in samples taken from such subjects. The disclosure also provides methods for training such algorithms. The y biomarker(s) can be used in combination with known endometriosis biomarkers, as discussed above.

The disclosure also provides a computer which is loaded with and/or is running a software product of the disclosure.

The disclosure also extends to methods for communicating the results of a method of the disclosure. This method may involve communicating assay results and/or diagnostic results. Such communication may be to, for example, technicians, physicians or patients. As described herein, detection methods of the disclosure will be performed in one country and the results will be communicated to a recipient in a different country.

The disclosure also provides an isolated antibody (preferably a human antibody) which recognises one of the antigens listed in Table 1. The disclosure also provides an isolated nucleic acid encoding the heavy and/or light chain of the antibody. The disclosure also provides a vector comprising this nucleic acid, and a host cell comprising this vector. The disclosure also provides a method for expressing the antibody comprising culturing the host cell under conditions which permit production of the antibody. The disclosure also provides derivatives of the human antibody e.g. F(ab')₂ and F(ab) fragments, Fv fragments, single-chain antibodies such as single chain Fv molecules (scFv), minibodies, dAbs, *etc.*

The disclosure also provides the use of a Table 1 biomarker as a biomarker for endometriosis.

The disclosure also provides the use of x different Table 1 biomarkers as biomarkers for endometriosis. The value of x is defined above. These may include (i) any specific one of the 343 biomarkers in Table 1 in combination with (ii) any of the other 342 biomarkers in Table 1. Preferably the disclosure uses at least one protein biomarker (e.g. auto-antibodies or an antigen) from Table 1 and at least one miRNA biomarker from Table 1. Preferably the panel includes protein biomarkers only (e.g. auto-antibodies or antigens) from Table 1. Preferably the panel includes miRNA biomarkers only from Table 1.

The disclosure also provides the use as combined biomarkers for endometriosis of (a) at least y Table 1 biomarker(s)and (b) biomarkers including auto-antibodies against CA125 , CA19-9 and/or any of the antigens from Table 5 (and optionally, any other known biomarkers e.g. see above). The value of y is defined above. When y>1 the disclosure uses a panel of biomarkers of the disclosure. When y>1, preferably the panel includes at least one protein biomarker (e.g. auto-antibodies or an antigen) from Table 1 and at least one miRNA biomarker from Table 1. Such combinations include those discussed above. Panels of particular interest include those listed in Tables 10 to 15.

### Biomarkers of the invention

### Antigens

The inventors identified auto-antibodies against 121 different human antigens (as listed in Table 1) and these can be used as endometriosis biomarkers. Further details of the 121 antigens are given in Table 2. Within the 121 antigens, the human antigens mentioned in Tables 6 and 7 are particularly useful for distinguishing between samples from subjects with endometriosis and from subjects without endometriosis. Further auto-antibody biomarkers can be used in addition to these 121 *(e.g.* any of the biomarkers listed in Table 5).

The sequence listing provides an example of a natural coding sequence for these antigens. These specific coding sequences are not limiting on the disclosure, however, and auto-antibody biomarkers may recognise variants of polypeptides encoded by these natural sequences (e.g. allelic variants, polymorphic forms, mutants, splice variants, or gene fusions), provided that the variant has an epitope recognised by the auto-antibody. Details on allelic variants of or mutations in human genes are available from various sources, such as the ALFRED database [54] or, in relation to disease associations, the OMIM [55] and HGMD [56] databases. Details of splice variants of human genes are available from various sources, such as ASD [57].

### miRNAs

The inventors identified 222 individual human miRNAs (as listed in Table 1), and these can be used as endometriosis biomarkers. Further details of these miRNAs are given in Table 3 and Table 4. Within the 222 miRNAs, the miRNAs mentioned in Tables 8, 9 and 16-21 are particularly useful for distinguishing between samples from subjects with endometriosis and from subjects without endometriosis.

Preferably, the disclosure uses any of the miRNAs mentioned in Tables 9 and 16-18.

Preferably, the disclosure uses any of the miRNAs listed in the group consisting of: ebv-miR-BART2-5p, hsa-let-7f, hsa-let-7g, hsa-miR-1260, hsa-miR142-3p, hsa-miR-197, hsa-miR-215, hsa-miR-223, hsa-miR-30b, hsa-miR-320c, hsa-miR34a, hsa-miR-497, hsa-miR-630, hsa-miR-663 and hsa-miR-720.

The specific sequences in Tables 3 and 4 are not limiting on the disclosure. The disclosure includes detecting and measuring the levels of polymorphic variants of these miRNAs. A database outlining in more detail the miRNAs listed herein is available: MiRBase [58, 59, 60, 61] or, in relation to target prediction, the DIANA-microT [62, 63], microRNA.org [64], miRDB [65, 66], TargetScan [67] and PicTar [68] databases.

As mentioned above, detection of a single Table 1 biomarker can provide useful diagnostic information, but each biomarker might not individually provide information which is useful i.e. auto-antibodies against a Table 1 antigen may be present in some, but not all, subjects with endometriosis. An inability of a single biomarker to provide universal diagnostic results for all subjects does not mean that this biomarker has no diagnostic utility; rather, any such inability means that the test results (as in all diagnostic tests) have to be properly understood and interpreted.

To address the possibility that a single biomarker might not provide universal diagnostic results, and to increase the overall confidence that an assay is giving sensitive and specific results across a disease population, it is advantageous to analyse a plurality of the Table 1 biomarkers (*i.e*. a panel). For instance, a negative signal for a particular Table 1 antigen is not necessarily indicative of the absence of endometriosis, confidence that a subject does not have endometriosis increases as the number of negative results increases. For example, if all 343 biomarkers are tested and are negative then the result provides a higher degree of confidence than if only 1 biomarker is tested and is negative. Thus biomarker panels are most useful for enhancing the distinction seen between diseased and non-diseased samples.

As mentioned above, though, preferred panels have from 2 to 6 biomarkers as the burden of measuring a higher number of markers is usually not rewarded by better sensitivity or specificity.

The inventors found that the combination of the information from protein and miRNA biomarkers improves diagnostic power, as measured by sensitivity, specificity and/or area under the Receiver Operating Characteristic (ROC) curve. Thus, the disclosure preferably uses at least one protein biomarker from Table 1 and at least one miRNA biomarker from Table 1 to assist in the diagnosis of endometriosis.

Preferred panels are given below, and are listed in Tables 11-15.

Some biomarkers of the disclosure have different relative differential expression profiles in endometriosis samples compared to a control. Pairs of these biomarkers, where one is up-regulated and the other is down-regulated relative to the same control sample, may provide a useful way of diagnosing or predicting endometriosis. For example, the inventors found that ebv-miR-BART2-5p is up-regulated in endometriosis samples vs. non-endometriosis samples (i.e. a negative control) and hsa-miR-564 is down-regulated in endometriosis samples vs. non-endometriosis samples (i.e. a negative control), so this pair would be useful. This divergent behaviour can enhance diagnosis or prediction of endometriosis when the pair of the biomarkers is assessed in the same sample.

### The subject

The disclosure is used, either alone or in combination with other measurements or data concerning the subject, for diagnosing disease in a subject.

The subject may be pre-symptomatic for endometriosis or may already be displaying clinical symptoms, e.g. pelvic pain, dysmenorrhea, dyspareunia, dyschesia, fatigue and infertility. For pre-symptomatic subjects the disclosure is useful for predicting that symptoms may develop in the future if no preventative action is taken. For subjects already displaying clinical symptoms, the disclosure may be used to confirm or resolve another diagnosis. The subject may already have begun treatment for endometriosis.

As described herein, the subject may already be known to be predisposed to development of endometriosis *e.g*. due to family or genetic links. As described herein, the subject may have no such predisposition, and may develop the disease as a result of environmental factors *e.g*. as a result of exposure to particular chemicals (such as toxins or pharmaceuticals), as a result of diet, as a result of infection, *etc.*

The subject will typically be a human being. As described herein, however, the disclosure is useful in non-human mammals, e.g. mouse, rat, rabbit, guinea pig, cat, dog, horse, pig, cow, or non-human primate (monkeys or apes, such as macaques or chimpanzees). In non-human descriptions, any detection antigens used with the disclosure will typically be based on the relevant non-human ortholog of the human auto-antigens disclosed herein. As described herein, animals can be used experimentally to monitor the impact of a therapeutic on a particular biomarker.

### The sample

The disclosure analyses samples from subjects. Many types of sample can include auto-antibodies and/or antigens or miRNAs suitable for detection by the disclosure. The sample can be a tissue sample, e.g. from regions of uterine or vaginal tissues. Alternatively, the sample can be or a body fluid sample, e.g. cervical discharge or peritoneal fluid.

As described herein, a method of the disclosure involves a step of obtaining the sample from the subject. As described herein, however, the sample is obtained separately from and prior to performing a method of the disclosure.

Detection of the biomarkers of the disclosure may be performed directly on a sample taken from a subject, or the sample may be treated between being taken from a subject and being analysed. For example, a blood sample may be treated to remove cells, leaving antibody-containing plasma for analysis, or to remove cells and various clotting factors, leaving antibody-containing serum for analysis. A bodily fluid sample, e.g. a blood sample, can be treated to extract circulating endometrial cells for analysis. Various types of sample may be subjected to treatments such as dilution, aliquoting, sub-sampling, heating, freezing, irradiation, *etc.* between being taken from the body and being analysed. Addition of processing reagents is also typical for various sample types e.g. addition of anticoagulants to blood samples.

### Biomarker detection

### Auto-antibody detection

The disclosure involves determining in a sample the level of auto-antibodies that bind to the antigens listed in Table 1. Immunochemical techniques for detecting antibodies against specific antigens are well known in the art, as are techniques for detecting specific antigens themselves. Detection of an antibody will typically involve contacting a sample with a detection antigen, wherein a binding reaction between the sample and the detection antigen indicates the presence of the antibody of interest. Detection of an antigen will typically involve contacting a sample with a detection antibody, wherein a binding reaction between the sample and the detection antibody indicates the presence of the antigen of interest. Detection of an antigen can also be determined by non-immunological methods, depending on the nature of the antigen e.g. if the antigen is an enzyme then its enzymatic activity can be assayed, or if the antigen is a receptor then its binding activity can be assayed, *etc.* For example, the CLK1 kinase can be assayed using methods known in the art.

A detection antigen can be a natural antigen recognised by the auto-antibody (*e.g*. a mature human protein disclosed in Table 1), or it may be an antigen comprising an epitope which is recognized by the auto-antibody. Where a detection antigen is a polypeptide, its amino acid sequence can vary from the natural sequences disclosed above, provided that it has the ability to specifically bind to an auto-antibody of the disclosure (*i.e*. the binding is not non-specific and so the detection antigen will not arbitrarily bind to antibodies in a sample). It may even have little in common with the natural sequence (*e.g*. a mimotope, an aptamer, *etc*.). Typically, though, a detection antigen will comprise an amino acid sequence (i) having at least 90% (*e*.*g*. ≥91%, ≥92%, ≥93%, ≥94%, ≥95%, ≥96%, ≥97%, ≥98%, ≥99%) sequence identity to the relevant SEQ ID NO disclosed herein (*i.e*. any one of SEQ ID NOs: 1-121) across the length of the detection antigen, and/or (ii) comprising at least one epitope from the relevant SEQ ID NO disclosed herein (*i.e*. any one of SEQ ID NOs: 1-121). Thus the detection antigen may be any of the variants discussed above.

Epitopes are the parts of an antigen that are recognised by and bind to the antigen binding sites of antibodies and are also known as "antigenic determinants". An epitope-containing fragment may contain a linear epitope from within a SEQ ID NO and so may comprise a fragment of at least *n* consecutive amino acids of the SEQ ID NO, wherein *n* may be 7 or more (*e.g*. 8, 10, 12, 14, 16, 18, 20, 25, 30, 35, 40, 50, 60, 70, 80, 90, 100, 150, 200, 250 or more). B-cell epitopes can be identified empirically (e.g. using PEPSCAN [69,70] or similar methods), or they can be predicted e.g. using the Jameson-Wolf antigenic index [71], ADEPT [72], hydrophilicity [73], antigenic index [74], MAPITOPE [75], SEPPA [76], matrix-based approaches [77], the amino acid pair antigenicity scale [78], or any other suitable method e.g. see ref.79. Predicted epitopes can readily be tested for actual immunochemical reactivity with samples.

Detection antigens can be purified from human sources, but it is more typical to use recombinant antigens (*e.g*. particularly where the detection antigen uses sequences which are not present in the natural antigen *e.g*. for attachment). Various systems are available for recombinant expression, and the choice of system may depend on the auto-antibody to be detected. For example, prokaryotic expression (*e.g*. using *E*. *coli*) are useful for detecting many auto-antibodies, but if an auto-antibody recognises a glycoprotein then eukaryotic expression may be required. Similarly, if an auto-antibody recognises a specific discontinuous epitope then a recombinant expression system which provides correct protein folding may be required.

The detection antigen may be a fusion polypeptide with a first region and a second region, wherein the first region can react with an auto-antibody in a sample and the second region can react with a substrate to immobilise the fusion polypeptide thereon.

A detection antibody for a biomarker antigen can be a monoclonal antibody or a polyclonal antibody. Typically it will be a monoclonal antibody. The detection antibody should have the ability to specifically bind to a Table 1 antigen (*i.e*. the binding is not non-specific and so the detection antibody will not arbitrarily bind to other antigens in a sample).

Various assay formats can be used for detecting biomarkers in samples. For example, the disclosure may use one or more of western blot, immunoprecipitation, silver staining, mass spectrometry (e.g. MALDI-MS), conductivity-based methods, dot blot, slot blot, colorimetric methods, fluorescence-based detection methods, or any form of immunoassay, *etc.* The binding of antibodies to antigens can be detected by any means, including enzyme-linked assays such as ELISA, radioimmunoassays (RIA), immunoradiometric assays (IRMA), immunoenzymatic assays (IEMA), DELFIA™ assays, surface plasmon resonance or other evanescent light techniques (e.g. using planar waveguide technology), label-free electrochemical sensors, *etc.* Sandwich assays are typical for immunological methods.

As described herein, where multiple biomarkers are to be detected an array-based assay format is preferable, in which a sample that potentially contains the biomarkers is simultaneously contacted with multiple detection reagents (antibodies and/or antigens) in a single reaction compartment. Antigen and antibody arrays are well known in the art *e.g*. see references 80-86, including arrays for detecting auto-antibodies. Such arrays may be prepared by various techniques, such as those disclosed in references 87-91, which are particularly useful for preparing microarrays of correctly-folded polypeptides to facilitate binding interactions with auto-antibodies. It has been estimated that most B-cell epitopes are discontinuous and such epitopes are known to be important in diseases with an autoimmune component. For example, in autoimmune thyroid diseases, auto-antibodies arise to discontinuous epitopes on the immunodominant region on the surface of thyroid peroxidase and in Goodpasture disease auto-antibodies arise to two major conformational epitopes. Protein arrays which have been developed to present correctly-folded polypeptides displaying native structures and discontinuous epitopes are therefore particularly well suited to studies of diseases where auto-antibody responses occur [84].

Methods and apparatuses for detecting binding reactions on antigen arrays are now standard in the art. Preferred detection methods are fluorescence-based detection methods. To detect auto-antibodies which have bound to immobilised antigens, a sandwich assay is typical, in which the primary antibody is an auto-antibody from the sample and the secondary antibody is a labelled anti-sample antibody (e.g. an anti-human antibody).

Where a biomarker is an auto-antibody the disclosure will generally detect IgG antibodies, but detection of auto-antibodies with other subtypes is also possible e.g. by using a detection reagent which recognises the appropriate class of auto-antibody (IgA, IgM, IgE or IgD rather than IgG). The assay format may be able to distinguish between different antibody subtypes and/or isotypes. Different subtypes [92] and isotypes [93] can influence auto-antibody repertoires. For instance, a sandwich assay can distinguish between different subtypes by using differentially-labelled secondary antibodies e.g. different labels for anti-IgG and anti-lgM.

As mentioned above, the disclosure provides a diagnostic device which permits determination of whether a sample contains Table 1 biomarkers. Such devices will typically comprise one or more antigen(s) and/or antibodies immobilised on a solid substrate (e.g. on glass, plastic, nylon, *etc.).* Immobilisation may be by covalent or non-covalent bonding (e.g. non-covalent bonding of a fusion polypeptide, as discussed above, to an immobilised functional group such as an avidin [89] or a bleomycin-family antibiotic [91]). Antigen arrays are a preferred format, with detection antigens being individually addressable. The immobilised antigens will be able to react with auto-antibodies which recognise a Table 1 antigen.

As described herein, the solid substrate may comprise a strip, a slide, a bead, a well of a microtitre plate, a conductive surface suitable for performing mass spectrometry analysis [94], a semiconductive surface [95,96], a surface plasmon resonance support, a planar waveguide technology support, a microfluidic devices, or any other device or technology suitable for detection of antibody-antigen binding.

Where the disclosure provides or uses an antigen array for detecting a panel of auto-antibodies as disclosed herein, as described herein, the array may include only antigens for detecting these auto-antibodies. As described herein, however, the array may include polypeptides in addition to those useful for detecting the auto-antibodies. For example, an array may include one or more control polypeptides. Suitable positive control polypeptides include an anti-human immunoglobulin antibody, such as an anti-IgM antibody, an anti-IgG antibody, an anti-lgA antibody, an anti-lgE antibody or combinations thereof. Other suitable positive control polypeptides which can bind to sample antibodies include protein A or protein G, typically in recombinant form. Suitable negative control polypeptides include, but are not limited to, β-gatactosidase, serum albumins (*e.g*. bovine serum albumin (BSA) or human serum albumin (HSA)), protein tags, bacterial proteins, yeast proteins, citrullinated polypeptides, *etc.* Negative control features on an array can also be polypeptide-free *e.g*. buffer alone, DNA, *etc.* An array's control features are used during performance of a method of the disclosure to check that the method has performed as expected e.g. to ensure that expected proteins are present (*e.g*. a positive signal from serum proteins in a serum sample) and that unexpected substances are not present (e.g. a positive signal from an array spot of buffer alone would be unexpected).

In an antigen array of the disclosure, at least 10% (e.g. ≥20%, ≥30%, ≥40%, ≥50%, ≥60%, ≥70%, ≥80%, ≥90%, ≥95%, or more) of the total number of different proteins present on the array may be for detecting auto-antibodies as disclosed herein.

An antigen array of the disclosure may include one or more replicates of a detection antigen and/or control feature e.g. duplicates, triplicates or quadruplicates. Replicates provide redundancy, provide intra-array controls, and facilitate inter-array comparisons.

An antigen array of the disclosure may include detection antigens for more than just the 121 different auto-antibodies described here, but preferably it can detect antibodies against fewer than 10000 antigens *(e.g.* <5000, <4000, <3000, <2000, <1000, <500, <250, <100, *etc.).*

An array is advantageous because it allows simultaneous detection of multiple biomarkers in a sample. Such simultaneous detection is not mandatory, however, and a panel of biomarkers can also be evaluated in series. Thus, for instance, a sample could be split into sub-samples and the sub-samples could be assayed in series. As described herein, it may not be necessary to complete analysis of the whole panel e.g. the diagnostic indicators obtained on a subset of the panel may indicate that a patient has endometriosis without requiring analysis of any further members of the panel. Such incomplete analysis of the panel is encompassed by the disclosure because of the intention or potential of the method to analyse the complete panel.

As mentioned above, some descriptions of the disclosure can include a contribution from known tests for endometriosis, such as CA125 , CA19-9 and/or any of the antigens listed in Table 5. Any known tests can be used e.g. laparoscopy, ultrasound, magnetic resonance imaging (MRI), *etc.*

### miRNA detection

Table 1 lists 222 human miRNAs, and methods of the disclosure can involve detecting and determining the level of these miRNA biomarker(s) in a sample. Further details of these miRNAs are provided in Tables 3 and 4. Tables 3 and 4 also include nucleotide sequences for these miRNAs, but polymorphisms of miRNA are known in the art and so the disclosure can also involve detecting and determining the level of a polymorphic miRNA variant of these listed miRNA sequences.

Techniques for detecting specific miRNAs are well known in the art, *e.g*. microarray analysis and NanoString's nCounter technology, polymerase chain reaction (PCR)-based methods (*e.g*. reverse transcription PCR, RT-PCR), *in-situ* hybridisation (ISH)-based methods (*e.g*. fluorescent ISH, FISH), northern blotting, sequencing (*e.g*. next-generation sequencing), fluorescence-based detection methods, *etc.* Any of the detection techniques mentioned above can be used with the disclosure. Where prognosis is the primary interest, a quantitative detection technique is preferred, *e.g*. real-time quantitative PCR (qPCR), TaqMan® or SYBR® Green.

Detection of a miRNA typically involves contacting ("hybridising") a sample with a complementary detection probe (e.g. a synthetic oligonucleotide strand), wherein a specific (rather than non-specific) binding reaction between the sample and the complementary probe indicates the presence of the miRNA of interest. As described herein, the miRNA in the sample is amplified prior to detection, *e.g.* by reverse transcription of the miRNA to produce a complementary DNA (cDNA) strand, and the derived cDNA can be used as a template in the subsequent PCR reaction.

Thus, the disclosure provides nucleic acids, which can be used, for example, as hybridization probes for specific detection of miRNA in biological samples or as single-stranded primers to amplify the miRNA.

The term "nucleic acid" includes in general means a polymeric form of nucleotides of any length, which contain deoxyribonucleotides, ribonucleotides, and/or their analogs. It includes DNA, RNA, DNA/RNA hybrids. It also includes DNA or RNA analogs, such as those containing modified backbones (e.g. peptide nucleic acids (PNAs) or phosphorothioates) or modified bases. Nucleic acid according to the disclosure can take various forms (*e.g*. single-stranded, primers, probes, labelled *etc.).* Primers and probes are generally single-stranded.

The nucleic acid can be identical or complementary to the mature miRNA sequences listed in Tables 3 and 4 (i.e. any one of SEQ ID NOs: 122-343).

The nucleic acid can comprise a nucleotide sequence that has ≥50%, ≥60%, ≥70%, ≥75%, ≥80%, ≥85%, ≥90%, ≥95%, ≥96%, ≥97%, ≥98%, ≥99% or more identity to any one of SEQ ID NOs: 122-343. Identity between sequences is preferably determined by the Smith-Waterman homology search algorithm as described above.

The nucleic acid can comprise a nucleotide sequence that has ≥50%, ≥60%, ≥70%, ≥75%, ≥80%, ≥85%, ≥90%, ≥95%, ≥96%, ≥97%, ≥98%, ≥99% or more complementarity to any one of SEQ ID NOs: 122-343.

The term "complementarity" when used in relation to nucleic acids refers to Watson-Crick base pairing. Thus the complement of C is G, the complement of G is C, the complement of A is T (or U), and the complement of T (or U) is A. It is also possible to use bases such as I (the purine inosine) e.g.to complement pyrimidines (C or T).

Where a nucleic acid is DNA, it will be appreciated that "U" in a RNA sequence will be replaced by "T" in the DNA. Similarly, where a nucleic acid is RNA, it will be appreciated that "T" in a DNA sequence will be replaced by "U" in the RNA.

The nucleic acid may be 12 or more, e.g. 12, 13, 14, 15, 16, 17 or 18, *etc.* (*e.g.* up to 50) nucleotides in length. The nucleic acid may be 15-30 nucleotides in length, 10-25 nucleotides in length, 15-25 nucleotides in length, or 20-25 nucleotides in length.

The nucleic acid may include sequences that do not hybridise to the miRNA biomarkers, and/or amplified products thereof. For example, the nucleic acid may contain additional sequences at the 5' end or at the 3' end. The additional sequences can be a linker, *e.g*. for cloning or PCR purposes.

Nucleic acid of the disclosure may be attached to a solid support (*e.g*. a bead, plate, filter, film, slide, microarray support, resin, *etc.).* Nucleic acid of the disclosure may be labelled *e.g*. with a radioactive or fluorescent label, or a biotin label. This is particularly useful where the nucleic acid is to be used in detection techniques *e*.*g*. where the nucleic acid is a primer or as a probe. Methods for preparing fluorescent labelled probes, e.g. for fluorescent in-situ hybridisation FISH analysis, are known in the art, and FISH probes can be obtained commercially, *e.g*. from Exiqon.

The disclosure may use *in-situ* hybridisation (ISH)-based methods, *e.g*. fluorescent in-situ hybridisation (FISH). Hybridization reactions can be performed under conditions of different "stringency" following by washing. Preferably, the nucleic acid of the disclosure hybridize under high stringency conditions, such that the nucleic acid specifically hybridizes to a miRNA in an amount that is detectably stronger than non-specific hybridization. Relatively high stringency conditions include, for example, low salt and/or high temperature conditions, such as provided by about 0.02-0.1 M NaCl or the equivalent, at temperatures of about 50-70 °C. A stringent wash removes nonspecific probe binding and overloaded probes. Relatively stringent wash conditions include, for example, low salt and/or presence of detergent, *e.g*. 0.02 % SDS in 1X Saline-Sodium Citrate (SSC) at about 50 °C.

As described herein, where multiple biomarkers are to be detected, an array-based assay or PCR format is preferable, in which a sample that potentially contains the biomarkers is simultaneously contacted with multiple oligonucleotide, complementary detection probes or PCR primers/probes ("multiplexed") in a single reaction compartment, whereby a reaction compartment is defined as, but not limited to, a microtitre well, microfluidic chamber or detection pore. As described herein, these multiple biomarkers could either be contacted with its complementary detection probe in separate, individual reaction compartments and/or; experiments could be separated over time and using different platform technologies in either multiplexed single reaction compartments or separate, individual reaction compartments. Microarray and PCR usage for the detection of miRNAs is well known in the art e.g. see reference 115. Microarrays may be prepared by various techniques, such as those disclosed in references 116, 117, 118. Methods based on nucleic acid amplification are also well known in the art.

Methods and apparatus for detecting binding reactions on DNA microarrays are now standard in the art. Preferred detection methods are fluorescence-based detection methods. To detect biomarkers which have bound to immobilised oligonucleotide strands on a glass substrate is typical e.g. in which the target miRNA is fluorescently labelled and then is hybridised to a complementary oligonucleotide strand (probe).

An array is advantageous because it allows simultaneous detection of multiple biomarkers in a sample. Such simultaneous detection is not mandatory, however, and a panel of biomarkers can also be evaluated in series. Thus, for instance, a sample could be split into sub-samples and the sub-samples could be assayed in series. As described herein, it may not be necessary to complete analysis of the whole panel e.g. the diagnostic indicators obtained on a subset of the panel may indicate that a patient has endometriosis without requiring analysis of any further members of the panel. Such incomplete analysis of the panel is encompassed by the disclosure because of the intention or potential of the method to analyse the complete panel.

### Data interpretation

The disclosure involves a step of determining the level of Table 1 biomarker(s). In some descriptions of the disclosure this determination for a particular marker can be a simple yes/no determination, whereas other descriptions may require a quantitative or semi-quantitative determination, still other descriptions may involve a relative determination (*e.g*. a ratio relative to another marker, or a measurement relative to the same marker in a control sample), and other descriptions may involve a threshold determination (*e.g*. a yes/no determination whether a level is above or below a threshold). Usually biomarkers will be measured to provide quantitative or semi-quantitative results (whether as relative concentration, absolute concentration, fold changes, titre, relative fluorescence *etc.)* as this gives more data for use with classifier algorithms.

Usually the raw data obtained from an assay for determining the presence, absence, or level (absolute or relative) require some sort of manipulation prior to their use. For instance, the nature of most detection techniques means that some signal will sometimes be seen even if no biomarker is actually present and so this noise may be removed before the results are interpreted. Similarly, there may be a background level of the biomarker in the general population which needs to be compensated for. Data may need scaling or standardising to facilitate inter-experiments comparisons. These and similar issues, and techniques for dealing with them, are well known in the immunodiagnostic area.

Various techniques are available to compensate for background signal in a particular experiment. For example, replicate measurements will usually be performed (*e.g*. using multiple features of the same detection probe on a single array) to determine intra-assay variation, and average values from the replicates can be compared (*e.g*. the median value of binding to quadruplicate array features). Furthermore, standard markers can be used to determine inter-assay variation and to permit calibration and/or normalisation *e.g*. an array can include one or more standards for indicating whether measured signals should be proportionally increased or decreased. For example, an assay might include a step of analysing the level of one or more control marker(s) in a sample *e.g*. levels of an antigen or antibody unrelated to endometriosis. Signal may be adjusted according to distribution in a single experiment. For instance, signals in a single array experiment may be expressed as a percentage of interquartile differences *e.g*. as [observed signal - 25th percentile] / [75th percentile - 25th percentile]. This percentage may then be normalised *e.g*. using a standard quantile normalization matrix, such as disclosed in reference 97, in which all percentage values on a single array are ranked and replaced by the average of percentages for antigens with the same rank on all arrays. Overall, this process gives data distributions with identical median and quartile values. Data transformations of this type are standard in the art for permitting valid inter-array comparisons despite variation between different experiments.

The level of an auto-antibody relative to a single baseline level may be defined as a fold difference. Normally it is desirable to use techniques that can indicate a change of at least 1.5-fold *e.g*. ≥1.75-fold, ≥2-fold, ≥2.5-fold, ≥5-fold, *etc.*

A control sample can also be used for data normalisation. For example, levels of an antibody or a miRNA unrelated to endometriosis can be measured in both the sample and a negative control, and signal from other antibodies can be adjusted accordingly. A negative control sample is from a subject that does not have any clinical presentation of endometriosis.

As described herein, rather than (or in addition to) comparing a biomarker against a 'normal' baseline, they will be compared to levels seen in a sample from a subject known to have endometriosis and known to have a particular biomarker (*i.e*. comparison to a positive control). For some biomarkers this comparison may be easier than using a lower negative control level. A preference for comparison against a negative or positive control may depend on the dynamic range between negative and positive signals.

Levels of the biomarkers in a control may be determined in parallel to determining levels in the sample. Rather than making a parallel determination, however, it can be more convenient to use an absolute control level based on empirical data. For example, the level of a particular biomarker may be measured in samples taken from a range of subjects without endometriosis. Those levels can be used to build a baseline across the range of subjects. This may involve normalization relative to a reference antibody. A population of negative control subjects can be used to provide a collection of baseline levels for subjects of different genders, ages, ethnicities, habits (*e.g*. smokers, non-smokers), *etc.,* so that, if there is variation across the population, a control can be matched to a particular subject as closely as possible. Thus, by analysing non-diseased samples from a sufficiently large number of subjects it is possible to establish an empirical baseline for any particular auto-antibody or miRNA, which can serve as the control level for comparison according to the disclosure. The control level is not necessarily a single value, but could be a range, against which a test value can be compared. For instance, if a particular auto-antibody titre is variable across non-diseased subjects, but is always in the range of 20-100 (arbitrary) units, a titre of 400 units in a sample would indicate a disease state.

As well as compensating for variation which is inherent between different experiments, it can also be important to compensate for background levels of a biomarker which are present in the general population. Again, suitable techniques are well known. For example, levels of a particular biomarker in a sample will usually be measured quantitatively or semi-quantitatively to permit comparison to the background level of that biomarker. Various controls can be used to provide a suitable baseline for comparison, and choosing suitable controls is routine in the diagnostic field. Further details of suitable controls are given below.

The measured level(s) of biomarker(s), after any compensation/normalisation/*etc*., can be transformed into a diagnostic result in various ways. This transformation may involve an algorithm which provides a diagnostic result as a function of the measured level(s). Where a panel is used then each individual biomarker may make a different contribution to the overall diagnostic result and so two biomarkers may be weighted differently.

The creation of algorithms for converting measured levels or raw data into scores or results is well known in the art. For example, linear or non-linear classifier algorithms can be used. These algorithms can be trained using data from any particular technique for measuring the marker(s). Suitable training data will have been obtained by measuring the biomarkers in "case" and "control" samples i.e. samples from subjects known to suffer from endometriosis and from subjects known not to suffer from endometriosis.

Most usefully the control samples will also include samples from subjects with a related disease which is to be distinguished from the disease of interest e.g. it is useful to train the algorithm with data from rheumatoid arthritis subjects and/or with data from subjects with connective tissue diseases other than endometriosis. The classifier algorithm is modified until it can distinguish between the case and control samples e.g. by adding or removing markers from the analysis, by changes in weighting, *etc.* Thus a method of the disclosure may include a step of analysing biomarker levels in a subject's sample by using a classifier algorithm which distinguishes between endometriosis subjects and non- endometriosis subjects based on measured biomarker levels in samples taken from such subjects.

Various suitable classifier algorithms are available *e*.*g*. linear discriminant analysis, naïve Bayes classifiers, perceptrons, support vector machines (SVM) [98] and genetic programming (GP) [99]. GP is particularly useful as it generally selects relatively small numbers of biomarkers and overcomes the problem of trapping in a local maximum which is inherent in many other classification methods. SVM-based approaches have previously been applied to endometriosis datasets [100]. The inventors have previously confirmed that both SVM and GP approaches can be trained on the same biomarker panels to distinguish the auto-antibody/antigen biomarker profiles of case and control cohorts with similar sensitivity and specificity i.e. auto-antibody biomarkers are not dependent on a single method of analysis. Moreover, these approaches can potentially distinguish endometriosis subjects from subjects with (i) other forms of autoimmune disease and (ii) rheumatoid arthritis. The biomarkers in Table 1 can be used to train such algorithms to reliably make such distinctions. The classification performance (sensitivity and specificity, ROC analysis) of any putative biomarkers can be rigorously assessed using nested cross validation and permutation analyses prior to further validation. Biological support for putative biomarkers can be sought using tools and databases including Genespring (version 11.5.1), Biopax pathway for GSEA analysis and Pathway Studio (version 9.1).

It will be appreciated that, although there may be some biomarkers in Table 1 which always give a negative absolute signal when contacted with negative control samples (and thus any positive signal is immediately indicative of endometriosis), it is more common that a biomarker will give at least a low absolute signal (and thus that a disease-indicating positive signal requires detection of auto-antibody levels above that background level). Thus references herein detecting a biomarker may not be references to absolute detection but rather (as is standard in the art) to a level above the levels seen in an appropriate negative control. Such controls may be assayed in parallel to a test sample but it can be more convenient to use an absolute control level based on empirical data, or to analyse data using an algorithm which can (e.g. by previous training) use biomarker levels to distinguish samples from disease patients vs. non-disease patients.

The level of a particular biomarker in a sample from an endometriosis-diseased subject may be above or below the level seen in a negative control sample. Antibodies that react with self-antigens occur naturally in healthy individuals and it is believed that these are necessary for survival of T- and B-cells in the peripheral immune system [101]. In a control population of healthy individuals there may thus be significant levels of circulating auto-antibodies against some of the antigens disclosed in Table 1 and these may occur at a significant frequency in the population. The level and frequency of these biomarkers may be altered in a disease cohort, compared with the control cohort. An analysis of the level and frequency of these biomarkers in the case and control populations may identify differences which provide diagnostic information. The level of auto-antibodies directed against a specific antigen may increase or decrease in an endometriosis sample, compared with a healthy sample.

When detecting combinations of protein and nucleic acid biomarkers, each class of biomarker is assayed and treated (e.g. data normalisation) as appropriate for that class, and then both the protein and the nucleic acid biomarkers are analysed together using an algorithm which classifies the sample.

In general, therefore, a method of the disclosure will involve determining whether a sample contains a biomarker level which is associated with endometriosis. Thus a method of the disclosure can include a step of comparing biomarker levels in a subject's sample to levels in (i) a sample from a patient with endometriosis and/or (ii) a sample from a patient without endometriosis. The comparison provides a diagnostic indicator of whether the subject has endometriosis. An aberrant level of one or more biomarker(s), as compared to known or standard expression levels of those biomarker(s) in a sample from a patient without endometriosis, indicates that the subject has endometriosis.

The level of a biomarker should be significantly different from that seen in a negative control. Advanced statistical tools (e.g. principal component analysis, unsupervised hierarchical clustering and linear modelling) can be used to determine whether two levels are the same or different. For example, an *in vitro* diagnosis will rarely be based on comparing a single determination. Rather, an appropriate number of determinations will be made with an appropriate level of accuracy to give a desired statistical certainty with an acceptable sensitivity and/or specificity. Antigen and/or antibody levels can be measured quantitatively to permit proper comparison, and enough determinations will be made to ensure that any difference in levels can be assigned a statistical significance to a level of *p*≤0.05 or better. The number of determinations will vary according to various criteria (e.g. the degree of variation in the baseline, the degree of up-regulation in disease states, the degree of noise, *etc.)* but, again, this falls within the normal design capabilities of a person of ordinary skill in this field. For example, interquartile differences of normalised data can be assessed, and the threshold for a positive signal (i.e. indicating the presence of a particular auto-antibody) can be defined as requiring that antibodies in a sample react with a diagnostic antigen at least 2.5-fold more strongly that the interquartile difference above the 75th percentile.

Other criteria are familiar to those skilled in the art and, depending on the assays being used, they may be more appropriate than quantile normalisation. Other methods to normalise data include data transformation strategies known in the art *e.g*. scaling, log normalisation, median normalisation, *etc.* For example, raw protein array data can be normalized by consolidating the replicates, transforming the data and applying median normalization which has been demonstrated to be appropriate for this type of analysis. Gene expression data can be subjected to background correction via 2D spatial correction and dye bias normalization via MvA lowess [102,103,104]. Normalized gene expression and proteomic data can be analysed for any potential signatures relating to differences between patient cohorts referring to levels of statistical significance (generally p < 0.05), multiple testing correction and fold changes within the expression data that could be indicative of biological effect (generally 2 fold in mRNA compared with a reference value).

The underlying aim of these data interpretation techniques is to distinguish between the presence of a Table 1 biomarker and of an arbitrary control biomarker, and also to distinguish between the response of sample from a endometriosis subject from a control subject. Methods of the disclosure may have sensitivity of at least 70% (*e.g.* >70%, >75%, >80%, >85%, >90%, >95%, >96%, >97%, >98%, >99%). Methods of the disclosure may have specificity of at least 70% (*e.g.* >70%, >75%, >80%, >85%, >90%, >95%, >96%, >97%, >98%, >99%). Advantageously, methods of the disclosure may have both specificity and sensitivity of at least 70% (*e.g.* >70%, >75%, >80%, >85%, >90%, >95%, >96%, >97%, >98%, >99%). As shown in the examples, the disclosure can consistently provide specificities above approximately 70% and sensitivities greater than approximately 70%.

Data obtained from methods of the disclosure, and/or diagnostic information based on those data, may be stored in a computer medium (*e.g*. in RAM, in non-volatile computer memory, on CD-ROM) and/or may be transmitted between computers *e.g*. over the internet.

If a method of the disclosure indicates that a subject has endometriosis, further steps may then follow. For instance, the subject may undergo confirmatory diagnostic procedures, such as those involving physical inspection of the subject, and/or may be treated with therapeutic agent(s) suitable for treating endometriosis.

Preferably, the biomarkers of the disclosure can determine if a subject has peritoneum endometriosis. Thus, the disclosure also provides a method for detecting peritoneum endometriosis. The method can use any of the biomarkers listed in any of Tables 16, 18 and 20-21, preferably in Table 16 or 18.

Alternatively, the biomarkers of the disclosure can determine if a subject has ovarian endometriosis, such as endometriomas. Thus, the disclosure also provides a method for detecting ovarian endometriosis. The method can use any of the biomarkers listed in any of Tables 17-21, preferably in Table 17 or 18.

### Monitoring the efficacy of therapy

As mentioned above, some methods of the disclosure involve testing samples from the same subject at two or more different points in time. In general, where the above text refers to the presence or absence of antibody(s), the disclosure also includes an increasing or decreasing level of the antibody(s) over time, or to a spread of antibodies in which additional antibodies or antibody classes are raised against a single auto-antigen. Methods which determine changes in antibody(s) over time can be used, for instance, to monitor the efficacy of a therapy being administered to the subject (e.g. in theranostics). The therapy may be administered before the first sample is taken, at the same time as the first sample is taken, or after the first sample is taken.

The disclosure can be used to monitor a subject who is receiving hormonal therapies. Hormonal treatments including the combine oral contraceptive pills, progesterone only pills and intrauterine progesterone (Levonorgestrel IUS), Progestins, Gestrinone, Danazol (despite its masculinisation side effects), GnRH analogues.

In related descriptions of the disclosure, the results of monitoring a therapy are used for future therapy prediction. For example, if treatment with a particular therapy is effective in reducing or eliminating disease symptoms in a subject, and is also shown to decrease levels of a particular auto-antibody in that subject, detection of that auto-antibody in another subject may indicate that this other subject will respond to the same therapy. Conversely, if a particular therapy was not effective in reducing or eliminating disease symptoms in a subject who had a particular auto-antibody or auto-antibody profile, detection of that auto-antibody or profile in another subject may indicate that this other subject will also fail to respond to the same therapy.

As described herein, the presence of auto-antibodies against a particular auto-antigen can be used as the basis of proposing or initiating a particular therapy. For instance, if it is known that levels of a particular auto-antibody can be reduced by administering a particular therapy then that auto-antibody's detection may suggest that the therapy should begin. Thus the disclosure is useful in a theranostic setting. Normally at least one sample will be taken from a subject before a therapy begins.

### Immunotherapy

Where the development of auto-antibodies to a newly-exposed auto-antigen is causative for a disease, early priming of the immune response can prepare the body to remove antigen-exposing cells when they arise, thereby removing the cause of disease before auto-antibodies develop dangerously. The auto-antigens listed in Table 2 are thus therapeutic targets for treating endometriosis. For example, one antigen known to be recognised by auto-antibodies is p53, and this protein is considered to be both a vaccine target and a therapeutic target for the modulation of cancer [105- 107].

Thus the disclosure provides a method for raising an antibody response in a subject, comprising eliciting to the subject an immunogen which elicits antibodies which recognise an auto-antigen listed in Table 2. The method is suitable for immunoprophylaxis of endometriosis.

The disclosure also provides an immunogen for use in medicine, wherein the immunogen can elicit antibodies which recognise an auto-antigen listed in Table 2. Similarly, the disclosure also provides the use of an immunogen in the manufacture of a medicament for immunoprophylaxis of endometriosis, wherein the immunogen can elicit antibodies which recognise an auto-antigen listed in Table 2.

As discussed above for detection antigens, the immunogen may be the auto-antigen itself or may comprise an amino acid sequence having identity and/or comprising an epitope from the auto-antigen. Thus the immunogen may comprise an amino acid sequence (i) having at least 90% (e.g. ≥91%, ≥92%, ≥93%, ≥94%, ≥95%, ≥96%, ≥97%, ≥98%, ≥99%) sequence identity to the relevant SEQ ID NO disclosed herein (i.e. any of SEQ ID NOs: 1-121), and/or (ii) comprising at least one epitope from the relevant SEQ ID NO disclosed herein (i.e. any of SEQ ID NOs: 1-121). Other immunogens may also be used, provided that they can elicit antibodies which recognise the auto-antigen of interest.

As an alternative to immunising a subject with a polypeptide immunogen, it is possible to administer a nucleic acid (e.g. DNA or RNA) immunogen encoding the polypeptide, for *in situ* expression in the subject, thereby leading to the development of an antibody response.

The immunogen may be delivered in conjunction (*e.g*. in admixture) with an immunological adjuvant. Such adjuvants include, but are not limited to, insoluble aluminium salts, water-in-oil emulsions, oil-in-water emulsions such as MF59 and AS03, saponins, ISCOMs, 3-O-deacylated MPL (3dMPL), immunostimulatory oligonucleotides (*e.g*. including one or more CpG motifs), bacterial ADP-ribosylating toxins and detoxified derivatives thereof, cytokines, chitosan, biodegradable microparticles, liposomes, imidazoquinolones, phosphazenes (*e*.*g*. endometriosisPP), aminoalkylglucosaminide phosphates, gamma inulins, *etc.* Combinations of such adjuvants can also be used. The adjuvant(s) may be selected to elicit an immune response involving CD4 or CD8 T cells. The adjuvant(s) may be selected to bias an immune response towards a TH1 phenotype or a TH2 phenotype.

The immunogen may be delivered by any suitable route. For example, it may be delivered by parenteral injection (e.g. subcutaneously, intraperitoneally, intravenously, intramuscularly), or mucosally, such as by oral (e.g. tablet, spray), topical, transdermal, transcutaneous, intranasal, ocular, aural, pulmonary or other mucosal administration.

The immunogen may be administered in a liquid or solid form. For example, the immunogen may be formulated for topical administration (e.g. as an ointment, cream or powder), for oral administration (e.g. as a tablet or capsule, as a spray, or as a syrup), for pulmonary administration (e.g. as an inhaler, using a fine powder or a spray), as a suppository or pessary, as drops, or as an injectable solution or suspension.

### RNA-based therapy

The miRNAs listed in Table 3 and 4 can be useful for RNA-based therapy, e.g., antisense therapy. There is literature precedent outlining the use of antisense therapy to manage cancer [108]. A synthetic nucleic acid complementary to a miRNA listed in Table 3 or Table 4 could be used to stimulate cell death of cancerous cells (either associated with endometriosis and/or aggressive endometriosis). Additionally, *in vivo* antisense therapy could be used to introduce a nucleic acid complementary to a miRNA listed in Table 3 or Table 4 to specifically bind to, and abrogate, overexpression of specific miRNA(s) associated with endometriosis and/or aggressive endometriosis.

Thus the disclosure provides a nucleic acid which hybridises to miRNA(s) listed in Table 3 or Table 4 *(i.e.* any of SEQ ID NOs: 122-343), and which is conjugated to a cytotoxic agent. The miRNA is preferably a human miRNA. Any suitable cytotoxic agent can be used. These conjugates miRNAs can be used in methods of therapy.

Thus the disclosure provides a complementary nucleic which recognises a miRNA listed in Table 3 or Table 4 for the purposes of RNA-based therapies, e.g. antisense therapy.

### Imaging

The biomarkers listed in Table 1 can be useful for imaging.

A labelled antibody against an auto-antigen listed in Table 2 can be injected *in vivo* and the distribution of the antigen can then be detected. This method may identify the source of the auto-antigen (e.g. an area in the body where there is a high concentration of the antigen), potentially offering early identification of a pathological condition. Imaging techniques can also be used to monitor the progress or remission of disease, or the impact of a therapy.

The disclosure also provides a labelled antibody which recognises an auto-antigen listed in Table 2. The antibody may be a human antibody, as discussed above. Any suitable label can be used e.g. quantum dots, spin labels, fluorescent labels, *etc.*

A labelled, synthetic nucleic complementary to a miRNA(s) listed in Table 3 or Table 4 could be used for the identification, in *ex vivo* (*e.g.* tissue samples taken from biopsies), and *in vivo* (*e.g.* magnetic resonance imaging (MRI), positron emission tomography (PET) computed tomography (CT) scans of patients) samples of miRNAs associated with endometriosis and/or aggressive endometriosis. This may potentially offer a method for the early identification of endometriosis and/or aggressive endometriosis. Imaging techniques can also be used to monitor the progress or remission of disease, or the impact of a therapy.

The miRNA listed in Table 3 or Table 4 can be useful for analysing tissue samples by staining e.g. using standard FISH. A fluorescently labelled nucleic acid, complementary in sequence to the miRNAs outlined in Table 3 or Table 4 can be contacted with a tissue sample to visualise the location of the miRNA. A single sample could be stained against multiple miRNAs, and these different miRNAs may be differentially labelled to enable them to be distinguished. As an alternative, a plurality of different samples can each be stained with a single, labelled miRNA.

Thus the disclosure provides a labelled nucleic acid which can hybridise to miRNA(s) listed in Table 3 or Table 4. The miRNA is preferably a human miRNA. Any suitable label can be used e.g. quantum dots, spin labels, fluorescent labels, dyes, *etc.* These labelled nucleic acids can be used in methods of *in vivo* and/or *in vitro* imaging.

### Alternative biomarkers

The disclosure refers to auto-antibody and antigen biomarkers, with assays of auto-antibodies against an antigen being used in preference to assays of the antigen itself. In addition to these biomarkers, however, the disclosure can be used with other biological manifestations of the Table 2 antigens. For example, the level of mRNA transcripts encoding a Table 2 antigen can be measured, particularly in tissues where that gene is not normally transcribed (such as in the potential disease tissue). Similarly, the chromosomal copy number of a gene encoding a Table 2 antigen can be measured e.g. to check for a gene duplication event. The level of a regulator of a Table 2 antigen can be measured e.g. to look at a microRNA regulator of a gene encoding the antigen. Furthermore, things which are regulated by or respond to a Table 2 antigen can be assessed e.g. if an antigen is a regulator of a metabolic pathway then disturbances in that pathway can be measured.

The disclosure also refers to miRNA biomarkers. In addition to these biomarkers, however, the disclosure can be used with other biological manifestations of the Table 3 and Table 4 miRNAs. For example, the expression level of mRNA transcripts which are a target of a Table 3 or a Table 4 miRNA can be measured, particularly in tissues where changes in transcription level can easily be determined (such as in the potential disease tissue). Similarly, the copy number variation of a chromosomal location of a Table 3 or a Table 4 miRNA can be measured e.g. to check for a chromosomal deletion or duplication events.

The level of a regulator of transcription for a Table 3 or a Table 4 miRNA can be measured e.g. the methylation status of the miRNA chromosomal region.

A single pre-miRNA precursor may lead to one or more mature miRNA sequences, such as sequences excised from the 5' and 3' arms of the hairpin. The disclosure can be used to look for other mature miRNA sequences from the same pre-miRNA precursor. For example, other mature miRNA sequences from the same precursor may be appropriate biomarkers as well.

Further possibilities will be apparent to the skilled reader.

### Preferred panels

Preferred descriptions of the disclosure are based on at least two different biomarkers i.e. a panel. Panels of particular interest consist of or comprise combinations of one or more biomarkers listed in Table 1, optionally in combination with at least 1 further biomarker(s) *e.g*. from Table 5 etc. Preferred panels have from 2 to 6 biomarkers in total. Panels of particular interest consist of or comprise the combinations of biomarkers listed in any of Tables 10 to 15. The panels useful for the disclosure (*e.g*. the panels listed in Tables 10 to 15) can be expanded by adding further (*i.e*. one or more) biomarker(s) to create a larger panel. The further biomarkers can usefully be selected from known biomarkers (as discussed above e.g. see Table 5). In general the addition does not decrease the sensitivity or specificity of the panel shown in the Tables. Such panels include, but are not limited to:
- A panel comprising a biomarker selected from Table 1.
- A panel comprising a biomarker selected from Table 2, Table 3 or Table 4.
- A panel comprising or consisting of 2 different biomarkers, namely: (i) a biomarker selected from Table 1 and (ii) a further biomarker selected from Table 2.
- A panel comprising or consisting of 2 different biomarkers, namely: (i) a biomarker selected from Table 1 and (ii) a further biomarker selected from Table 3.
- A panel comprising or consisting of 2 different biomarkers, namely: (i) a biomarker selected from Table 1 and (ii) a further biomarker selected from Table 4.
- A panel comprising or consisting of 3 different biomarkers, namely: (i) a group of 2 biomarkers selected from Table 11 and (ii) a further biomarker selected from Table 1.
- A panel comprising or consisting of 3 different biomarkers, namely: (i) a group of 2 biomarkers selected from Table 11 and (ii) a further biomarker selected from Table 2.
- A panel comprising or consisting of 3 different biomarkers, namely: (i) a group of 2 biomarkers selected from Table 11 and (ii) a further biomarker selected from Table 3.
- A panel comprising or consisting of 3 different biomarkers, namely: (i) a group of 2 biomarkers selected from Table 11 and (ii) a further biomarker selected from Table 4.
- A panel comprising or consisting of 4 different biomarkers, namely: (i) a group of 3 biomarkers selected from Table 12 and (ii) a further biomarker selected from Table 1.
- A panel comprising or consisting of 4 different biomarkers, namely: (i) a group of 3 biomarkers selected from Table 12 and (ii) a further biomarker selected from Table 2.
- A panel comprising or consisting of 4 different biomarkers, namely: (i) a group of 3 biomarkers selected from Table 12 and (ii) a further biomarker selected from Table 3.
- A panel comprising or consisting of 4 different biomarkers, namely: (i) a group of 3 biomarkers selected from Table 12 and (ii) a further biomarker selected from Table 4.
- A panel comprising or consisting of 5 different biomarkers, namely: (i) a group of 4 biomarkers selected from Table 13 and (ii) a further biomarker selected from Table 1.
- A panel comprising or consisting of 5 different biomarkers, namely: (i) a group of 4 biomarkers selected from Table 13 and (ii) a further biomarker selected from Table 2.
- A panel comprising or consisting of 5 different biomarkers, namely: (i) a group of 4 biomarkers selected from Table 13 and (ii) a further biomarker selected from Table 3.
- A panel comprising or consisting of 5 different biomarkers, namely: (i) a group of 4 biomarkers selected from Table 13 and (ii) a further biomarker selected from Table 4.
- A panel comprising or consisting of 6 different biomarkers, namely: (i) a group of 5 biomarkers selected from Table 14 and (ii) a further biomarker selected from Table 1.
- A panel comprising or consisting of 6 different biomarkers, namely: (i) a group of 5 biomarkers selected from Table 14 and (ii) a further biomarker selected from Table 2.
- A panel comprising or consisting of 6 different biomarkers, namely: (i) a group of 5 biomarkers selected from Table 14 and (ii) a further biomarker selected from Table 3.
- A panel comprising or consisting of 6 different biomarkers, namely: (i) a group of 5 biomarkers selected from Table 14 and (ii) a further biomarker selected from Table 4.
- A panel comprising or consisting of 7 different biomarkers, namely: (i) a group of 6 biomarkers selected from Table 15 and (ii) a further biomarker selected from Table 1.
- A panel comprising or consisting of 7 different biomarkers, namely: (i) a group of 6 biomarkers selected from Table 15 and (ii) a further biomarker selected from Table 2.
- A panel comprising or consisting of 7 different biomarkers, namely: (i) a group of 6 biomarkers selected from Table 15 and (ii) a further biomarker selected from Table 3.
- A panel comprising or consisting of 7 different biomarkers, namely: (i) a group of 6 biomarkers selected from Table 15 and (ii) a further biomarker selected from Table 4.
- A panel comprising or consisting of a group of 12 different biomarkers selected from Table 10. This panel is particularly useful for diagnosis.

Preferred panels have between 2 and 6 biomarkers in total.

A preferred panel comprises hsa-miR-150 and hsa-miR-574-5p. Another preferred panel comprises hsa-miR-342-3p and hsa-miR-574-5p. Another preferred panel comprises hsa-miR-150 and hsa-miR-342-3p. Another preferred panel comprises hsa-miR-150, hsa-miR-122 and hsa-miR-574-5p. Another preferred panel comprises TPM1, hsa-miR-150 and hsa-miR-574-5p.

Different biomarkers can have different relative differential expression profiles in an endometriosis sample compared to a control sample. Pairs of these biomarkers (i.e. where one is up-regulated and the other is down-regulated relative to the same control) may provide a useful way of diagnosing endometriosis. For example, the inventors found that ebv-miR-BART2-5p and hsa-miR-564 are up- and down-regulated, respectively, in endometriosis samples vs. control samples (healthy, non-endometriosis samples), so this pair would be useful. This divergent behaviour can enhance diagnosis of endometriosis when a pair of the biomarkers is assessed in the same sample.

Thus, a method of the disclosure can include a step of determining the expression levels of a first and a second biomarker of the disclosure in a subject's sample, wherein the first biomarker is up-regulated in an endometriosis sample compared to a non-endometriosis sample and the second biomarker is down-regulated in an endometriosis sample compared to the same non- endometriosis sample.

A method of the disclosure can include: (i) determining the expression level of a first biomarker of the disclosure in a subject's sample, (ii) determining the expression level of a second biomarker of the disclosure in the subject's sample, wherein the first biomarker is up-regulated in an endometriosis sample compared to a non-endometriosis sample and the second biomarker is down-regulated in an endometriosis sample compared to the same non-endometriosis sample, and (iii) comparing the determinations of (i) and (ii) with a non- endometriosis sample, an endometriosis sample and/or an absolute value, wherein the comparison provides a diagnostic indicator of whether the subject has endometriosis. Aberrant levels of the first and the second biomarkers, as compared to the known or standard expression levels of them in the non- endometriosis sample or endometriosis sample, and/or the absolute value, indicate that the subject has endometriosis.

### General

The term "comprising" encompasses "including" as well as "consisting" e.g. a composition "comprising" X may consist exclusively of X or may include something additional e.g. X + Y.

References to an antibody's ability to "bind" an antigen mean that the antibody and antigen interact strongly enough to withstand standard washing procedures in the assay in question. Thus non-specific binding will be minimised or eliminated.

An assay's "sensitivity" is the proportion of true positives which are correctly identified i.e. the proportion of endometriosis subjects with auto-antibodies against the relevant antigen who test positive.

An assay's "specificity" is the proportion of true negatives which are correctly identified by *i.e.* the proportion of subjects without endometriosis who test negative for antibodies against the relevant antigen. Unless specifically stated, a method comprising a step of mixing two or more components does not require any specific order of mixing. Thus components can be mixed in any order. Where there are three components then two components can be combined with each other, and then the combination may be combined with the third component, *etc.*

References to a percentage sequence identity between two amino acid sequences means that, when aligned, that percentage of amino acids are the same in comparing the two sequences. This alignment and the percent homology or sequence identity can be determined using software programs known in the art, for example those described in section 7.7.18 of ref. 109. A preferred alignment is determined by the Smith-Waterman homology search algorithm using an affine gap search with a gap open penalty of 12 and a gap extension penalty of 2, BLOSUM matrix of 62. The Smith-Waterman homology search algorithm is disclosed in ref. 110.

### BRIEF DESCRIPTION OF DRAWINGS

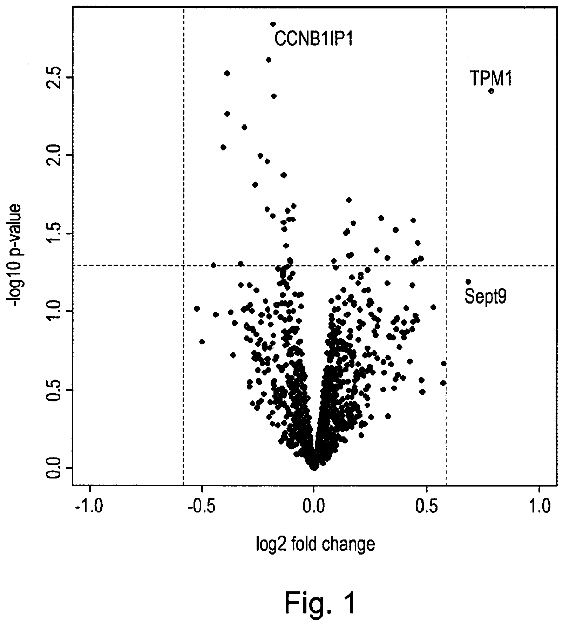

Figure 1 shows a volcano plot displaying the p-value of a microarray t-test on the y-axis versus the fold change in antibody levels between case and controls on the x-axis. The most interesting features can be found in the top left and top right area of the volcano plot. A dotted line is plotted in the graph to differentiate between potential markers and insignificant events. These cut-offs can be varied but a typical minimum selection criteria of a p-value less than 0.05 and a fold change of greater than log2 of 0.585 (1.5-fold) was used to identify candidate biomarkers. Global median normalised data and not raw data is used to derive the fold-change values. Large differences in raw RFUs translate to small changes in this value following normalisation. Several of the best-performing markers (CCNB1IP1, TPM1 and Sept9) in this analysis are indicated.
Figure 2 shows box plots for normalised data for: (A) RAN, (B) STUB1, (C) TPM1 and (D) HSPD1.
Figure 3 shows a volcano plot displaying the p-value of a microarray t-test on the y-axis versus the fold change in miRNA levels between case and controls on the x-axis. Several of the best-performing markers (hsa-miR-150, hsa-miR-122, hsa-miR-342-3p, hsa-miR-483-3p, hsa-miR-1290, hsa-miR-3194-5p, hsa-miR-3683-5p, hsa-miR-3937, hsa-miR-1224-5p and hsa-miR-3648) in this analysis are highlighted.
Figure 4 shows the hierarchical clustering of the significant miRNAs according to the type of tissue (i.e. case vs. control), where black signifies high expression, white signifies intermediate expression, and grey signifies low expression.
Figure 5 is a scatter plot showing the correlation between the data from the microarray and TaqMan® miRNA qPCR assays.
Figure 6 shows receiver operating characteristic (ROC) curves for individual miRNAs from the microarray data: (A) hsa-miR-150; sensitivity=0.8, specificity=0.65, AUC=0.8; (B) hsa-miR-574-5p; sensitivity=0.73, specificity=0.71, AUC=0.8; (C) hsa-miR-342-3p; sensitivity=0.66, specificity=0.76, AUC=0.78.
Figure 7 shows receiver operating characteristic (ROC) curves for panels from the microarray data: (A) hsa-miR-150 and hsa-miR-574-5p; sensitivity=0.86, specificity=0.76, AUC=0.91; (B) hsa-miR-342-3p and hsa-miR-574-5p; sensitivity=0.76, specificity=0.76, AUC=0.86; (C) hsa-miR-150 and hsa-miR-342-3p; sensitivity=0.68, specificity=0.76, AUC=0.81; (D) hsa-miR-150, hsa-miR-122 and hsa-miR-574-5p; sensitivity=0.83, specificity=0.76, AUC=0.9.
Figure 8 shows a receiver operating characteristic (ROC) curve for a combination of TPM1, hsa-miR-150 and hsa-miR-574-5p; sensitivity=0.9, specificity=0.75, AUC=0.92.
Figure 9 shows a Venn diagram depicting the miRNAs which are statistically significantly differentially expressed (P<0.05) in normal endometrium (NE), normal peritoneum (NP), peritoneum endometriosis (ES) and ovarian endometriomas (OvES). The number of miRNAs that were statistically significantly expressed in more than one group is indicated in overlapping areas corresponding to appropriate groups. Areas A-F are examples of such overlapping areas.
Figure 10 shows quantitative PCR results of ebv-miR-BART2-5p miRNA expression in endometriosis, normal endometrium from subjects having endometriosis and normal endometrium from subjects not having endometriosis.

### MODES FOR CARRYING OUT THE INVENTION

### Study 1

### 1. Detection of auto-antibodies in serum of subjects suffering from endometriosis

### a. Array preparation

The examples refer to use of a "functional protein" array technology which has the ability to display native, discontinuous epitopes [111,112]. Proteins are full-length, expressed with a folding tag in insect cells and screened for correct folding before being arrayed in a specific, oriented manner designed to conserve native epitopes. Each array contains approximately 1550 human proteins representing -1500 distinct genes chosen from multiple functional and disease pathways printed in quadruplicate together with control proteins. In addition to the proteins on each array, four control proteins for the BCCP-myc tag (BCCP, BCCP-myc, β-galactosidase-BCCP-myc and β-galactosidase-BCCP) were arrayed, along with additional controls including Cy3-labeled biotin-BSA, dilution series of biotinylated-IgG and biotinylated-IgM and buffer-only spots.

Incubation of the arrays with serum samples allows detection of binding of serum immunoglobulins to specific proteins on the arrays, enabling the identification of both autoantibodies and their cognate antigens [112].

### b. Biomarker confirmation

Serum samples were obtained from two groups of subjects:
1. serum samples from subjects diagnosed with endometriosis (n=36).
2. serum samples from age-matched healthy donors (n=35).

For autoantibody profiling, serum samples were incubated with arrays separately. All arrays were incubated for 2 hours at room temperature (RT, 20°C), followed by washing three times in fresh Triton-BSA buffer at RT for 20 minutes. The washed slides were incubated in a labelled anti-human IgG antibody at RT for 2 hours. Slides were washed three times in Triton-BSA buffer for 5 minutes at RT, rinsed, and centrifuged for 2 minutes at 240g.

The probed and dried arrays were scanned using an Agilent High-Resolution microarray scanner at 10µm resolution. The resulting 20-bit tiff images were feature extracted using Agilent's Feature Extraction software version 10.5 or 10.7.3.1. The microarray scans produced images for each array that were used to determine the intensity of fluorescence bound to each protein spot which were used to normalize and score array data.

Raw median signal intensity (also referred to as the relative fluorescent unit, RFU) of each protein feature (also referred to as a spot or antigen) on the array was subtracted from the local median background intensity. Alternative analyses use other measures of spot intensity such as the mean fluorescence, total fluorescence, as known in the art. The results of QC analyses showed that the platform performed well within expected parameters with relatively low technical variation.

The raw array data was normalized by consolidating the replicates (median consolidation), followed by normal transformation and then global median normalisation. Outliers were identified and removed. There is no method of normalisation which is universally appropriate and factors such as study design and sample properties must be considered. For the current study median normalisation was used. Other normalisation methods include, amongst others, SAM, quantile normalisation [113], multiplication of net fluorescent intensities by a normalisation factor consisting of the product of the 1st quartile of all intensities of a sample and the mean of the 1st quartiles of all samples and the "VSN" method [114]. Such normalisation methods are known in the art of microarray analysis.

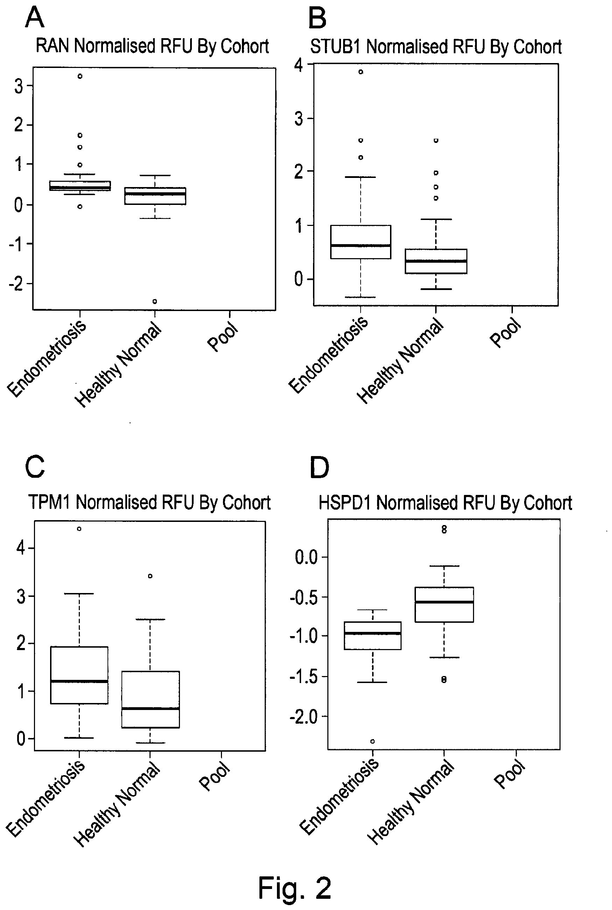

This normalised data was then used for the identification of individual candidate biomarkers and for the development of combinations of biomarkers ("panels"). Tools such as volcano plots (Figure 1), scatter plots (Figure 2) and boxplots were used to identify candidate biomarkers (Tables 6 and 8) with combinations of strong p-values and robust fold-changes when comparing case and control cohorts. Several proteins previously associated with endometriosis were identified (Table 5) including CDC42, EGFR, KIT, PPARG and WT1, thus validating this approach.

### 2. Detection of miRNA in serum of subjects suffering from endometriosis using microarrays

### a. Array preparation

For microarray fabrication and usage, Agilent Technologies' ("Agilent") miRNA microarray was used. The content of the microarray is continuously aligned with releases from the miRBase database [115, 116, 117, 118], representing all known miRNAs from human beings, as well as all know human viral miRNAs. These arrays are printed using Agilent's ink-jet *in situ* synthesis microarray fabrication machines.

### b. Biomarker confirmation

A set of 71 serum samples, sourced from patients with endometriosis ("case"; n=36) and normal ("control"; n=35) patients were processed to extract total RNA (including miRNA) using standard column filtration methodologies. The extracted serum samples were analysed using the Agilent miRNA microarray (G4870A-031181), according to their standard protocol, (manual part number G4170-90011, version 2.4). However, deviations from the standard protocol included labelling of the samples using 2.25 µl Cyanine 3-pCp, and hybridising the microarray slides for 44 hours.

The probed and dried arrays were then scanned using a microarray scanner capable of using an excitation wavelength suitable for the detection of the labelled miRNAs and to determine magnitude of miRNA binding to the complementary detection probe. The microarray scans produced images for each array that were used to determine the intensity of fluorescence bound to each oligonucleotide spot which were used to normalise and score array data.

The raw microarray scan image contains raw signal intensity (also referred to as the relative fluorescent unit, RFU) for each oligonucleotide spot (also referred to as a feature) on the array. These images were then feature extracted using Agilent's proprietary feature extraction software. Alternative analyses use other measures of spot intensity such as the mean fluorescence, total fluorescence, as known in the art.

The resulting average intensities of all oligonucleotide features on each array were then normalised to reduce the influence of technical bias (e.g. laser power variation, surface variation, input miRNA concentration, *etc.)* by a percentile normalisation procedure. Other methods for data normalisation suitable for the data include, amongst others, quantile normalisation [97]. Such normalisation methods are known in the art of microarray analysis.

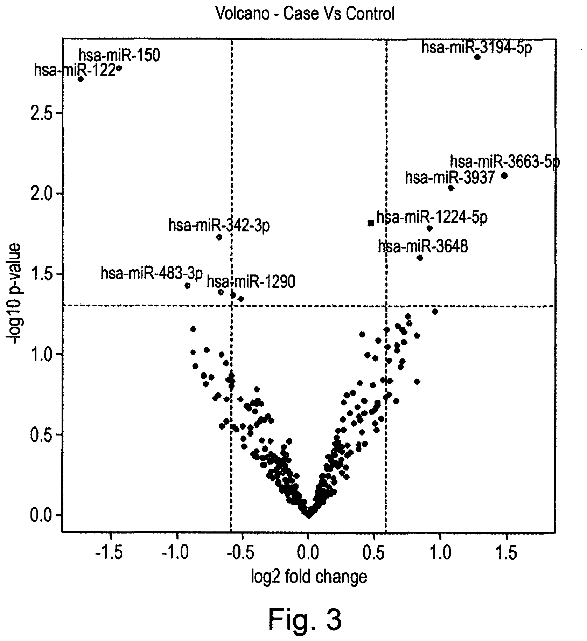

A linear model was fitted to evaluate statistical differences in miRNA expression between cases and controls. Volcano plot analysis of the miRNA microarray data is shown in Figure 3. On the volcano plot (Figure 3), the x-axis shows the log2 fold change between case and control and the y-axis shows statistical significance (negative log in base 10 of p). Dots above the horizontal dashed line are a selection of significant hits.

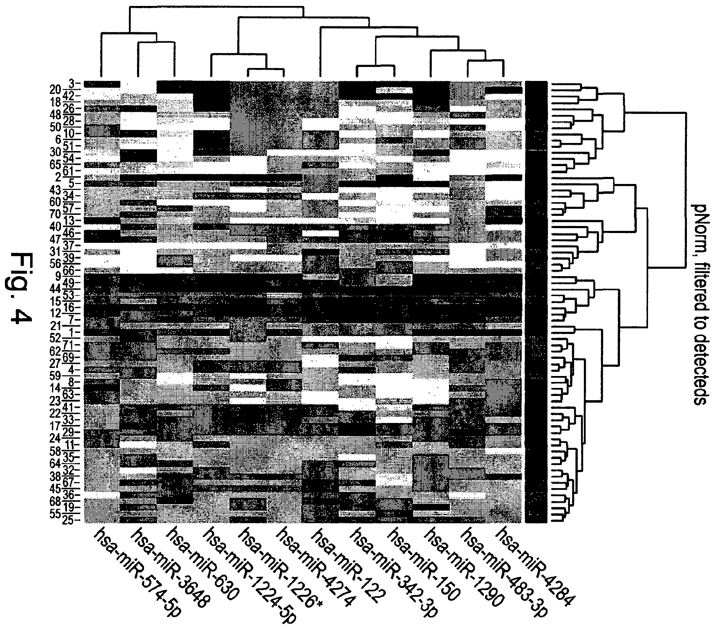

The hierarchical clustering of the significant miRNAs according to the type of tissue (i.e. case vs. control) is shown in Figure 4, where black signifies high expression, white signifies intermediate expression, and grey signifies low expression.

### 3. Validation of miRNA in serum of subjects suffering from endometriosis using qPCR

For quantitative PCR ("qPCR") usage, Life Technologies' ("LifeTech") TaqMan® miRNA assays were used. These assays are continuously aligned with releases from the miRBase database, representing all known miRNAs from human beings, as well as all know human viral miRNAs. These TaqMan® miRNA assays employ a novel target-specific stem-loop reverse transcription primer to address the challenge of the short length of mature miRNA. The primer extends the 3' end of the target to produce a template that can be used in standard TaqMan® assay-based real-time PCR. Also, the stem-loop structure in the tail of the primer confers a key advantage to these assays: specific detection of the mature, biologically active miRNA.

Using the significant markers identified in the miRNA microarray experiments, a sub-selection of miRNAs were analysed using the LifeTech TaqMan® miRNA qPCR assays, according to their standard protocol, (manual part number 4465407, revision date 30^{th} March 2012 (Rev. B)).

The TaqMan® miRNA assays were scanned using a ViiA™ 7 Real-Time PCR System using an excitation wavelength suitable for the detection of the labelled miRNAs (for example, but not limited to, 6-FAM™ Dye). The qPCR scans produced traces for each TaqMan® miRNA assay which can be used, if applicable, to determine the amount of specific miRNA within a given sample, relative to a passive reference dye (for example, but not limited to, ROX).

The raw qPCR traces contain raw signal intensity (also referred to as ΔRn) to which an assay threshold (horizontal line) is applied after the raw traces have been baseline normalised, which is necessary to remove aberrant signal. This threshold line intersects the qPCR trace at the point on the qPCR trace where the trace is logarithmic. From this, the qPCR cycle (Ct) can be determined. These qPCR traces are analysed using LifeTech's proprietary analysis software. Alternative analyses and analysis techniques are known in the art.

The median Ct and mean quantity was taken across the three sample replicates for each TaqMan® miRNA assay. Testing for statistically significant associations between the two groups (case vs control) was carried out by applying linear models to the normalised sample data to identify general miRNA changes between case samples and control samples. Statistical differences were calculated using a t-test.

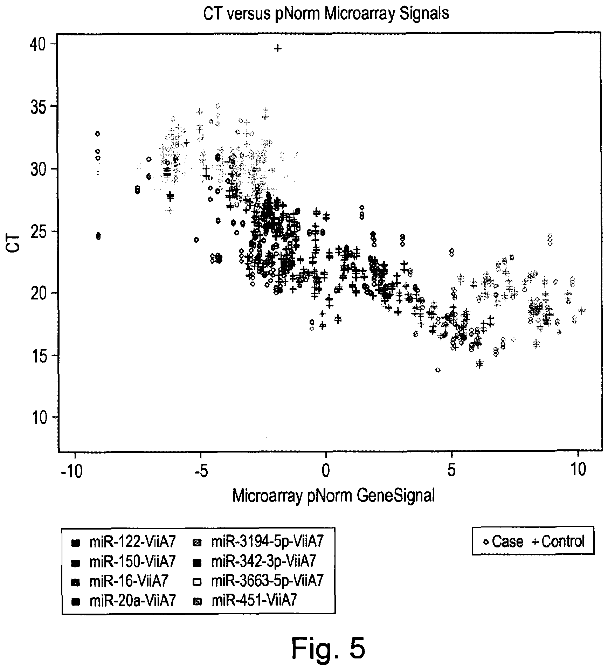

The data from the microarray and TaqMan® miRNA qPCR assays were analysed for Pearson correlation to assess the cross-platform robustness of the observed results (Figure 5). Figure 5 demonstrates that there is good correlation between a sub-set of the miRNA probes used for qPCR compared to those identified on the microarrays.

### 4. Multivariate analysis: combination of miRNA and autoantibody biomarkers in serum of subjects suffering from endometriosis

Panels of putative biomarkers were developed consisting of either autoantibodies alone, miRNAs alone or combinations containing both autoantibodies and miRNA species. Multivariate analysis was also performed incorporating data for galectin-3 and CA125 as variables however their inclusion did not improve on the performance of the miRNA and autoantibodies identified here. It is not possible to predict a *priori* which classifier will perform best with a given dataset, therefore data analysis was performed with 5 different feature ranking methods (1-5) plus forward and backward feature selection:
1. Entropy
2. Bhattacharyya
3. T-test
4. Wilcoxon
5. ROC
6. Forward selection
7. Backward selection

Other classification methods as known in the art could be used. Classifiers were then assessed for performance by referring to the combined sensitivity and specificity (S+S score) and area under the curve (AUC). Data were repeatedly split and analysis cycles repeated until a stable set of classifiers ("panels") was identified. Nested cross validation was applied to the classification procedures in order to avoid overfitting of the study data. The performance of the classification was compared to a randomized set of case-control status samples (permutation assay) which should give no predictive performance and provides an indication of the background in the analysis. A figure close to 1.0 is expected for the null assay (equivalent to a sensitivity + specificity (S+S) score of 0.5 + 0.5, respectively) whereas an S+S score of 2.0 would indicate 100% sensitivity and 100% specificity. The difference between the values for the permutation analysis and the classifier performance indicates the relative strength of the classifier. The antigens and miRNAs identified from this study are provided in Tables 2 and 3.

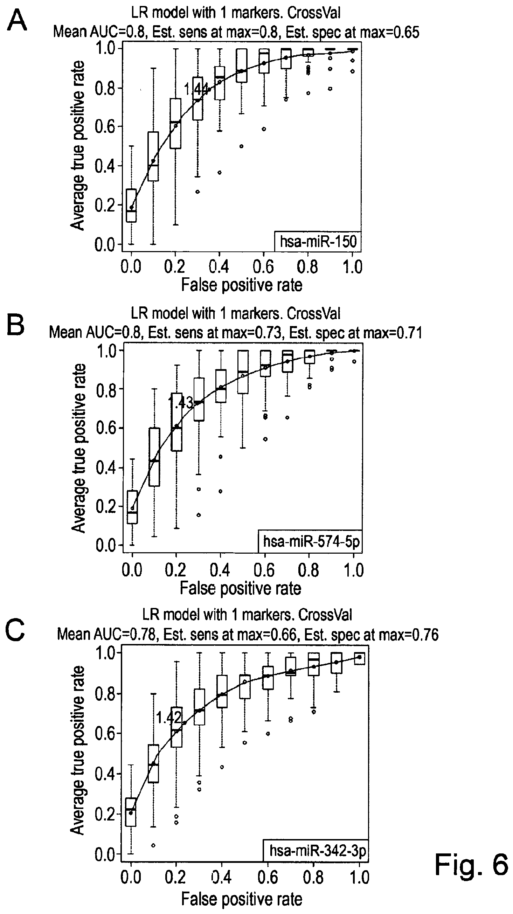

Table 6 shows the protein biomarkers that provided good performance, as judged by p value, fold-change, sensitivity, specificity, AUC. The best performing protein biomarkers are shown in Table 7. Table 8 shows the miRNA biomarkers that provided good performance, as judged by p value, fold-change, sensitivity, specificity, AUC. The best performing miRNA biomarkers are shown in Table 9. The ROC curves for some of the best performing miRNA biomarkers (hsa-miR-150, hsa-miR-574-5p and hsa-miR-342-3p) are shown in Figure 6. The best performing protein and miRNA biomarkers are shown in Table 10. These represent biomarkers of particular interest as they correspond to the subset of biomarkers with the greatest predictive properties.

The analysis methods described above were used to build, test and identify combinations of biomarkers with greater sensitivity, specificity or AUC than the individual biomarkers disclosed in Table 1.

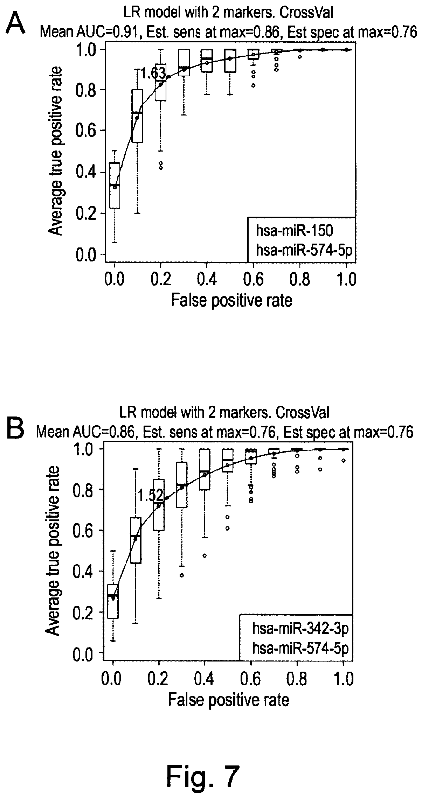

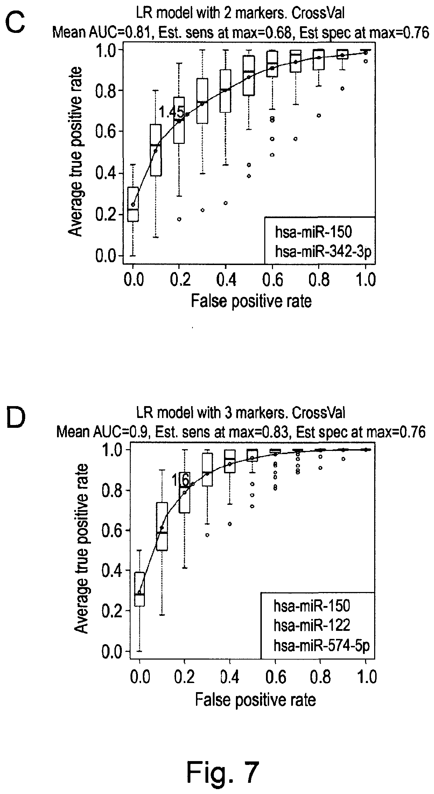

For each analysis, multiple combinations of putative biomarkers were derived and the performance of the derived panels was then ranked (Tables 11-15). Tables 11-15 show 2-mer, 3-mer, 4-mer, 5-mer and 6-mer panels that provide good performance. The ROC curves for some of the best performing combinations are shown in Figure 7 (A=2-mer panel of hsa-miR-150 and hsa-miR-574-5p; B=2-mer panel of hsa-miR-342-3p and hsa-miR-574-5p; C=2-mer panel of hsa-miR-150 and hsa-miR-342-3p; D=3-mer panel of hsa-miR-150, hsa-miR-122 and hsa-miR-574-5p).

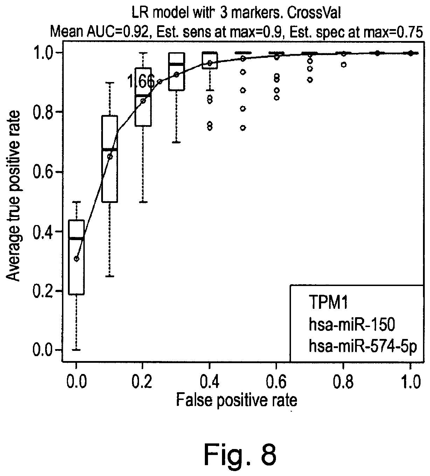

The biomarkers with the greatest diagnostic power, as judged by p value, fold-change, sensitivity, specificity, AUC and / or frequency of appearance in the panels derived were identified and combined into a single list of antigens and miRNAs. Figure 8 show the ROC curve for a 3-mer panel of TPM1, hsa-miR-150 and hsa-miR-574-5p, which is one of the best performing combinations. Thus, panels containing a mixture of protein and miRNA biomarkers also provide good diagnostic performance.

### Study 2

### 1. Detection of miRNA in tissue samples of subjects suffering from endometriosis using NanoString Technology

The fresh tissue samples used in this study include: peritoneal endometriosis (ES), ovarian endometriomas (OvES), normal endometrium (NE) and normal peritoneum (NP).

### miRNA extraction

Total RNA from specimens was extracted using the mirVana™ PARIS™ kit (Ambion) as per their supplied protocol. RNA quantification and integrity was assessed using the Eukaryote total RNA nano assay by Aligent Technologies™. Following validation, 100ng of total RNA was used in the nCounter® miRNA Expression Assay (NanoString Technologies) enabling an ultrasensitive miRNA detection in total RNA across all biological levels of expression without the use of reverse transcription or amplification. 735 human and human-associated viral miRNAs derived from miRBase were scanned. Unique multiplexed annealing of specific oligonucleotide tags were ligated onto their target miRNA followed by an enzymatic purification to remove all unligated tags. Excess unbound probes and RNA were washed using a two-step magnetic bead-based purification system on the nCounter Prep Station. Remaining miRNA was attached to a cartridge surface and polarised. The Cartridge was scanned and data collection was performed on the nCounter digital analyser. Digital images were processed and miRNA counts were tabulated.

### Statistical analysis

The R project (R version 2.12.1) (http://www.R-project.org) was used for statistical and clustering analysis. Quantified gene expression signal levels derived from the nCounter® miRNA Expression Assay (NanoString Technologies) were logarithmically transformed (base 2) and quantile-normalized prior to further exploration. In order to determine miRNA expression detectability threshold the log2 signal values were ranked in increasing order and binned into 0.5 expression level categories. Subsequently, the differences in successive expression level frequencies were calculated and the expression value following the most significant signal increase was considered as miRNA expression detectability threshold. miRNA expressions at any level (above log2 expression value of 3.25) were counted in each group and subsequently chi-square test was performed to assess if the numbers are significantly different from expected average. The global expression of miRNAs between all groups was not noted to be significantly different (chi square p-value 0.82664147).

Analysis of variance (ANOVA) was performed per each gene across all samples assigned to appropriate groups in order to identify differentially expressed miRNAs. The Benjamini & Hochberg multiple hypothesis testing correction was applied to control the false discovery rate (FDR). Genes that remained statistically significant (corrected p-value < 0.05) were selected for unsupervised hierarchical clustering performed based on distances calculated by means of Spearman correlation coefficient and ward agglomeration method.

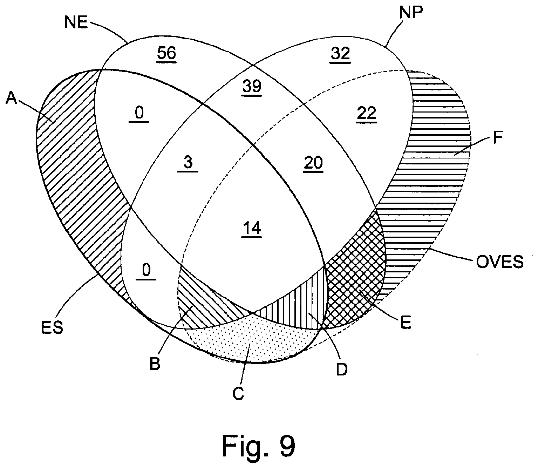

Subsequently, pairwise t-tests with corrections for multiple testing were applied as a post hoc analysis. T-test was performed between a particular sample group and all other sample groups. For example, ES samples were compared against all other groups (OvES, NP and NE), OvES samples were compared against all other groups (ES, NP and NE) *etc.* Computed t-statistics and signal intensity fold changes were further used to determine significantly up- and down-regulated miRNAs in each sample group and to generate Venn diagrams depicting the overlap between miRNAs differentially expressed in investigated groups (Figure 9).

Area A in Figure 9 shows that the levels of 2 miRNAs (ebv-miR-BART2-5p and hsa-miR-564, also listed in Table 16) are significantly different in ES samples, compared to that in OvES, NE and NP samples. Thus, these miRNAs are particularly useful for detecting peritoneum endometriosis. These miRNAs are particularly useful in practice because peritoneum endometriosis is relatively more difficult to detect using conventional techniques, e.g. ultrasound or MRI, compared to endometriomas.

Area C in Figure 9 shows that the level of 1 miRNA (listed in Table 18) is significantly different in ES and OvES samples compared to that in NE and NP samples. Thus, these miRNAs are particularly useful for detecting endometriosis, irrespective of where the endometriosis is.

Area F in Figure 9 shows that the levels of the miRNAs listed in Table 17 are significantly different in OvES samples compared to that in ES, NE and NP samples. Thus, these miRNAs are particularly useful for detecting endometriosis in the ovaries, e.g. endometriomas.

Area B in Figure 9 shows that the levels of the miRNAs listed in Table 20 are significantly different in ES, OvES and NP samples compared to that in NE samples.

Area D in Figure 9 shows that the levels of 2 miRNAs (listed in Table 21) are significantly different in ES, OvES and NE samples compared to that in NP samples.

Area E in Figure 9 shows that the levels of the miRNAs listed in Table 19 are significantly different in OvES and NE samples compared to that in ES and NP samples.

### 2. Validation of miRNA in tissue samples of subjects suffering from endometriosis using qPCR

PCR was performed to validate the microarray data. TaqMan® MicroRNA Assays (Applied Biosystems) were used.

cDNA was synthesized from total RNA using a commercially available specific EBV-miR-bart2-5p assay. RNU-44 and hsa-miR-26b were used as the mature miRNA endogenous controls. The miRNA 26b is a commonly expressed vertebrate miRNA and was used as a control primer for the reactions. Real time PCR amplification was performed on triplicates of each sample using the TaqMan2X Universal PCR Master Mix (Applied Biosystems).

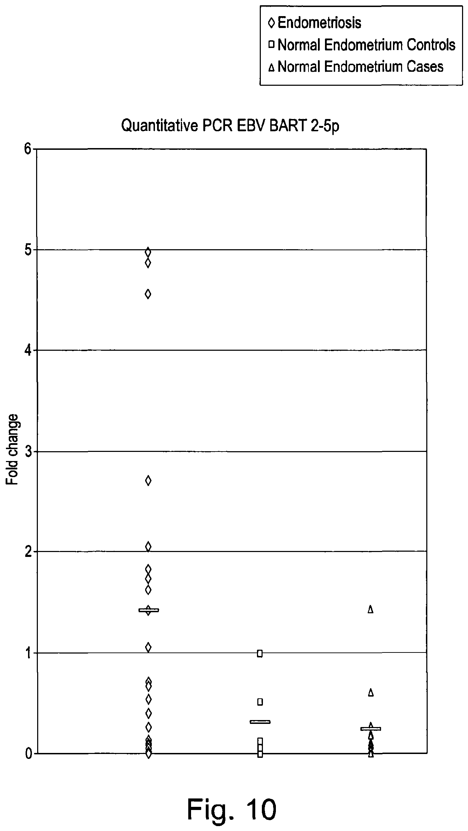

Significant differential expression of the ebv-miR-BART2-5p miRNA was observed between normal endometrium from controls, normal endometrium from subjects having endometriosis and endometriosis (Figure 10). The level of expression of ebv-miR-BART2-5p in normal endometrium from non-endometriosis subjects is significantly different from that in endometriosis (p=0.0067). There was no statistical significance between normal endometrial samples from subjects having endometriosis and normal endometrial samples from non-endometriosis subjects (p=0.02).

**TABLE 1: Biomarkers useful with the invention**

| **No. ⁽ⁱ⁾** | **Symbol ⁽ⁱⁱ⁾** | **Name ⁽ⁱⁱⁱ⁾** | **Type** |
|---|---|---|---|
| 1. | ACTB | Homo sapiens actin beta | Auto-antigen |
| 2. | ADD1 | Homo sapiens adducin 1 (alpha) | Auto-antigen |
| 3. | ADSL | Homo sapiens adenylosuccinate lyase | Auto-antigen |
| 4. | AK2 | Homo sapiens adenylate kinase 2 transcript variant AK2A | Auto-antigen |
| 5. | KLK3 | Homo sapiens kallikrein 3 (prostate specific antigen; transcript variant 1 | Auto-antigen |
| 6. | ATF1 | Homo sapiens activating transcription factor 1 | Auto-antigen |
| 7. | CKM | Homo sapiens creatine kinase muscle | Auto-antigen |
| 8. | CLK2 | Homo sapiens CDC-like kinase 2 transcript variant phclk2 | Auto-antigen |
| 9. | DDB2 | Homo sapiens damage-specific DNA binding protein 2 48kDa | Auto-antigen |
| 10. | DTYMK | Homo sapiens deoxythymidylate kinase (thymidylate kinase) | Auto-antigen |
| 11. | DUSP4 | Homo sapiens dual specificity phosphatase 4 transcript variant 1 | Auto-antigen |
| 12. | E2F6 | Homo sapiens E2F transcription factor 6 | Auto-antigen |
| 13. | EXT2 | Homo sapiens exostoses (multiple) 2 | Auto-antigen |
| 14. | FGFR4_ext | Homo sapiens fibroblast growth factor receptor 4 transcript variant 3 | Auto-antigen |
| 15. | FIGF | Homo sapiens c-fos induced growth factor (vascular endothelial growth factor D) | Auto-antigen |
| 16. | FKBP3 | Homo sapiens FK506 binding protein 3 25kDa | Auto-antigen |
| 17. | GALK1 | Homo sapiens galactokinase 1 | Auto-antigen |
| 18. | GK2 | Homo sapiens glycerol kinase 2 | Auto-antigen |
| 19. | GRB7 | Homo sapiens growth factor receptor-bound protein 7 | Auto-antigen |
| 20. | GTF2B | Homo sapiens general transcription factor IIB | Auto-antigen |
| 21. | GTF2H1 | Homo sapiens general transcription factor IIH polypeptide 1 62kDa | Auto-antigen |
| 22. | GTF2H2 | Homo sapiens general transcription factor IIH polypeptide 2 44kDa | Auto-antigen |
| 23. | HSPD1 | Homo sapiens heat shock 60kDa protein 1 (chaperonin) | Auto-antigen |
| 24. | IDI1 | Homo sapiens isopentenyl-diphosphate delta isomerase | Auto-antigen |
| 25. | IFI16 | Homo sapiens interferon, gamma-inducible protein 16, | Auto-antigen |
| 26. | LDHA | Homo sapiens lactate dehydrogenase A | Auto-antigen |
| 27. | LYL1 | Homo sapiens lymphoblastic leukemia derived sequence 1 | Auto-antigen |
| 28. | MARK3 | Homo sapiens MAP/microtubule affinity-regulating kinase 3 | Auto-antigen |
| 29. | MPP3 | Homo sapiens membrane protein palmitoylated 3 (MAGUK p55 subfamily member 3) | Auto-antigen |
| 30. | TRIM37 | Homo sapiens tripartite motif-containing 37, | Auto-antigen |
| 31. | NCF2 | Homo sapiens neutrophil cytosolic factor 2 (65kDa, chronic granulomatous disease, autosomal 2), | Auto-antigen |
| 32. | RPL10A | Homo sapiens ribosomal protein L10a | Auto-antigen |
| 33. | NFYA | Homo sapiens nuclear transcription factor Y alpha | Auto-antigen |
| 34. | NRAS | Homo sapiens neuroblastoma RAS viral (v-ras) oncogene homolog | Auto-antigen |
| 35. | NTRK3_ext | Homo sapiens neurotrophic tyrosine kinase receptor type 3 transcript variant 3 | Auto-antigen |
| 36. | OAS2 | Homo sapiens 2'-5'-oligoadenylate synthetase 2 69/71 kDa | Auto-antigen |
| 37. | endometriosis | Homo sapiens pyruvate carboxylase | Auto-antigen |
| 38. | CDK17 | Homo sapiens endometriosisTAIRE protein kinase 2 | Auto-antigen |
| 39. | PDE4A | Homo sapiens Homo sapiens phosphodiesterase 4A, cAMP-specific (phosphodiesterase E2 dunce homolog, Dro | Auto-antigen |
| 40. | PHF1 | Homo sapiens PHD finger protein 1 transcript variant 2 | Auto-antigen |
| 41. | PKM2 | Homo sapiens pyruvate kinase muscle transcript variant 1 | Auto-antigen |
| 42. | MAP2K5 | Homo sapiens mitogen-activated protein kinase kinase 5, transcript variant A | Auto-antigen |
| 43. | PRPS2 | Homo sapiens phosphoribosyl pyrophosphate synthetase 2 | Auto-antigen |
| 44. | RAN | Homo sapiens RAN member RAS oncogene family | Auto-antigen |
| 45. | RB1 | Homo sapiens retinoblastoma 1 (including osteosarcoma) | Auto-antigen |
| 46. | RBMS1 | Homo sapiens Homo sapiens RNA binding motif single stranded interacting protein 1 transcript variant | Auto-antigen |
| 47. | RET_a | Homo sapiens ret proto-oncogene (multiple endocrine neoplasia and medullary thyroid carci | Auto-antigen |
| 48. | RORC | Homo sapiens RAR-related orphan receptor C | Auto-antigen |
| 49. | RPL18 | Homo sapiens ribosomal protein L18 | Auto-antigen |
| 50. | RPL18A | Homo sapiens ribosomal protein L18a | Auto-antigen |
| 51. | RPL28 | Homo sapiens ribosomal protein L28 | Auto-antigen |
| 52. | RPL31 | Homo sapiens ribosomal protein L31 | Auto-antigen |
| 53. | RPL32 | Homo sapiens ribosomal protein L32 | Auto-antigen |
| 54. | S100A6 | Homo sapiens S100 calcium binding protein A6 (calcyclin) | Auto-antigen |
| 55. | SCP2 | Homo sapiens sterol carrier protein 2 transcript variant 2 | Auto-antigen |
| 56. | SIAH1 | Homo sapiens seven in absentia homolog 1 (Drosophila) transcript variant 2 | Auto-antigen |
| 57. | SMARCD1 | Homo sapiens SWI/SNF related matrix associated actin dependent regulator of chromatin subfamily d member 1 | Auto-antigen |
| 58. | SMARCE1 | Homo sapiens SWI/SNF related matrix associated actin dependent regulator of chromatin | Auto-antigen |
| 59. | SOD2 | Homo sapiens superoxide dismutase 2 mitochondrial | Auto-antigen |
| 60. | SOX2 | Homo sapiens SRY (sex determining region Y)-box 2 | Auto-antigen |
| 61. | SRPK1 | Homo sapiens SFRS protein kinase 1 | Auto-antigen |
| 62. | TPM1 | Homo sapiens tropomyosin 1 (alpha) | Auto-antigen |
| 63. | NR2C1 | Homo sapiens nuclear receptor subfamily 2 group C member 1 | Auto-antigen |
| 64. | TRIP6 | Homo sapiens thyroid hormone receptor interactor 6, | Auto-antigen |
| 65. | UBA1 | Homo sapiens Homo sapiens ubiquitin-activating enzyme E1 (A1S9T and BN75 temperature sensitivity compl | Auto-antigen |
| 66. | VCL | Homo sapiens vinculin | Auto-antigen |
| 67. | ZNF41 | Homo sapiens zinc finger protein 41 transcript variant 2 | Auto-antigen |
| 68. | PAX8 | Homo sapiens paired box gene 8 transcript variant PAX8A | Auto-antigen |
| 69. | NRIP1 | Homo sapiens nuclear receptor interacting protein 1 | Auto-antigen |
| 70. | PIP4K2B | Homo sapiens phosphatidylinositol-4-phosphate 5-kinase type II beta transcript variant 2 | Auto-antigen |
| 71. | UXT | Homo sapiens ubiquitously-expressed transcript | Auto-antigen |
| 72. | API5 | Homo sapiens apoptosis inhibitor 5 | Auto-antigen |
| 73. | MKNK1 | Homo sapiens MAP kinase-interacting serine/threonine kinase 1 | Auto-antigen |
| 74. | SUCLA2 | Homo sapiens succinate-CoA ligase ADP-forming beta subunit | Auto-antigen |
| 75. | LDB1 | Homo sapiens LIM domain binding 1 | Auto-antigen |
| 76. | NAE1 | Homo sapiens amyloid beta precursor protein binding protein 1 transcript variant 1 | Auto-antigen |
| 77. | PAPSS2 | Homo sapiens 3'-phosphoadenosine 5'-phosphosulfate synthase 2 | Auto-antigen |
| 78. | USP10 | Homo sapiens ubiquitin specific protease 10 | Auto-antigen |
| 79. | PRPF4 | Homo sapiens Homo sapiens PRP4 pre- | Auto-antigen |
| 80. | AIM2 | Homo sapiens absent in melanoma 2 | Auto-antigen |
| 81. | TBPL1 | Homo sapiens TBP-like 1 | Auto-antigen |
| 82. | TRAF4 | Homo sapiens TNF receptor-associated factor 4 transcript variant 1 | Auto-antigen |
| 83. | SOCS5 | Homo sapiens suppressor of cytokine signaling 5 | Auto-antigen |
| 84. | ZSCAN12 | Homo sapiens zinc finger protein 305 | Auto-antigen |
| 85. | HDAC4 | Homo sapiens cDNA | Auto-antigen |
| 86. | KIAA0101 | Homo sapiens KIAA0101 gene product | Auto-antigen |
| 87. | IP6K1 | Homo sapiens inositol hexaphosphate kinase 1 | Auto-antigen |
| 88. | RNF40 | Homo sapiens ring finger protein 40 transcript variant 1 | Auto-antigen |
| 89. | GPHN | Homo sapiens gephyrin | Auto-antigen |
| 90. | HRSP12 | Homo sapiens translational inhibitor protein p14.5 | Auto-antigen |
| 91. | STUB1 | Homo sapiens STIP1 homology and U-Box containing protein 1 | Auto-antigen |
| 92. | TRAIP | Homo sapiens TRAF interacting protein | Auto-antigen |
| 93. | PLK4 | Homo sapiens serine/threonine kinase 18 | Auto-antigen |
| 94. | CCNI | Homo sapiens cyclin I | Auto-antigen |
| 95. | IL24 | Homo sapiens interleukin 24 transcript variant 1 | Auto-antigen |
| 96. | CDK20 | Homo sapiens cell cycle related kinase | Auto-antigen |
| 97. | PABendometriosis1 | Homo sapiens poly(A) binding protein cytoplasmic 1 | Auto-antigen |
| 98. | MED4 | Homo sapiens vitamin D receptor interacting protein | Auto-antigen |
| 99. | NME7 | Homo sapiens non-metastatic cells 7 protein expressed in (nucleoside-diphosphate kinase) transcript variant 1 | Auto-antigen |
| 100. | PHF11 | Homo sapiens PHD finger protein 11 | Auto-antigen |
| 101. | IRAK4 | Homo sapiens interleukin-1 receptor-associated kinase 4 mRNA (cDNA clone MGC:13330 ) | Auto-antigen |
| 102. | TXNDC3 | Homo sapiens thioredoxin domain containing 3 (spermatozoa) | Auto-antigen |
| 103. | TAOK3 | Homo sapiens STE20-like kinase | Auto-antigen |
| 104. | STYXL 1 | Homo sapiens dual specificity phosphatase 24 (putative) | Auto-antigen |
| 105. | ASB1 | Homo sapiens ankyrin repeat and SOCS box-containing 1 | Auto-antigen |
| 106. | MST4 | Homo sapiens Mst3 and SOK1-related kinase (MASK) | Auto-antigen |
| 107. | PELO | Homo sapiens pelota homolog (Drosophila) | Auto-antigen |
| 108. | ETNK2 | Homo sapiens ethanolamine kinase 2 | Auto-antigen |
| 109. | RFK | Homo sapiens riboflavin kinase | Auto-antigen |
| 110. | C9orf86 | Homo sapiens chromosome 9 open reading frame 86 | Auto-antigen |
| 111. | DDX55 | Homo sapiens DEAD (Asp-Glu-Ala-Asp) box polypeptide 55 | Auto-antigen |
| 112. | CCNB1IP1 | Homo sapiens cDNA | Auto-antigen |
| 113. | KLHL12 | Homo sapiens kelch-like protein C3IP1 | Auto-antigen |
| 114. | SAV1 | Homo sapiens salvador homolog 1 (Drosophila) | Auto-antigen |
| 115. | CAMKV | Homo sapiens hypothetical protein MGC8407 | Auto-antigen |
| 116. | NSBP1 | Homo sapiens nucleosomal binding protein 1, | Auto-antigen |
| 117. | ALPK1 | Homo sapiens alpha-kinase 1 mRNA (cDNA clone MGC:71554 ) | Auto-antigen |
| 118. | ELMOD3 | Homo sapiens RNA binding motif and ELMO domain 1 | Auto-antigen |
| 119. | AIFM2 | Homo sapiens apoptosis-inducing factor (AIF)-like mitochondrion-associated inducer of death | Auto-antigen |
| 120. | RHOT2 | Homo sapiens ras homolog gene family member T2 | Auto-antigen |
| 121. | PYGO2 | Homo sapiens pygopus 2 | Auto-antigen |
| 122. | ebv-miR-BART12 | ebv-miR-BART12 | miRNA |
| 123. | ebv-miR-BART14 | ebv-miR-BART14 | miRNA |
| 124. | ebv-miR-BART16 | ebv-miR-BART16 | miRNA |
| 125. | ebv-miR-BART20-5p | ebv-miR-BART20-5p | miRNA |
| 126. | ebv-miR-BART2-5p | ebv-miR-BART2-5p | miRNA |
| 127. | MIRLET7B | hsa-let-7b* | miRNA |
| 128. | MIRLET7F1 | hsa-let-7f | miRNA |
| 129. | MIRLET7F2 | hsa-let-7f-1* | miRNA |
| 130. | MIRLET7G | hsa-let-7g | miRNA |
| 131. | MIR103A1 | hsa-miR-103a | miRNA |
| 132. | MIR10b | hsa-miR-10b | miRNA |
| 133. | MIR1183 | hsa-miR-1183 | miRNA |
| 134. | MIR1202 | hsa-miR-1202 | miRNA |
| 135. | MIR1207 | hsa-miR-1207-5p | miRNA |
| 136. | MIR122 | hsa-miR-122 | miRNA |
| 137. | MIR1224 | hsa-miR-1224-5p | miRNA |
| 138. | MIR1225 | hsa-miR-1225-3p | miRNA |
| 139. | MIR1225 | hsa-miR-1225-5p | miRNA |
| 140. | MIR1226 | hsa-miR-1226* | miRNA |
| 141. | MIR1228 | hsa-miR-1228* | miRNA |
| 142. | MIR1234 | hsa-miR-1234 | miRNA |
| 143. | MIR1237 | hsa-miR-1237 | miRNA |
| 144. | MIR1238 | hsa-miR-1238 | miRNA |
| 145. | MIR125a | hsa-miR-125a-5p | miRNA |
| 146. | MIR1260A | hsa-miR-1260 | miRNA |
| 147. | MIR1260b | hsa-miR-1260b | miRNA |
| 148. | MIR1280 | hsa-miR-1280 | miRNA |
| 149. | MIR1281 | hsa-miR-1281 | miRNA |
| 150. | MIR1290 | hsa-miR-1290 | miRNA |
| 151. | MIR1291 | hsa-miR-1291 | miRNA |
| 152. | MIR129-1 / MIR129-2 | hsa-miR-129-3p | miRNA |
| 153. | MIR1301 | hsa-miR-1301 | miRNA |
| 154. | MIR135A1 / MIR135A2 | hsa-miR-135a | miRNA |
| 155. | MIR135A1 / MIR135A2 | hsa-miR-135a* | miRNA |
| 156. | MIR141 | hsa-miR-141 | miRNA |
| 157. | MIR142 | hsa-miR-142-3p | miRNA |
| 158. | MIR149 | hsa-miR-149 | miRNA |
| 159. | MIR150 | hsa-miR-150 | miRNA |
| 160. | MIR150 | hsa-miR-150* | miRNA |
| 161. | MIR1539 | hsa-miR-1539 | miRNA |
| 162. | MIR181A2 | hsa-miR-181a | miRNA |
| 163. | MIR1825 | hsa-miR-1825 | miRNA |
| 164. | MIR186 | hsa-miR-186 | miRNA |
| 165. | MIR18a | hsa-miR-18a | miRNA |
| 166. | MIR18b | hsa-miR-18b | miRNA |
| 167. | MIR191 | hsa-miR-191* | miRNA |
| 168. | MIR197 | hsa-miR-197 | miRNA |
| 169. | MIR198 | hsa-miR-198 | miRNA |
| 170. | MIR205 | hsa-miR-205 | miRNA |
| 171. | MIR2116 | hsa-miR-2116* | miRNA |
| 172. | MIR215 | hsa-miR-215 | miRNA |
| 173. | MIR22 | hsa-miR-22 | miRNA |
| 174. | MIR223 | hsa-miR-223 | miRNA |
| 175. | MIR2276 | hsa-miR-2276 | miRNA |
| 176. | MIR23c | hsa-miR-23c | miRNA |
| 177. | MIR26A1 / MIR26A2 | hsa-miR-26a | miRNA |
| 178. | MIR28 | hsa-miR-28-3p | miRNA |
| 179. | MIR28 | hsa-miR-28-5p | miRNA |
| 180. | MIR29B1 / MIR29B2 | hsa-miR-29b | miRNA |
| 181. | MIR30a | hsa-miR-30a | miRNA |
| 182. | MIR30b | hsa-miR-30b | miRNA |
| 183. | MIR3131 | hsa-miR-3131 | miRNA |
| 184. | MIR3138 | hsa-miR-3138 | miRNA |
| 185. | MIR3149 | hsa-miR-3149 | miRNA |
| 186. | MIR3156-1 / MIR3156-2 / MIR3156-3 | hsa-miR-3156-5p | miRNA |
| 187. | MIR3180-1 / MIR3180-2 / MIR3180-3 / MIR3180-4 / MIR3180-5 | hsa-miR-3180-5p | miRNA |
| 188. | MIR3194 | hsa-miR-3194-5p | miRNA |
| 189. | MIR3195 | hsa-miR-3195 | miRNA |
| 190. | MIR3196 | hsa-miR-3196 | miRNA |
| 191. | MIR32 | hsa-miR-32 | miRNA |
| 192. | MIR320C1 / MIR320C2 | hsa-miR-320c | miRNA |
| 193. | MIR324 | hsa-miR-324-3p | miRNA |
| 194. | MIR328 | hsa-miR-328 | miRNA |
| 195. | MIR331 | hsa-miR-331-5p | miRNA |
| 196. | MIR33b | hsa-miR-33b* | miRNA |
| 197. | MIR342 | hsa-miR-342-3p | miRNA |
| 198. | MIR34a | hsa-miR-34a | miRNA |
| 199. | MIR3610 | hsa-miR-3610 | miRNA |
| 200. | MIR3648 | hsa-miR-3648 | miRNA |
| 201. | MIR3652 | hsa-miR-3652 | miRNA |
| 202. | MIR3663 | hsa-miR-3663-5p | miRNA |
| 203. | MIR3667 | hsa-miR-3667-5p | miRNA |
| 204. | MIR382 | hsa-miR-382 | miRNA |
| 205. | MIR3911 | hsa-miR-3911 | miRNA |
| 206. | MIR3937 | hsa-miR-3937 | miRNA |
| 207. | MIR4257 | hsa-miR-4257 | miRNA |
| 208. | MIR425 | hsa-miR-425* | miRNA |
| 209. | MIR4270 | hsa-miR-4270 | miRNA |
| 210. | MIR4274 | hsa-miR-4274 | miRNA |
| 211. | MIR4281 | hsa-miR-4281 | miRNA |
| 212. | MIR4284 | hsa-miR-4284 | miRNA |
| 213. | MIR4286 | hsa-miR-4286 | miRNA |
| 214. | MIR429 | hsa-miR-429 | miRNA |
| 215. | MIR4290 | hsa-miR-4290 | miRNA |
| 216. | MIR4313 | hsa-miR-4313 | miRNA |
| 217. | MIR4323 | hsa-miR-4323 | miRNA |
| 218. | MIR466 | hsa-miR-466 | miRNA |
| 219. | MIR483 | hsa-miR-483-3p | miRNA |
| 220. | MIR484 | hsa-miR-484 | miRNA |
| 221. | MIR485 | hsa-miR-485-3p | miRNA |
| 222. | MIR486 | hsa-miR-486-5p | miRNA |
| 223. | MIR488 | hsa-miR-488 | miRNA |
| 224. | MIR497 | hsa-miR-497 | miRNA |
| 225. | MIR511-1 | hsa-miR-511 | miRNA |
| 226. | MIR511-2 | hsa-miR-512 | miRNA |
| 227. | MIR520c | hsa-miR-520c-3p | miRNA |
| 228. | MIR550A1 / MIR550A2 / MIR550A3 | hsa-miR-550a | miRNA |
| 229. | MIR556 | hsa-miR-556-3p | miRNA |
| 230. | MIR557 | hsa-miR-557 | miRNA |
| 231. | MIR561 | hsa-miR-561 | miRNA |
| 232. | MIR572 | hsa-miR-572 | miRNA |
| 233. | MIR574 | hsa-miR-574-3p | miRNA |
| 234. | MIR574 | hsa-miR-574-5p | miRNA |
| 235. | MIR595 | hsa-miR-595 | miRNA |
| 236. | MIR630 | hsa-miR-630 | miRNA |
| 237. | MIR642b | hsa-miR-642b | miRNA |
| 238. | MIR646 | hsa-miR-646 | miRNA |
| 239. | MIR659 | hsa-miR-659 | miRNA |
| 240. | MIR660 | hsa-miR-660 | miRNA |
| 241. | MIR663A | hsa-miR-663 | miRNA |
| 242. | MIR664 | hsa-miR-664 | miRNA |
| 243. | MIR720 | hsa-miR-720 | miRNA |
| 244. | MIR762 | hsa-miR-762 | miRNA |
| 245. | MIR766 | hsa-miR-766 | miRNA |
| 246. | MIR877 | hsa-miR-877* | miRNA |
| 247. | MIR888 | hsa-miR-888 | miRNA |
| 248. | MIR933 | hsa-miR-933 | miRNA |
| 249. | MIR940 | hsa-miR-940 | miRNA |
| 250. | MIR95 | hsa-miR-95 | miRNA |
| 251. | hsv1-miR-H1* | hsv1-miR-H1*/ hsv1-miR-H1-3p | miRNA |
| 252. | hsv1-miR-H2-3p | hsv1-miR-H2-3p | miRNA |
| 253. | hsv1-miR-H6-3p | hsv1-miR-H6-3p | miRNA |
| 254. | hsv1-miR-H7* | hsv1-miR-H7* | miRNA |
| 255. | hsv1-miR-H8 | hsv1-miR-H8 | miRNA |
| 256. | hsv2-miR-H22 | hsv2-miR-H22 | miRNA |
| 257. | hsv2-miR-H25 | hsv2-miR-H25 | miRNA |
| 258. | hsv2-miR-H6 | hsv2-miR-H6 | miRNA |
| 259. | hsv2-miR-H6* | hsv2-miR-H6* | miRNA |
| 260. | kshv-miR-K12-10a | kshv-miR-K12-10a | miRNA |
| 261. | kshv-miR-K12-12* | kshv-miR-K12-12* | miRNA |
| 262. | kshv-miR-K12-8* | kshv-miR-K12-8* | miRNA |
| 263. | MIR564 | hsa-miR-564 | miRNA |
| 264. | MIRLET7D | hsa-let-7d-5p | miRNA |
| 265. | MIRLET7D | hsa-let-7d-3p | miRNA |
| 266. | MIR29A | hsa-miR-29a | miRNA |
| 267. | MIR219 | hsa-miR-219-1-3p | miRNA |
| 268. | MIR296 | hsa-miR-296-3p | miRNA |
| 269. | MIR337 | hsa-miR-337-5p | miRNA |
| 270. | MIR369 | hsa-miR-369-3p | miRNA |
| 271. | MIR450 | hsa-miR-450b-5p | miRNA |
| 272. | MIR483 | hsa-miR-483-5p | miRNA |
| 273. | MIR507 | hsa-miR-507 | miRNA |
| 274. | MIR517C | hsa-miR-517c | miRNA |
| 275. | MIR519A | hsa-miR-519a | miRNA |
| 276. | MIR519D | hsa-miR-519d | miRNA |
| 277. | MIR520G | hsa-miR-520g | miRNA |
| 278. | MIR576 | hsa-miR-576-5p | miRNA |
| 279. | MIR577 | hsa-miR-577 | miRNA |
| 280. | MIR604 | hsa-miR-604 | miRNA |
| 281. | MIR610 | hsa-miR-610 | miRNA |
| 282. | MIR619 | hsa-miR-619 | miRNA |
| 283. | MIR1256 | hsa-miR-1256 | miRNA |
| 284. | MIR1278 | hsa-miR-1278 | miRNA |
| 285. | MIR1297 | hsa-miR-1297 | miRNA |
| 286. | MIRUS33 | hcmv-miR-US33-5p | miRNA |
| 287. | MIRH1 | hsv1-miR-H1-5p | miRNA |
| 288. | MIR877 | hsa-miR-877 | miRNA |
| 289. | LET7E | hsa-let-7e-5p | miRNA |
| 290. | LET7E | hsa-let-7e-3p | miRNA |
| 291. | MIR9 | hsa-miR-9 | miRNA |
| 292. | MIR92B | hsa-miR-92b | miRNA |
| 293. | MIR96 | hsa-miR-96-5p | miRNA |
| 294. | MIR96 | hsa-miR-96-3p | miRNA |
| 295. | MIR106A | hsa-miR-106a-5p | miRNA |
| 296. | MIR106A | hsa-miR-106a-3p | miRNA |
| 297. | MIR17 | hsa-miR-17-5p | miRNA |
| 298. | MIR17 | hsa-miR-17-3p | miRNA |
| 299. | MIR106B | hsa-miR-106b-5p | miRNA |
| 300. | MIR106B | hsa-miR-106b-3p | miRNA |
| 301. | MIR127 | hsa-miR-127-3p | miRNA |
| 302. | MIR128 | hsa-miR-128 | miRNA |
| 303. | MIR154 | hsa-miR-154 | miRNA |
| 304. | MIR155 | hsa-miR-155 | miRNA |
| 305. | MIR183 | hsa-miR-183-5p | miRNA |
| 306. | MIR183 | hsa-miR-183-3p | miRNA |
| 307. | MIR194 | hsa-miR-194 | miRNA |
| 308. | MIR196B | hsa-miR-196b-5p | miRNA |
| 309. | MIR196B | hsa-miR-196b-3p | miRNA |
| 310. | MIR203 | hsa-miR-203 | miRNA |
| 311. | MIR204 | hsa-miR-204 | miRNA |
| 312. | MIR221 | hsa-miR-221-5p | miRNA |
| 313. | MIR221 | hsa-miR-221-3p | miRNA |
| 314. | MIR376 | hsa-miR-376a | miRNA |
| 315. | MIR376C | hsa-miR-376c | miRNA |
| 316. | MIR377 | hsa-miR-377 | miRNA |
| 317. | MIR379 | hsa-miR-379 | miRNA |
| 318. | MIR423 | hsa-miR-423-3p | miRNA |
| 319. | MIR424 | hsa-miR-424-5p | miRNA |
| 320. | MIR424 | hsa-miR-424-3p | miRNA |
| 321. | MIR425 | hsa-miR-425-5p | miRNA |
| 322. | MIR425 | hsa-miR-425-3p | miRNA |
| 323. | MIR454 | hsa-miR-454-5p | miRNA |
| 324. | MIR454 | hsa-miR-454-3p | miRNA |
| 325. | MIR486 | hsa-miR-486-3p | miRNA |
| 326. | MIR509 | hsa-miR-509-3p | miRNA |
| 327. | MIR514 | hsa-miR-514 | miRNA |
| 328. | MIR542 | hsa-miR-542-3p | miRNA |
| 329. | MIR545 | hsa-miR-545-5p | miRNA |
| 330. | MIR545 | hsa-miR-545-3p | miRNA |
| 331. | MIR626 | hsa-miR-626 | miRNA |
| 332. | MIR758 | hsa-miR-758-5p | miRNA |
| 333. | MIR758 | hsa-miR-758-3p | miRNA |
| 334. | MIR876 | hsa-miR-876-3p | miRNA |
| 335. | MIR1185 | hsa-miR-1185 | miRNA |
| 336. | MIR1266 | hsa-miR-1266-5p | miRNA |
| 337. | MIR1266 | hsa-miR-1266-3p | miRNA |
| 338. | MIR199A | hsa-miR-199a-3p | miRNA |
| 339. | MIR199B | hsa-miR-199b-3p | miRNA |
| 340. | MIR625 | hsa-miR-625-5p | miRNA |
| 341. | MIR625 | hsa-miR-625-3p | miRNA |
| 342. | MIR631 | hsa-miR-631 | miRNA |
| 343. | MIR635 | hsa-miR-635 | miRNA |

### Columns

(i) This number is the SEQ ID NO as shown in the sequence listing. For an auto-antigen biomarker, the SEQ ID NO in the sequence listing provides the coding sequence for the auto-antigen biomarker. For a miRNA biomarker, the SEQ ID NO in the sequence listing provides the sequence of the mature, expressed miRNA biomarker, as shown in Tables 3 and 4.
(ii) The "Symbol" column gives the gene symbol which has been approved by the HGNC. The HGNC aims to give unique and meaningful names to every miRNA and human gene. An additional dash-number suffix indicates pre-miRNAs that lead to identical mature miRNAs but that are located at different places in the genome.
(iii) The names for auto-antigens are taken from the Official Full Name provided by NCBI. An auto-antigen may have been referred to by one or more pseudonyms in the prior art. The invention relates to these auto-antigens regardless of their nomenclature. The names of the miRNA are taken from the specialist database, miRBase, according to version 16 (released, August 2010).

**Table 2**

| **No. ⁽ⁱ⁾** | **Symbol** ⁽ⁱⁱ⁾ | **ID ⁽ⁱⁱⁱ⁾** | **Name ^{(vi)}** | **HGNC ^{(v)}** | **GI ^{(vi)}** |
|---|---|---|---|---|---|
| 1. | ACTB | 60 | Homo sapiens actin beta | 132 | 12654910 |
| 2. | ADD1 | 118 | Homo sapiens adducin 1 (alpha) | 243 | 33869910 |
| 3. | ADSL | 158 | Homo sapiens adenylosuccinate lyase | 291 | 12652984 |
| 4. | AK2 | 204 | Homo sapiens adenylate kinase 2 transcript variant AK2A | 362 | 39645192 |
| 5. | KLK3 | 354 | Homo sapiens kallikrein 3 (prostate specific antigen) transcript variant 1 | 6364 | 34193547 |
| 6. | ATF1 | 466 | Homo sapiens activating transcription factor 1 | 783 | 20810444 |
| 7. | CKM | 1158 | Homo sapiens creatine kinase muscle | 1994 | 13938618 |
| 8. | CLK2 | 1196 | Homo sapiens CDC-like kinase 2 transcript variant phclk2 | 2069 | 33873844 |
| 9. | DDB2 | 1643 | Homo sapiens damage-specific DNA binding protein 2 48kDa | 2718 | 34783036 |
| 10. | DTYMK | 1841 | Homo sapiens deoxythymidylate kinase (thymidylate kinase) | 3061 | 38114725 |
| 11. | DUSP4 | 1846 | Homo sapiens dual specificity phosphatase 4 transcript variant 1 | 3070 | 33869553 |
| 12. | E2F6 | 1876 | Homo sapiens E2F transcription factor 6 | 3120 | 14249933 |
| 13. | EXT2 | 2132 | Homo sapiens exostoses (multiple) 2 | 3513 | 14603195 |
| 14. | FGFR4_ ext | 2264 | Homo sapiens fibroblast growth factor receptor 4 transcript variant 3 | 3691 | 33873872 |
| 15. | FIGF | 2277 | Homo sapiens c-fos induced growth factor (vascular endothelial growth factor D) | 3708 | 20379761 |
| 16. | FKBP3 | 2287 | Homo sapiens FK506 binding protein 3 25kDa | 3719 | 16740853 |
| 17. | GALK1 | 2584 | Homo sapiens galactokinase 1 | 4118 | 12654656 |
| 18. | GK2 | 2712 | Homo sapiens glycerol kinase 2 | 4291 | 20987489 |
| 19. | GRB7 | 2886 | Homo sapiens growth factor receptor-bound protein 7 | 4567 | 33870450 |
| 20. | GTF2B | 2959 | Homo sapiens general transcription factor IIB | 4648 | 18088836 |
| 21. | GTF2H1 | 2965 | Homo sapiens general transcription factor IIH polypeptide 1 62kDa | 4655 | 33991027 |
| 22. | GTF2H2 | 2966 | Homo sapiens general transcription factor IIH polypeptide 2 44kDa | 4656 | 40674449 |
| 23. | HSPD1 | 3329 | Homo sapiens heat shock 60kDa protein 1 (chaperonin) | 5261 | 38197215 |
| 24. | IDI1 | 3422 | Homo sapiens isopentenyl-diphosphate delta isomerase | 5387 | 34782883 |
| 25. | IFI16 | 3428 | Homo sapiens interferon, gamma-inducible protein 16, | 5395 | 16877621 |
| 26. | LDHA | 3939 | Homo sapiens lactate dehydrogenase A | 6535 | 45501321 |
| 27. | LYL1 | 4066 | Homo sapiens lymphoblastic leukemia derived sequence 1 | 6734 | 33988028 |
| 28. | MARK3 | 4140 | Homo sapiens MAP/microtubule affinity-regulating kinase 3 | 6897 | 19353235 |
| 29. | MPP3 | 4356 | Homo sapiens membrane protein palmitoylated 3 (MAGUK p55 subfamily member 3) | 7221 | 34785138 |
| 30. | TRIM37 | 4591 | Homo sapiens tripartite motif-containing 37, | 7523 | 23271191 |
| 31. | NCF2 | 4688 | Homo sapiens neutrophil cytosolic factor 2 (65kDa, chronic granulomatous disease, autosomal 2), | 7661 | 12804408 |
| 32. | RPL10A | 4736 | Homo sapiens ribosomal protein L10a | 10299 | 13905015 |
| 33. | NFYA | 4800 | Homo sapiens nuclear transcription factor Y alpha | 7804 | 24660183 |
| 34. | NRAS | 4893 | Homo sapiens neuroblastoma RAS viral (v-ras) oncogene homolog | 7989 | 13528839 |
| 35. | NTRK3_ ext | 4916 | Homo sapiens neurotrophic tyrosine kinase receptor type 3 transcript variant 3 | 8033 | 15489167 |
| 36. | OAS2 | 4939 | Homo sapiens 2'-5'-oligoadenylate synthetase 2 69/71 kDa | 8087 | 29351562 |
| 37. | endometr iosis | 5091 | Homo sapiens pyruvate carboxylase | 8636 | 33871110 |
| 38. | CDK17 | 5128 | Homo sapiens endometriosisTAIRE protein kinase 2 | 8750 | 21542570 |
| 39. | PDE4A | 5141 | Homo sapiens Homo sapiens phosphodiesterase 4A, cAMP-specific (phosphodiesterase E2 dunce homolog, Dro | 8780 | 18043808 |
| 40. | PHF1 | 5252 | Homo sapiens PHD finger protein 1 transcript variant 2 | 8919 | 33874006 |
| 41. | PKM2 | 5315 | Homo sapiens pyruvate kinase muscle transcript variant 1 | 9021 | 14043290 |
| 42. | MAP2K5 | 5607 | Homo sapiens mitogen-activated protein kinase kinase 5, transcript variant A | 6845 | 33871775 |
| 43. | PRPS2 | 5634 | Homo sapiens phosphoribosyl pyrophosphate synthetase 2 | 9465 | 26251732 |
| 44. | RAN | 5901 | Homo sapiens RAN member RAS oncogene family | 9846 | 33871120 |
| 45. | RB1 | 5925 | Homo sapiens retinoblastoma 1 (including osteosarcoma) | 9884 | 24660139 |
| 46. | RBMS1 | 5937 | Homo sapiens Homo sapiens RNA binding motif single stranded interacting protein 1 transcript variant | 9907 | 33869903 |
| 47. | RET_a | 5979 | Homo sapiens ret proto-oncogene (multiple endocrine neoplasia and medullary thyroid carci | 9967 | 13279040 |
| 48. | RORC | 6097 | Homo sapiens RAR-related orphan receptor C | 10260 | 21594879 |
| 49. | RPL18 | 6141 | Homo sapiens ribosomal protein L18 | 10310 | 38197133 |
| 50. | RPL18A | 6142 | Homo sapiens ribosomal protein L18a | 10311 | 38196939 |
| 51. | RPL28 | 6158 | Homo sapiens ribosomal protein L28 | 10330 | 15079502 |
| 52. | RPL31 | 6160 | Homo sapiens ribosomal protein L31 | 10334 | 40226052 |
| 53. | RPL32 | 6161 | Homo sapiens ribosomal protein L32 | 10336 | 15079341 |
| 54. | S100A6 | 6277 | Homo sapiens S100 calcium binding protein A6 (calcyclin) | 10496 | 33876209 |
| 55. | SCP2 | 6342 | Homo sapiens sterol carrier protein 2 transcript variant 2 | 10606 | 45501107 |
| 56. | SIAH1 | 6477 | Homo sapiens seven in absentia homolog 1 (Drosophila) transcript variant 2 | 10857 | 27503513 |
| 57. | SMARC D1 | 6602 | Homo sapiens SWI/SNF related matrix associated actin dependent regulator of chromatin subfamily d member 1 | 11106 | 33874464 |
| 58. | SMARCE 1 | 6605 | Homo sapiens SWI/SNF related matrix associated actin dependent regulator of chromatin | 11109 | 13937940 |
| 59. | SOD2 | 6648 | Homo sapiens superoxide dismutase 2 mitochondrial | 11180 | 15214594 |
| 60. | SOX2 | 6657 | Homo sapiens SRY (sex determining region Y)-box 2 | 11195 | 33869633 |
| 61. | SRPK1 | 6732 | Homo sapiens SFRS protein kinase 1 | 11305 | 23468344 |
| 62. | TPM1 | 7168 | Homo sapiens tropomyosin 1 (alpha) | 12010 | 33873609 |
| 63. | NR2C1 | 7181 | Homo sapiens nuclear receptor subfamily 2 group C member 1 | 7971 | 25304018 |
| 64. | TRIP6 | 7205 | Homo sapiens thyroid hormone receptor interactor 6, | 12311 | 13436460 |
| 65. | UBA1 | 7317 | Homo sapiens Homo sapiens ubiquitin-activating enzyme E1 (A1S9T and BN75 temperature sensitivity compl | 12469 | 33989140 |
| 66. | VCL | 7414 | Homo sapiens vinculin | 12665 | 24657578 |
| 67. | ZNF41 | 7592 | Homo sapiens zinc finger protein 41 transcript variant 2 | 13107 | 21955337 |
| 68. | PAX8 | 7849 | Homo sapiens paired box gene 8 transcript variant PAX8A | 8622 | 33987990 |
| 69. | NRIP1 | 8204 | Homo sapiens nuclear receptor interacting protein 1 | 8001 | 25955638 |
| 70. | PIP4K2B | 8396 | Homo sapiens phosphatidylinositol-4-phosphate 5-kinase type II beta transcript variant 2 | 8998 | 20071965 |
| 71. | UXT | 8409 | Homo sapiens ubiquitously-expressed transcript | 12641 | 14424496 |
| 72. | API5 | 8539 | Homo sapiens apoptosis inhibitor 5 | 594 | 17389324 |
| 73. | MKNK1 | 8569 | Homo sapiens MAP kinase-interacting serine/threonine kinase 1 | 7110 | 33877125 |
| 74. | SUCLA2 | 8803 | Homo sapiens succinate-CoA ligase ADP-forming beta subunit | 11448 | 34783884 |
| 75. | LDB1 | 8861 | Homo sapiens LIM domain binding 1 | 6532 | 38197167 |
| 76. | NAE1 | 8883 | Homo sapiens amyloid beta precursor protein binding protein 1 transcript variant 1 | 621 | 38197227 |
| 77. | PAPSS2 | 9060 | Homo sapiens 3'-phosphoadenosine 5'-phosphosulfate synthase 2 | 8604 | 33869502 |
| 78. | USP10 | 9100 | Homo sapiens ubiquitin specific protease 10 | 12608 | 12653004 |
| 79. | PRPF4 | 9128 | Homo sapiens Homo sapiens PRP4 pre- | 17349 | 33876345 |
| 80. | AIM2 | 9447 | Homo sapiens absent in melanoma 2 | 357 | 15012076 |
| 81. | TBPL1 | 9519 | Homo sapiens TBP-like 1 | 11589 | 33988482 |
| 82. | TRAF4 | 9618 | Homo sapiens TNF receptor-associated factor 4 transcript variant 1 | 12034 | 12804686 |
| 83. | SOCS5 | 9655 | Homo sapiens suppressor of cytokine signaling 5 | 16852 | 23273933 |
| 84. | ZSCAN1 2 | 9753 | Homo sapiens zinc finger protein 305 | 13172 | 27371192 |
| 85. | HDAC4 | 9759 | Homo sapiens cDNA | 14063 | 25058272 |
| 86. | KIAA010 1 | 9768 | Homo sapiens KIAA0101 gene product | 28961 | 33873244 |
| 87. | IP6K1 | 9807 | Homo sapiens inositol hexaphosphate kinase 1 | 18360 | 15277916 |
| 88. | RNF40 | 9810 | Homo sapiens ring finger protein 40 transcript variant 1 | 16867 | 13543993 |
| 89. | GPHN | 10243 | Homo sapiens gephyrin | 15465 | 34783414 |
| 90. | HRSP12 | 10247 | Homo sapiens translational inhibitor protein p14.5 | 16897 | 16307462 |
| 91. | STUB1 | 10273 | Homo sapiens STIP1 homology and U-Box containing protein 1 | 11427 | 14043118 |
| 92. | TRAIP | 10293 | Homo sapiens TRAF interacting protein | 30764 | 17939476 |
| 93. | PLK4 | 10733 | Homo sapiens serine/threonine kinase 18 | 11397 | 23243308 |
| 94. | CCNI | 10983 | Homo sapiens cyclin I | 1595 | 38197480 |
| 95. | IL24 | 11009 | Homo sapiens interleukin 24 transcript variant 1 | 11346 | 16307184 |
| 96. | CDK20 | 23552 | Homo sapiens cell cycle related kinase | 21420 | 33988018 |
| 97. | PABendo metriosis 1 | 26986 | Homo sapiens poly(A) binding protein cytoplasmic 1 | 8554 | 33872187 |
| 98. | MED4 | 29079 | Homo sapiens vitamin D receptor interacting protein | 17903 | 13528773 |
| 99. | NME7 | 29922 | Homo sapiens non-metastatic cells 7 protein expressed in (nucleoside-diphosphate kinase) transcript variant 1 | 20461 | 13937770 |
| 100. | PHF11 | 51131 | Homo sapiens PHD finger protein 11 | 17024 | 33880652 |
| 101. | IRAK4 | 51135 | Homo sapiens interleukin-1 receptor-associated kinase 4 mRNA (cDNA clone MGC:13330 ) | 17967 | 15426431 |
| 102. | TXNDC3 | 51314 | Homo sapiens thioredoxin domain containing 3 (spermatozoa) | 16473 | 22477641 |
| 103. | TAOK3 | 51347 | Homo sapiens STE20-like kinase | 18133 | 33877128 |
| 104. | STYXL 1 | 51657 | Homo sapiens dual specificity phosphatase 24 (p utative) | 18165 | 33869206 |
| 105. | ASB1 | 51665 | Homo sapiens ankyrin repeat and SOCS box-containing 1 | 16011 | 33878672 |
| 106. | MST4 | 51765 | Homo sapiens Mst3 and SOK1-related kinase (MASK) | na | 109633024 |
| 107. | PELO | 53918 | Homo sapiens pelota homolog (Drosophila) | 8829 | 33870521 |
| 108. | ETNK2 | 55224 | Homo sapiens ethanolamine kinase 2 | 25575 | 33873304 |
| 109. | RFK | 55312 | Homo sapiens riboflavin kinase | 30324 | 13937919 |
| 110. | C9orf86 | 55684 | Homo sapiens chromosome 9 open reading frame 86 | 24703 | 18089263 |
| 111. | DDX55 | 57696 | Homo sapiens DEAD (Asp-Glu-Ala-Asp) box polypeptide 55 | 20085 | 34190861 |
| 112. | CCNB1I P1 | 57820 | Homo sapiens cDNA | 19437 | 12654750 |
| 113. | KLHL12 | 59349 | Homo sapiens kelch-like protein C3IP1 | 19360 | 13112018 |
| 114. | SAV1 | 60485 | Homo sapiens salvador homolog 1 (Drosophila) | 17795 | 18088227 |
| 115. | CAMKV | 79012 | Homo sapiens hypothetical protein MGC8407 | 28788 | 33875513 |
| 116. | NSBP1 | 79366 | Homo sapiens nucleosomal binding protein 1, | 8013 | 13529139 |
| 117. | ALPK1 | 80216 | Homo sapiens alpha-kinase 1 mRNA (cDNA clone MGC:71554 ) | 20917 | 38174241 |
| 118. | ELMOD3 | 84173 | Homo sapiens RNA binding motif and ELMC domain 1 | 26158 | 33877554 |
| 119. | AIFM2 | 84883 | Homo sapiens apoptosis-inducing factor (AIF)-like mitochondrion-associated inducer of death | 21411 | 13543963 |
| 120. | RHOT2 | 89941 | Homo sapiens ras homolog gene family member T2 | 21169 | 15928946 |
| 121. | PYGO2 | 90780 | Homo sapiens pygopus 2 | 30257 | 33991480 |

### Columns

(i) This number is the SEQ ID NO: for the coding sequence for the auto-antigen biomarker, as shown in the sequence listing.
(ii) The "ID" column shows the Entrez GenelD number for the antigen marker. An Entrez GenelD value is unique across all taxa.
(iii) The "Symbol" column gives the gene symbol which has been approved by the HGNC. The HGNC aims to give unique and meaningful names to every miRNA and human gene.
(iv) This name is taken from the Official Full Name provided by NCBI. An auto-antigen may have been referred to by one or more pseudonyms in the prior art. The invention relates to these auto-antigens regardless of their nomenclature.
(v) The HUGO Gene Nomenclature Committee aims to give unique and meaningful names to every human gene. The HGNC number thus identifies a unique human gene. An additional dash-number suffix indicates pre-miRNAs that lead to identical mature miRNAs but that are located at different places in the genome.
(vi) A "GI" number, or "Genlnfo Identifier", is a series of digits assigned consecutively to each sequence record processed by NCBI when sequences are added to its databases. The GI number bears no resemblance to the accession number of the sequence record. When a sequence is updated (e.g. for correction, or to add more annotation or information) it receives a new GI number. Thus the sequence associated with a given GI number is never changed.

**Table 3**

| **No. ⁽ⁱ⁾** | **miRNA name ⁽ⁱⁱ⁾** | **Symbol⁽ⁱⁱⁱ⁾** | **HGNC ^{(iv)}** | **Sequence** |
|---|---|---|---|---|
| 122. | ebv-miR-BART12 | ebv-miR-BART12 | | UCCUGUGGUGUUUGGUGUGGUU |
| 123. | ebv-miR-BART14 | ebv-miR-BART14 | | UAAAUGCUGCAGUAGUAGGGAU |

| **No. ⁽ⁱ⁾** | **miRNA name⁽ⁱⁱ⁾** | **Symbol⁽ⁱⁱⁱ⁾** | **HGNC ^{(iv)}** | **Sequence** |
|---|---|---|---|---|
| 124. | ebv-miR-BART16 | ebv-miR-BART16 | | UUAGAUAGAGUGGGUGUGUGCUCU |
| 125. | ebv-miR-BART20-5p | ebv-miR-BART20-5p | | UAGCAGGCAUGUCUUCAUUCC |
| 126. | ebv-miR-BART2-5p | ebv-miR-BART2-5p | | UAUUUUCUGCAUUCGCCCUUGC |
| 127. | hsa-let-7b* | MIRLET7B | HGNC:31479 | UGAGGUAGUAGGUUGUGUGGUU |
| 128. | hsa-let-7f | MIRLET7F1 | HGNC:31483 | UGAGGUAGUAGAUUGUAUAGUU |
| 129. | hsa-let-7f-1* | MIRLET7F2 | HGNC:31484 | UGAGGUAGUAGAUUGUAUAGUU |
| 130. | hsa-let-7g | MIRLET7G | HGNC:31485 | UGAGGUAGUAGUUUGUACAGUU |
| 131. | hsa-miR-103a | MIR103A1 | HGNC:31490 | AGCAGCAUUGUACAGGGCUAUGA |
| 132. | hsa-miR-10b | MIR10b | HGNC:31498 | UACCCUGUAGAACCGAAUUUGUG |
| 133. | hsa-miR-1183 | MIR1183 | HGNC:35264 | CACUGUAGGUGAUGGUGAGAGUGGGCA |
| 134. | hsa-miR-1202 | MIR1202 | HGNC:35268 | GUGCCAGCUGCAGUGGGGGAG |
| 135. | hsa-miR-1207-5p | MIR1207 | HGNC:35273 | UGGCAGGGAGGCUGGGAGGGG |
| 136. | hsa-miR-122 | MIR122 | HGNC:31501 | UGGAGUGUGACAAUGGUGUUUG |
| 137. | hsa-m iR-1224-5p | MIR1224 | HGNC:33923 | GUGAGGACUCGGGAGGUGG |
| 138. | hsa-m iR-1225-3p | MIR1225 | HGNC:33931 | GUGGGUACGGCCCAGUGGGGGG |
| 139. | hsa-m iR-1225-5p | MIR1225 | HGNC:33931 | GUGGGUACGGCCCAGUGGGGGG |
| 140. | hsa-miR-1226* | MIR1226 | HGNC:33922 | UCACCAGCCCUGUGUUCCCUAG |
| 141. | hsa-miR-1228* | MIR1228 | HGNC:33928 | UCACACCUGCCUCGCCCCCC |
| 142. | hsa-miR-1234 | MIR1234 | HGNC:33926 | UCGGCCUGACCACCCACCCCAC |
| 143. | hsa-miR-1237 | MIR1237 | HGNC:33927 | UCCUUCUGCUCCGUCCCCCAG |
| 144. | hsa-miR-1238 | MIR1238 | HGNC:33933 | CUUCCUCGUCUGUCUGCCCC |
| 145. | hsa-m iR-125a-5p | MIR125a | HGNC:31505 | UCCCUGAGACCCUUUAACCUGUGA |
| 146. | hsa-miR-1260 | MIR1260A | HGNC:35325 | AUCCCACCUCUGCCACCA |
| 147. | hsa-miR-1260b | MIR1260b | HGNC:38258 | AUCCCACCACUGCCACCAU |
| 148. | hsa-miR-1280 | MIR1280 | HGNC:35368 | UCCCACCGCUGCCACCC |
| 149. | hsa-miR-1281 | MIR1281 | HGNC:35359 | UCGCCUCCUCCUCUCCC |
| 150. | hsa-miR-1290 | MIR1290 | HGNC:35283 | UGGAUUUUUGGAUCAGGGA |
| 151. | hsa-miR-1291 | MIR1291 | HGNC:35284 | UGGCCCUGACUGAAGACCAGCAGU |
| 152. | hsa-miR-129-3p | MIR129-1 / MIR129-2 | HGNC:31512 /31513 | CUUUUUGCGGUCUGGGCUUGC |
| 153. | hsa-miR-1301 | MIR1301 | HGNC:35253 | UUGCAGCUGCCUGGGAGUGACUUC |
| 154. | hsa-miR-135a | MIR135A1 / MIR135A2 | HGNC:31520 / 31521 | UAUGGCUUUUUAUUCCUAUGUGA |
| 155. | hsa-miR-135a* | MIR135A1/ MIR135A2 | HGNC:31520 / 31521 | UAUGGCUUUUUAUUCCUAUGUGA |
| 156. | hsa-miR-141 | MIR141 | HGNC:31528 | UAACACUGUCUGGUAAAGAUGG |
| 157. | hsa-miR-142-3p | MIR142 | HGNC:31529 | CAUAAAGUAGAAAGCACUACU |
| 158. | hsa-miR-149 | MIR149 | HGNC:31536 | UCUGGCUCCGUGUCUUCACUCCC |
| 159. | hsa-miR-150 | MIR150 | HGNC:31537 | UCUCCCAACCCUUGUACCAGUG |
| 160. | hsa-miR-150* | MIR150 | HGNC:31537 | UCUCCCAACCCUUGUACCAGUG |
| 161. | hsa-miR-1539 | MIR1539 | HGNC:35383 | UCCUGCGCGUCCCAGAUGCCC |
| 162. | hsa-miR-181a | MIR181A2 | HGNC:31549 | AACAUUCAACGCUGUCGGUGAGU |
| 163. | hsa-miR-1825 | MIR1825 | HGNC:35389 | UCCAGUGCCCUCCUCUCC |
| 164. | hsa-miR-186 | MIR186 | HGNC:31557 | CAAAGAAUUCUCCUUUUGGGCU |
| 165. | hsa-miR-18a | MIR18a | HGNC:31548 | UAAGGUGCAUCUAGUGCAGAUAG |
| 166. | hsa-miR-18b | MIR18b | HGNC:32025 | UAAGGUGCAUCUAGUGCAGUUAG |
| 167. | hsa-miR-191* | MIR191 | HGNC:31561 | CAACGGAAUCCCAAAAGCAGCUG |
| 168. | hsa-miR-197 | MIR197 | HGNC:31569 | UUCACCACCUUCUCCACCCAGC |
| 169. | hsa-miR-198 | MIR198 | HGNC:31570 | GGUCCAGAGGGGAGAUAGGUUC |
| 170. | hsa-miR-205 | MIR205 | HGNC:31583 | UCCUUCAUUCCACCGGAGUCUG |
| 171. | hsa-miR-2116* | MIR2116 | HGNC:37310 | GGUUCUUAGCAUAGGAGGUCU |
| 172. | hsa-miR-215 | MIR215 | HGNC:31592 | AUGACCUAUGAAUUGACAGAC |
| 173. | hsa-miR-22 | MIR22 | HGNC:31599 | AAGCUGCCAGUUGAAGAACUGU |
| 174. | hsa-miR-223 | MIR223 | HGNC:31603 | UGUCAGUUUGUCAAAUACCCCA |
| 175. | hsa-miR-2276 | MIR2276 | HGNC:37313 | UCUGCAAGUGUCAGAGGCGAGG |
| 176. | hsa-miR-23c | MIR23c | HGNC:38913 | AUCACAUUGCCAGUGAUUACCC |
| 177. | hsa-miR-26a | MIR26A1 / MIR26A2 | HGNC:31610 /31611 | UUCAAGUAAUCCAGGAUAGGCU |
| 178. | hsa-miR-28-3p | MIR28 | HGNC:31615 | AAGGAGCUCACAGUCUAUUGAG |
| 179. | hsa-miR-28-5p | MIR28 | HGNC:31615 | AAGGAGCUCACAGUCUAUUGAG |
| 180. | hsa-miR-29b | MIR29B1 / MIR29B2 | HGNC:31619 /31620 | UAGCACCAUUUGAAAUCAGUGUU |
| 181. | hsa-miR-30a | MIR30a | HGNC:31624 | UGUAAACAUCCUCGACUGGAAG |
| 182. | hsa-miR-30b | MIR30b | HGNC:31625 | UGUAAACAUCCUACACUCAGCU |
| 183. | hsa-miR-3131 | MIR3131 | HGNC:38347 | UCGAGGACUGGUGGAAGGGCCUU |
| 184. | hsa-miR-3138 | MIR3138 | HGNC:38341 | UGUGGACAGUGAGGUAGAGGGAGU |
| 185. | hsa-miR-3149 | MIR3149 | HGNC:38251 | UUUGUAUGGAUAUGUGUGUGUAU |
| 186. | hsa-m iR-3156-5p | MIR3156-1 / MIR3156-2/ MIR3156-3 | HGNC:38241 / 38213 / 38229 | AAAGAUCUGGAAGUGGGAGACA |
| 187. | hsa-m iR-3180-5p | MIR3180-1 / MIR3180-2 / MIR3180-3/ MIR3180-4 / MIR3180-5 | HGNC:38382/ 38343 / 38239 / 38920 / 38969 | UGGGGCGGAGCUUCCGGAG |
| 188. | hsa-m iR-3194-5p | MIR3194 | HGNC:38346 | GGCCAGCCACCAGGAGGGCUG |
| 189. | hsa-miR-3195 | MIR3195 | HGNC:38250 | CGCGCCGGGCCCGGGUU |
| 190. | hsa-miR-3196 | MIR3196 | HGNC:38198 | CGGGGCGGCAGGGGCCUC |
| 191. | hsa-miR-32 | MIR32 | HGNC:31631 | UAUUGCACAUUACUAAGUUGCA |
| 192. | hsa-miR-320c | MIR320C1 / MIR320C2 | HGNC:35248/ 35387 | AAAAGCUGGGUUGAGAGGGU |
| 193. | hsa-miR-324-3p | MIR324 | HGNC:31767 | CGCAUCCCCUAGGGCAUUGGUGU |
| 194. | hsa-miR-328 | MIR328 | HGNC:31770 | CUGGCCCUCUCUGCCCUUCCGU |
| 195. | hsa-miR-331-5p | MIR331 | HGNC:31772 | CUAGGUAUGGUCCCAGGGAUCC |
| 196. | hsa-miR-33b* | MIR33b | HGNC:32791 | GUGCAUUGCUGUUGCAUUGC |
| 197. | hsa-miR-342-3p | MIR342 | HGNC:31778 | UCUCACACAGAAAUCGCACCCGU |
| 198. | hsa-miR-34a | MIR34a | HGNC:31635 | UGGCAGUGUCUUAGCUGGUUGU |
| 199. | hsa-miR-3610 | MIR3610 | HGNC:38942 | GAAUCGGAAAGGAGGCGCCG |
| 200. | hsa-miR-3648 | MIR3648 | HGNC:38941 | AGCCGCGGGGAUCGCCGAGGG |
| 201. | hsa-miR-3652 | MIR3652 | HGNC:38894 | CGGCUGGAGGUGUGAGGA |
| 202. | hsa-miR-3663-5p | MIR3663 | HGNC:38958 | GCUGGUCUGCGUGGUGCUCGG |
| 203. | hsa-miR-3667-5p | MIR3667 | HGNC:38990 | AAAGACCCAUUGAGGAGAAGGU |
| 204. | hsa-miR-382 | MIR382 | HGNC:31875 | GAAGUUGUUCGUGGUGGAUUCG |
| 205. | hsa-miR-3911 | MIR3911 | HGNC:38962 | UGUGUGGAUCCUGGAGGAGGCA |
| 206. | hsa-miR-3937 | MIR3937 | HGNC:38970 | ACAGGCGGCUGUAGCAAUGGGGG |
| 207. | hsa-miR-4257 | MIR4257 | HGNC:38312 | CCAGAGGUGGGGACUGAG |
| 208. | hsa-miR-425* | MIR425 | HGNC:31882 | AAUGACACGAUCACUCCCGUUGA |
| 209. | hsa-miR-4270 | MIR4270 | HGNC:38377 | UCAGGGAGUCAGGGGAGGGC |
| 210. | hsa-miR-4274 | MIR4274 | HGNC:38194 | CAGCAGUCCCUCCCCCUG |
| 211. | hsa-miR-4281 | MIR4281 | HGNC:38357 | GGGUCCCGGGGAGGGGGG |
| 212. | hsa-miR-4284 | MIR4284 | HGNC:38322 | GGGCUCACAUCACCCCAU |
| 213. | hsa-miR-4286 | MIR4286 | HGNC:38186 | ACCCCACUCCUGGUACC |
| 214. | hsa-miR-429 | MIR429 | HGNC: 13784 | UAAUACUGUCUGGUAAAACCGU |
| 215. | hsa-miR-4290 | MIR4290 | HGNC:38360 | UGCCCUCCUUUCUUCCCUC |
| 216. | hsa-miR-4313 | MIR4313 | HGNC:38310 | AGCCCCCUGGCCCCAAACCC |
| 217. | hsa-miR-4323 | MIR4323 | HGNC:38394 | CAGCCCCACAGCCUCAGA |
| 218. | hsa-miR-466 | MIR466 | HGNC:38359 | AUACACAUACACGCAACACACAU |
| 219. | hsa-miR-483-3p | MIR483 | HGNC:32340 | AAGACGGGAGGAAAGAAGGGAG |
| 220. | hsa-miR-484 | MIR484 | HGNC:32341 | UCAGGCUCAGUCCCCUCCCGAU |
| 221. | hsa-miR-485-3p | MIR485 | HGNC:32067 | AGAGGCUGGCCGUGAUGAAUUC |
| 222. | hsa-miR-486-5p | MIR486 | HGNC:32342 | UCCUGUACUGAGCUGCCCCGAG |
| 223. | hsa-miR-488 | MIR488 | HGNC:32073 | UUGAAAGGCUAUUUCUUGGUC |
| 224. | hsa-miR-497 | MIR497 | HGNC:32088 | CAGCAGCACACUGUGGUUUGU |
| 225. | hsa-miR-511 | MIR511-1 | HGNC:32077 | GUGUCUUUUGCUCUGCAGUCA |
| 226. | hsa-miR-512 | MIR511-2 | HGNC:32078 | GUGUCUUUUGCUCUGCAGUCA |
| 227. | hsa-miR-520c-3p | MIR520c | HGNC:32108 | CUCUAGAGGGAAGCACUUUCUG |
| 228. | hsa-miR-550a | MIR550A1 / MIR550A2 / MIR550A3 | HGNC:32804 /32805 /41870 | AGUGCCUGAGGGAGUAAGAGCCC |
| 229. | hsa-miR-556-3p | MIR556 | HGNC:32812 | GAUGAGCUCAUUGUAAUAUGAG |
| 230. | hsa-miR-557 | MIR557 | HGNC:32813 | GUUUGCACGGGUGGGCCUUGUCU |
| 231. | hsa-miR-561 | MIR561 | HGNC:32817 | CAAAGUUUAAGAUCCUUGAAGU |
| 232. | hsa-miR-572 | MIR572 | HGNC:32828 | GUCCGCUCGGCGGUGGCCCA |
| 233. | hsa-miR-574-3p | MIR574 | HGNC:32830 | UGAGUGUGUGUGUGUGAGUGUGU |
| 234. | hsa-miR-574-5p | MIR574 | HGNC:32830 | UGAGUGUGUGUGUGUGAGUGUGU |
| 235. | hsa-miR-595 | MIR595 | HGNC:32851 | GAAGUGUGCCGUGGUGUGUCU |
| 236. | hsa-miR-630 | MIR630 | HGNC:32886 | AGUAUUCUGUACCAGGGAAGGU |
| 237. | hsa-miR-642b | MIR642b | HGNC:38902 | AGACACAUUUGGAGAGGGACCC |
| 238. | hsa-miR-646 | MIR646 | HGNC:32902 | AAGCAGCUGCCUCUGAGGC |
| 239. | hsa-miR-659 | MIR659 | HGNC:32915 | CUUGGUUCAGGGAGGGUCCCCA |
| 240. | hsa-miR-660 | MIR660 | HGNC:32916 | UACCCAUUGCAUAUCGGAGUUG |
| 241. | hsa-miR-663 | MIR663A | HGNC:32919 | AGGCGGGGCGCCGCGGGACCGC |
| 242. | hsa-miR-664 | MIR664 | HGNC:35370 | UAUUCAUUUAUCCCCAGCCUACA |
| 243. | hsa-miR-720 | MIR720 | HGNC:35375 | UCUCGCUGGGGCCUCCA |
| 244. | hsa-miR-762 | MIR762 | HGNC:37303 | GGGGCUGGGGCCGGGGCCGAGC |
| 245. | hsa-miR-766 | MIR766 | HGNC:33139 | ACUCCAGCCCCACAGCCUCAGC |
| 246. | hsa-miR-877* | MIR877 | HGNC:33660 | GUAGAGGAGAUGGCGCAGGG |
| 247. | hsa-miR-888 | MIR888 | HGNC:33648 | UACUCAAAAAGCUGUCAGUCA |
| 248. | hsa-miR-933 | MIR933 | HGNC:33676 | UGUGCGCAGGGAGACCUCUCCC |
| 249. | hsa-miR-940 | MIR940 | HGNC:33683 | AAGGCAGGGCCCCCGCUCCCC |
| 250. | hsa-miR-95 | MIR95 | HGNC:31647 | UUCAACGGGUAUUUAUUGAGCA |
| 251. | hsv1-miR-H1*/ hsv1-miR-H1-3p | hsv1-miR-H1* | | UACACCCCCCUGCCUUCCACCCU |
| 252. | hsv1-miR-H2-3p | hsv1-miR-H2-3p | | CCUGAGCCAGGGACGAGUGCGACU |
| 253. | hsv1-miR-H6-3p | hsv1-miR-H6-3p | | CACUUCCCGUCCUUCCAUCCC |
| 254. | hsv1-miR-H7* | hsv1-miR-H7* | | UUUGGAUCCCGACCCCUCUUC |
| 255. | hsv1-miR-H8 | hsv1-miR-H8 | | UAUAUAGGGUCAGGGGGUUC |
| 256. | hsv2-miR-H22 | hsv2-miR-H22 | | AGGGGUCUGGACGUGGGUGGGC |
| 257. | hsv2-miR-H25 | hsv2-miR-H25 | | CUGCGCGGCGGAGACCGGGAC |
| 258. | hsv2-miR-H6 | hsv2-miR-H6 | | AAUGGAAGGCGAGGGGAUGC |
| 259. | hsv2-miR-H6* | hsv2-miR-H6* | | CCCAUCUUCUGCCCUUCCAUCCU |
| 260. | kshv-miR-K12-10a | kshv-miR-K12-10a | | UAGUGUUGUCCCCCCGAGUGGC |
| 261. | kshv-miR-K12-12* | kshv-miR-K12-12* | | UGGGGGAGGGUGCCCUGGUUGA |
| 262. | kshv-miR-K12-8* | kshv-miR-K12-8* | | ACUCCCUCACUAACGCCCCGCU |

### Columns for Tables 3 and 4

(i) The SEQ ID NO: for the sequence of the mature, expressed miRNA biomarker.
(ii) The "miRNA name" column gives the name of the human miRNA as provided by the specialist database, miRBase, according to version 16 (released, August 2010).
(iii) The "Symbol" column gives the gene symbol which has been approved by the HGNC. The HGNC aims to give unique and meaningful names to every miRNA and human gene. An additional dash-number suffix indicates pre-miRNAs that lead to identical mature miRNAs but that are located at different places in the genome.
(iv) The HGNC aims to give unique and meaningful names to every miRNA (and human gene). The HGNC number thus identifies a unique human gene. Inclusion on to HUGO is for human genes only.

**Table 4**

| **No.** | **miRNA name⁽ⁱ⁾** | **Symbol⁽ⁱⁱ⁾** | **HGNC⁽ⁱⁱⁱ⁾** | **Sequence** |
|---|---|---|---|---|
| 126 | ebv-miR-BART2-5p | ebv-miR-BART2-5p | N/A | UAUUUUCUGCAUUCGCCCUUGC |
| 263. | hsa-miR-564 | MIR564 | 32820 | AGGCACGGUGUCAGCAGGC |
| 264. | hsa-let-7d-5p | MIRLET7D | 31481 | AGAGGUAGUAGGUUGCAUAGUU |
| 265. | hsa-let-7d-3p | MIRLET7D | 31481 | CUAUACGACCUGCUGCCUUUCU |
| 266. | hsa-miR-29a | MIR29A | 31616 | UAGCACCAUCUGAAAUCGGUUA |
| 157 | hsa-miR-142-3p | MIR142 | 31529 | CAUAAAGUAGAAAGCACUACU |
| 168 | hsa-miR-197 | MIR197 | 31569 | UUCACCACCUUCUCCACCCAGC |
| 172 | hsa-miR-215 | MIR215 | 31592 | AUGACCUAUGAAUUGACAGAC |
| 267. | hsa-miR-219-1-3p | MIR-219-1 | 31597 | AGAGUUGAGUCUGGACGUCCCG |
| 268. | hsa-miR-296-3p | MIR296 | 31617 | GAGGGUUGGGUGGAGGCUCUCC |
| 192 | hsa-miR-320c | MIR320C | 35248 | AAAAGCUGGGUUGAGAGGGU |
| 269. | hsa-miR-337-5p | MIR337 | 31774 | GAACGGCUUCAUACAGGAGUU |
| 270. | hsa-miR-369-3p | MIR369 | 31783 | AAUAAUACAUGGUUGAUCUUU |
| 271. | hsa-miR-450b-5p | MIR450B | 33642 | UUUUGCAAUAUGUUCCUGAAUA |
| 272. | hsa-miR-483-5p | MIR483 | 32340 | AAGACGGGAGGAAAGAAGGGAG |
| 273. | hsa-miR-507 | MIR507 | 32144 | UUUUGCACCUUUUGGAGUGAA |
| 274. | hsa-miR-517c | MIR517C | 32124 | AUCGUGCAUCCUUUUAGAGUGU |
| 275. | hsa-miR-519a | MIR519A1 / MIR519A2 | 32128 / 32132 | AAAGUGCAUCCUUUUAGAGUGU |
| 276. | hsa-miR-519d | MIR519D | 32112 | CAAAGUGCCUCCCUUUAGAGUG |
| 277. | hsa-miR-520g | MIR520G | 32116 | ACAAAGUGCUUCCCUUUAGAGUGU |
| 278. | hsa-miR-576-5p | MIR576 | 32832 | AUUCUAAUUUCUCCACGUCUUU |
| 279. | hsa-miR-577 | MIR577 | 32833 | UAGAUAAAAUAUUGGUACCUG |
| 280. | hsa-miR-604 | MIR604 | 32860 | AGGCUGCGGAAUUCAGGAC |
| 281. | hsa-miR-610 | MIR610 | 32866 | UGAGCUAAAUGUGUGCUGGGA |
| 282. | hsa-miR-619 | MIR619 | 32875 | GACCUGGACAUGUUUGUGCCCAGU |
| 283. | hsa-miR-1256 | MIR1256 | 35321 | AGGCAUUGACUUCUCACUAGCU |
| 284. | hsa-miR-1278 | MIR1278 | 35356 | UAGUACUGUGCAUAUCAUCUAU |
| 285. | hsa-miR-1297 | MIR1297 | 35289 | UUCAAGUAAUUCAGGUG |
| 286. | hcmv-miR-US33-5p | N/A | N/A | GAUUGUGCCCGGACCGUGGGCG |
| 287. | hsv1-miR-H1-5p | N/A | N/A | GAUGGAAGGACGGGAAGUGGA |
| 288. | hsa-miR-877 | MIR877 | 33660 | GUAGAGGAGAUGGCGCAGGG |
| 289. | hsa-let-7e-5p | MIRLET7E | 31482 | UGAGGUAGGAGGUUGUAUAGUU |
| 290. | hsa-let-7e-3p | MIRLET7E | 31482 | CUAUACGGCCUCCUAGCUUUCC |
| 128 | hsa-let-7f | MIRLET7F1 | 31483 | UGAGGUAGUAGAUUGUAUAGUU |
| 130 | hsa-let-7g | MIRLET7G | 31485 | UGAGGUAGUAGUUUGUACAGUU |
| 291. | hsa-miR-9 | MIR9-1 / MIR9-2/ MIR9-3 | 31641 / 31642 / 31646 | UCUUUGGUUAUCUAGCUGUAUGA |
| 182 | hsa-miR-30b | MIR30a | 31624 | UGUAAACAUCCUCGACUGGAAG |
| 198 | hsa-miR-34a | MIR34A | 31635 | UGGCAGUGUCUUAGCUGGUUGU |
| 292. | hsa-miR-92b | MIR92B | 32920 | UAUUGCACUCGUCCCGGCCUCC |
| 293. | hsa-miR-96-5p | MIR96 | 31648 | UUUGGCACUAGCACAUUUUUGCU |
| 294. | hsa-miR-96-3p | MIR96 | 31648 | AAUCAUGUGCAGUGCCAAUAUG |
| 295. | hsa-miR-106a-5p | MIR106A | 31494 | AAAAGUGCUUACAGUGCAGGUAG |
| 296. | hsa-miR-106a-3p | MIR106A | 31494 | CUGCAAUGUAAGCACUUCUUAC |
| 297. | hsa-miR-17-5p | MIR17 | 31547 | CAAAGUGCUUACAGUGCAGGUAG |
| 298. | hsa-miR-17-3p | MIR17 | 31547 | ACUGCAGUGAAGGCACUUGUAG |
| 299. | hsa-miR-106b-5p | MIR106B | 31495 | UAAAGUGCUGACAGUGCAGAU |
| 300. | hsa-miR-106b-3p | MIR106B | 31495 | CCGCACUGUGGGUACUUGCUGC |
| 301. | hsa-miR-127-3p | MIR127 | 31509 | UCGGAUCCGUCUGAGCUUGGCU |
| 302. | hsa-miR-128 | MIR128-1 / MIR128-2 | 31510 / 31511 | UCACAGUGAACCGGUCUCUUU |
| 303. | hsa-miR-154 | MIR154 | 31541 | UAGGUUAUCCGUGUUGCCUUCG |
| 304. | hsa-miR-155 | MIR155 | 31542 | UUAAUGCUAAUCGUGAUAGGGGU |
| 305. | hsa-miR-183-5p | MIR183 | 31554 | UAUGGCACUGGUAGAAUUCACU |
| 306. | hsa-miR-183-3p | MIR183 | 31554 | GUGAAUUACCGAAGGGCCAUAA |
| 307. | hsa-miR-194 | MIR194-1 / MIR194-2 | 31564 / 31565 | UGUAACAGCAACUCCAUGUGGA |
| 308. | hsa-miR-196b-5p | MIR196B | 31790 | UAGGUAGUUUCCUGUUGUUGGG |
| 309. | hsa-miR-196b-3p | MIR196B | 31790 | UCGACAGCACGACACUGCCUUC |
| 310. | hsa-miR-203 | MIR203 | 31581 | GUGAAAUGUUUAGGACCACUAG |
| 311. | hsa-miR-204 | MIR204 | 31582 | UUCCCUUUGUCAUCCUAUGCCU |
| 312. | hsa-miR-221-5p | MIR221 | 31601 | ACCUGGCAUACAAUGUAGAUUU |
| 313. | hsa-miR-221-3p | MIR221 | 31601 | AGCUACAUUGUCUGCUGGGUUUC |
| 174 | hsa-miR-223 | MIR223 | 31603 | UGUCAGUUUGUCAAAUACCCCA |
| 314. | hsa-miR-376a | MIR376A1 / MIR376A2 | 31869 / 32532 | AUCAUAGAGGAAAAUCCACGU |
| 315. | hsa-miR-376c | MIR376C | 31782 | AACAUAGAGGAAAUUCCACGU |
| 316. | hsa-miR-377 | MIR377 | 31870 | AUCACACAAAGGCAACUUUUGU |
| 317. | hsa-miR-379 | MIR379 | 31872 | UGGUAGACUAUGGAACGUAGG |
| 318. | hsa-miR-423-3p | MIR423 | 31880 | AGCUCGGUCUGAGGCCCCUCAGU |
| 319. | hsa-miR-424-5p | MIR424 | 31881 | CAGCAGCAAUUCAUGUUUUGAA |
| 320. | hsa-miR-424-3p | MIR424 | 31881 | CAAAACGUGAGGCGCUGCUAU |
| 321. | hsa-miR-425-5p | MIR425 | 31882 | AAUGACACGAUCACUCCCGUUGA |
| 322. | hsa-miR-425-3p | MIR425 | 31882 | AUCGGGAAUGUCGUGUCCGCCC |
| 323. | hsa-miR-454-5p | MIR454 | 33137 | ACCCUAUCAAUAUUGUCUCUGC |
| 324. | hsa-miR-454-3p | MIR454 | 33137 | UAGUGCAAUAUUGCUUAUAGGGU |
| 325. | hsa-miR-486-3p | hsa-miR-486 MIR486 | 32342 | CGGGGCAGCUCAGUACAGGAU |
| 326. | hsa-miR-509-3p | MIR509-3 | 33675 | UGAUUGGUACGUCUGUGGGUAG |
| 327. | hsa-miR-514 | MIR514-1 / MIR514-2 / MIR514-3 | 32148 / 32149 / 32150 | AUUGACACUUCUGUGAGUAGA |
| 328. | hsa-miR-542-3p | MIR542 | 32534 | UGUGACAGAUUGAUAACUGAAA |
| 329. | hsa-miR-545-5p | MIR545 | 32531 | UCAGUAAAUGUUUAUUAGAUGA |
| 330. | hsa-miR-545-3p | MIR545 | 32531 | UCAGCAAACAUUUAUUGUGUGC |
| 331. | hsa-miR-626 | MIR626 | 32882 | AGCUGUCUGAAAAUGUCUU |
| 236 | hsa-miR-630 | MIR630 | 32886 | AGUAUUCUGUACCAGGGAAGGU |
| 241 | hsa-miR-663 | MIR663A | 32919 | AGGCGGGGCGCCGCGGGACCGC |
| 243 | hsa-miR-720 | MIR720 | 35375 | UCUCGCUGGGGCCUCCA |
| 332. | hsa-miR-758-5p | MIR758 | 33133 | GAUGGUUGACCAGAGAGCACAC |
| 333. | hsa-miR-758-3p | MIR758 | 33133 | UUUGUGACCUGGUCCACUAACC |
| 334. | hsa-miR-876-3p | MIR876 | 33653 | UGGUGGUUUACAAAGUAAUUCA |
| 335. | hsa-miR-1185 | MIR1185-1 / MIR1185-2 | 35257 / 35254 | AGAGGAUACCCUUUGUAUGUU |
| 146 | hsa-miR-1260 | MIR1260A | 35325 | AUCCCACCUCUGCCACCA |
| 336. | hsa-miR-1266-5p | MIR1266 | 35334 | CCUCAGGGCUGUAGAACAGGGCU |
| 337. | hsa-miR-1266-3p | MIR1266 | 35334 | CCCUGUUCUAUGCCCUGAGGGA |
| 338. | hsa-miR-199a-3p | MIR199A1 / MIR199A2 | 31571 / 31572 | ACAGUAGUCUGCACAUUGGUUA |
| 339. | hsa-miR-199b-3p | MIR199B | 31573 | ACAGUAGUCUGCACAUUGGUUA |
| 224 | hsa-miR-497 | MIR497 | 32088 | CAGCAGCACACUGUGGUUUGU |
| 340. | hsa-miR-625-5p | MIR625 | 32881 | AGGGGGAAAGUUCUAUAGUCC |
| 341. | hsa-miR-625-3p | MIR625 | 32881 | GACUAUAGAACUUUCCCCCUCA |
| 342. | hsa-miR-631 | MIR631 | 32887 | AGACCUGGCCCAGACCUCAGC |
| 343. | hsa-miR-635 | MIR635 | 32891 | ACUUGGGCACUGAAACAAUGUCC |

**Table 5**

| **No. (i)** | **Symbol (ii)** | **ID (iii)** | **Name (iv)** | **HGNC (v)** | **GI (vi)** |
|---|---|---|---|---|---|
| 344. | CDC42 | 998 | Homo sapiens cell division cycle 42 (GTP binding protein 25kDa) transcript variant 1 | 1736 | 33990903 |
| 345. | EGFR_in t | 1956 | Homo sapiens epidermal growth factor receptor (erythroblastic leukemia viral (v-erb-b) oncogene homolog avian) | 3236 | 63101669 |
| 346. | KIT_ext | 3815 | Homo sapiens v-kit Hardy-Zuckerman 4 feline sarcoma viral oncogene homolog | 6342 | 47938801 |
| 347. | PPARG | 5468 | Homo sapiens peroxisome proliferative activated receptor gamma transcript variant 3 | 9236 | 13905055 |
| 348. | WT1 | 7490 | Homo sapiens Wilms tumor 1 | 12796 | 34190661 |

### Columns

(i)-(vi) are the same as those in Table 2.

**Table 6**

| **Symbol (i)** | **ID (ii)** | **P.Value (iii)** | **Expression (iv)** |
|---|---|---|---|
| CCNB1IP1 | 57820 | 8.16E-07 | Down |
| HSPD1 | 3329 | 1.96E-06 | Down |
| PRPF4 | 9128 | 2.96E-06 | Down |
| STYXL1 | 51657 | 2.22E-05 | Down |
| ETNK2 | 55224 | 2.23E-05 | Down |
| GALK1 | 2584 | 2.75E-05 | Down |
| RNF40 | 9810 | 2.95E-05 | Down |
| NTRK3_ext | 4916 | 4.98E-05 | Down |
| UBA1 | 7317 | 8.54E-05 | Up |
| GTF2H1 | 2965 | 9.23E-05 | Down |
| PDE4A | 5141 | 0.000115 | Down |
| SMARCE1 | 6605 | 0.000133 | Up |
| TBPL1 | 9519 | 0.000197 | Down |
| PIP4K2B | 8396 | 0.000252 | Down |
| LDB1 | 8861 | 0.000304 | Down |
| RHOT2 | 89941 | 0.00037 | Down |
| SMARCD1 | 6602 | 0.000384 | Down |
| SAV1 | 60485 | 0.000403 | Down |
| ZSCAN12 | 9753 | 0.000412 | Down |
| ASB1 | 51665 | 0.000414 | Down |
| KLHL12 | 59349 | 0.000428 | Up |
| UXT | 8409 | 0.000642 | Down |
| E2F6 | 1876 | 0.000747 | Up |
| RBMS1 | 5937 | 0.000829 | Up |
| MAP2K5 | 5607 | 0.000909 | Down |
| PAPSS2 | 9060 | 0.001015 | Down |
| RAN | 5901 | 0.001377 | Up |
| STUB1 | 10273 | 0.015531 | Up |
| TPM1 | 7168 | 0.022489 | Up |

### Columns (Tables 6 & 7)

(i) The "ID" column shows the Entrez GenelD number for the antigen marker. An Entrez GenelD value is unique across all taxa.
(ii) The "Symbol" column gives the gene symbol which has been approved by the HGNC. The HGNC aims to give unique and meaningful names to every miRNA and human gene.
(iii) The "p-value" represents the p-value of a microarray T-test derived from comparing case with control, as determined in study 1.
(iv) The biomarkers can be up-regulated (i.e. an increase in fold-change, when compared to control samples) or down-regulated (i.e. a decrease in fold-change, when compared to control samples), as determined in study 1.

**Table 7**

| **Symbol (i)** | **ID (ii)** | **P.Value (iii)** | **Expression (iv)** |
|---|---|---|---|
| TPM1 | 7168 | 0.002915091 | Up |
| RORC | 6097 | 0.006178466 | Down |
| HSPD1 | 3329 | 0.008954673 | Down |
| SRPK1 | 6732 | 0.015397291 | Down |
| PYGO2 | 90780 | 0.024303172 | Down |
| SOD2 | 6648 | 0.028978148 | Up |
| STUB1 | 10273 | 0.039435959 | Up |
| RAN | 5901 | 0.02988746 | Up |

**Table 8**

| **miRNA Name (i)** | **P.Value (ii)** | **Expression (iii)** |
|---|---|---|
| hsa-miR-150 | 6.39411E-05 | Down |
| hsa-miR-122 | 0.025339825 | Down |
| hsa-miR-342-3p | 3.34153E-05 | Down |
| hsa-miR-3648 | 0.005587809 | Up |
| hsa-miR-574-5p | 2.92134E-05 | Down |
| hsa-miR-1224-5p | 0.005657118 | Up |
| hsa-miR-483-3p | 1.0516E-07 | Down |
| hsa-miR-1290 | 0.027502289 | Down |
| hsa-miR-630 | 7.28115E-07 | Down |
| hsa-miR-4284 | 6.22332E-06 | Down |
| hsa-miR-4274 | 0.005782413 | Down |
| hsa-miR-1539 | 0.016898976 | Down |
| hsa-miR-484 | 5.86031E-05 | Down |
| hsa-miR-150* | 0.0001524 | Up |
| hsa-miR-103a | 0.000151533 | Down |
| hsa-miR-595 | 3.69681E-06 | Down |
| hsa-let-7f | 0.011036922 | Down |
| hsa-miR-2116* | 0.009660572 | Down |
| hsa-let-7g | 0.018048768 | Down |
| hsa-miR-10b | 0.000371424 | Up |
| hsa-miR-557 | 0.001717161 | Up |
| hsa-miR-574-3p | 1.5527E-05 | Down |
| hsa-miR-29b | 0.021302443 | Down |
| hsa-miR-3675-3p | 0.02426579 | Down |
| hsa-miR-3196 | 0.000777606 | Up |
| hsa-miR-142-3p | 0.001847633 | Down |
| hsa-miR-762 | 0.003100267 | Up |
| hsv2-miR-H22 | 0.017619039 | Up |
| hsa-miR-1260 | 2.30838E-06 | Down |
| hsv1-miR-H17 | 0.047904609 | Up |
| hsa-miR-1281 | 7.78815E-06 | Down |
| hsa-miR-720 | 1.08053E-05 | Down |
| ebv-miR-BART12 | 5.95912E-05 | Down |
| hsa-miR-146a | 0.043521819 | Down |
| hsa-miR-1183 | 0.011113703 | Up |
| hsa-miR-1228* | 8.27861E-07 | Down |
| hsa-miR-3131 | 0.000773528 | Up |
| hsa-miR-1825 | 1.48649E-06 | Down |
| hsa-miR-30b | 0.006449318 | Down |
| kshv-miR-K12-12* | 2.87415E-05 | Down |
| hsv2-miR-H6 | 0.008254315 | Up |
| hsa-miR-26a | 2.87528E-05 | Down |
| hsa-miR-4281 | 8.77129E-05 | Up |
| hsa-miR-3911 | 0.012758018 | Up |
| ebv-miR-BART16 | 4.54055E-05 | Down |
| hsv1-miR-H1* | 3.24311E-06 | Down |
| hsa-miR-1202 | 0.000551047 | Up |
| hsa-miR-3610 | 0.006875895 | Up |
| hsv1-miR-H6-3p | 4.84168E-05 | Down |
| hsa-miR-3138 | 0.008993721 | Up |
| hsa-miR-1207-5p | 0.002819455 | Up |
| hsa-miR-3149 | 9.53187E-05 | Down |
| hsa-miR-4270 | 4.37146E-05 | Up |
| hsa-miR-3195 | 0.006823245 | Up |
| hsv1-miR-H8 | 0.002026819 | Down |
| hsa-miR-4257 | 0.002878782 | Down |
| hsa-miR-1225-5p | 3.94558E-05 | Up |
| hsa-miR-142-5p | 0.022979438 | Down |
| hsa-miR-320c | 0.004906198 | Up |
| hsa-miR-466 | 0.00012439 | Down |
| hsa-miR-3665 | 0.021943146 | Down |
| hsa-miR-3180-5p | 7.83908E-06 | Down |
| hsa-miR-572 | 3.35607E-05 | Down |
| hsa-miR-498 | 0.038996714 | Down |
| hsv1-miR-H7* | 1.53033E-06 | Down |
| hsa-miR-4290 | 8.97207E-05 | Down |
| hsa-miR-766 | 5.28053E-05 | Down |
| hsa-miR-550a | 2.463E-06 | Down |
| hsa-miR-642b | 0.000388557 | Up |
| hiv1-miR-H1 | 0.023890902 | Up |
| hsa-miR-4286 | 9.71844E-06 | Down |
| hsa-miR-1275 | 0.028532583 | Up |
| hsa-miR-328 | 5.89449E-06 | Down |
| hsa-miR-940 | 0.000858136 | Down |
| hsa-miR-425* | 1.95809E-06 | Down |
| hsa-miR-877* | 1.97343E-06 | Down |
| hsa-miR-1237 | 0.004737656 | Down |
| hsa-miR-197 | 0.006897737 | Down |
| hsa-miR-30a | 0.002379932 | Up |
| hsa-miR-23c | 8.16183E-05 | Down |
| hsa-miR-3652 | 8.4982E-05 | Down |
| hsa-miR-22 | 0.0051271 | Up |
| hsa-miR-181a | 0.001321192 | Down |
| hsa-miR-3663-3p | 0.022163221 | Up |
| hsv2-miR-H25 | 0.018411737 | Up |
| hsa-miR-485-3p | 3.09662E-05 | Down |
| hsa-miR-324-3p | 0.000347692 | Down |
| hsa-miR-486-5p | 0.008641648 | Up |
| hsa-miR-1238 | 0.001191715 | Down |
| hsa-miR-1280 | 0.000217071 | Down |
| hsa-miR-129* | 0.024770209 | Down |
| hsa-miR-1225-3p | 0.000183052 | Down |
| hsa-let-7f-1* | 0.0106806 | Down |
| hsa-miR-4313 | 0.000725719 | Down |
| hsa-miR-4323 | 0.000144211 | Down |
| hsa-miR-149 | 9.57722E-05 | Down |
| hsa-miR-199a-5p | 0.038394728 | Down |
| hsa-miR-19b | 0.034029748 | Up |
| hsa-miR-92a | 0.031607002 | Up |
| hsv2-miR-H6* | 6.38059E-05 | Down |
| hsa-miR-16-2* | 0.03334112 | Up |
| hsa-miR-933 | 1.91512E-07 | Down |
| hsa-miR-660 | 0.007009129 | Up |
| hsa-miR-494 | 0.049102428 | Down |
| hsa-miR-1260b | 0.000504765 | Down |
| hsa-miR-33b* | 2.24675E-06 | Down |
| hsa-miR-186 | 0.021144881 | Up |
| hsa-let-7b* | 0.000296882 | Down |
| hsa-miR-424 | 0.028889653 | Up |
| kshv-miR-K12-8* | 2.07786E-05 | Down |
| hsa-miR-191* | 0.002534839 | Down |
| hsa-miR-1234 | 0.0094781 | Down |

### Columns (Tables 8 & 9)

(i) The "miRNA name" column gives the name of the human miRNA as provided by the specialist database, miRBase, according to version 16 (released, August 2010).
(ii) The "p-value" represents the p-value of a microarray T-test derived from comparing case with control, as determined in study 1.
(iii) The biomarkers can be up-regulated (i.e. an increase in fold-change, when compared to control samples) or down-regulated (i.e. a decrease in fold-change, when compared to control samples), as determined in study 1.

**Table 9**

| **miRNA Name (i)** | **P.Value (ii)** | **Expression (iii)** |
|---|---|---|
| hsa-miR-150 | 0.000731818 | Down |
| hsa-miR-122 | 0.001736842 | Down |
| hsa-miR-1224-5p | 0.005657118 | Up |
| hsa-miR-342-3p | 0.007122454 | Down |
| hsa-miR-3648 | 0.038141456 | Up |
| hsa-miR-574-5p | 0.043514783 | Down |

**Table 10**

| **Biomarker** | **AUC** | **S+S (i)** | **Sensitivity (ii)** | **Specificity (iii)** | **Assay (iv)** |
|---|---|---|---|---|---|
| hsa-miR-122 | 0.66 | 1.25 | 0.4 | 0.85 | qPCR |
| | 0.65 | 1.19 | 0.55 | 0.65 | microarray |
| hsa-miR-150 | 0.8 | 1.44 | 0.8 | 0.65 | microarray |
| | 0.69 | 1.31 | 0.47 | 0.85 | qPCR |
| hsa-miR-342-3p | 0.78 | 1.42 | 0.66 | 0.76 | microarray |
| | 0.62 | 1.14 | 0.76 | 0.38 | qPCR |
| hsa-miR-1224-5p | 0.48 | 1.06 | 0.06 | 1 | microarray |
| hsa-m iR-3194-5p | 0.62 | 1.14 | 0.68 | 0.46 | qPCR |
| hsa-miR-3648 | 0.63 | 1.21 | 0.85 | 0.35 | microarray |
| hsa-miR-3663-5p | 0.7 | 1.26 | 0.41 | 0.85 | qPCR |
| hsa-miR-574-5p | 0.8 | 1.43 | 0.73 | 0.71 | microarray |
| HSPD1 | 0.62 | 1.15 | 0.81 | 0.33 | autoAb |
| RAN | 0.54 | 1.1 | 0.87 | 0.22 | autoAb |
| STUB1 | 0.66 | 1.21 | 0.65 | 0.56 | autoAb |
| TPM1 | 0.67 | 1.25 | 0.69 | 0.56 | autoAb |

### Columns (Tables 10 to 14)

(i) S+S is the sum of the sensitivity and specificity columns, as determined in study 1.
(ii) and (iii) These two columns show the sensitivity and specificity of a test based solely on the relevant biomarker (or, for Tables 11-15, panel) shown in the left-hand column in the same row when applied to the samples used in study 1.
(iv) For miRNA analysis, data was generated using either a microarray ("microarray") or qPCR ("qPCR") platform, as described in study 1. Autoantbody ("autoAb") biomarkers were identified using the protein array platform described in study 1. Where panels were developed incorporating both miRNA and autoantibody biomarkers as variables (see study 1), these are described as "combiAbMir".

**Table 11 (2-mers)**

| **Panel** | **AUC** | **S+S (i)** | **Sensitivity (ii)** | **Specificity (iii)** | **Assay (iv)** |
|---|---|---|---|---|---|
| hsa-miR-150, hsa-miR-574-5p | 0.91 | 1.63 | 0.86 | 0.76 | microarray |
| hsa-miR-342-3p, hsa-miR-574-5p | 0.86 | 1.52 | 0.76 | 0.76 | microarray |
| hsa-miR-122, hsa-miR-574-5p | 0.84 | 1.51 | 0.75 | 0.76 | microarray |
| hsa-miR-3648, hsa-miR-574-5p | 0.83 | 1.49 | 0.79 | 0.71 | microarray |
| hsa-miR-150, hsa-miR-342-3p | 0.81 | 1.45 | 0.68 | 0.76 | microarray |
| TPM1, hsa-miR-150 | 0.811875 | 1.4825 | 0.8575 | 0.625 | combiAbMir |
| hsa-miR-342-3p, hsa-miR-3648 | 0.81 | 1.45 | 0.69 | 0.76 | microarray |
| RAN, hsa-miR-574-5p | 0.810156 | 1.430625 | 0.805625 | 0.625 | combiAbMir |
| HSPD1, hsa-miR-342-3p | 0.809219 | 1.450625 | 0.575625 | 0.875 | combiAbMir |
| RAN, hsa-miR-342-3p | 0.807813 | 1.459375 | 0.709375 | 0.75 | combiAbMir |
| TPM1, hsa-miR-342-3p | 0.806406 | 1.445625 | 0.695625 | 0.75 | combiAbMir |
| hsa-miR-1224-5p, hsa-miR-342-3p | 0.80 | 1.45 | 0.68 | 0.76 | microarray |
| TPM1, hsa-miR-574-5p | 0.796406 | 1.435 | 0.81 | 0.625 | combiAbMir |
| HSPD1, hsa-miR-150 | 0.795625 | 1.42875 | 0.67875 | 0.75 | combiAbMir |
| RAN, hsa-miR-150 | 0.784063 | 1.385625 | 0.760625 | 0.625 | combiAbMir |
| hsa-miR-1224-5p, hsa-miR-574-5p | 0.78 | 1.42 | 0.66 | 0.76 | microarray |
| hsa-miR-150, hsa-miR-3648 | 0.78 | 1.39 | 0.74 | 0.65 | microarray |
| hsa-miR-150, hsa-miR-1224-5p | 0.78 | 1.39 | 0.68 | 0.71 | microarray |
| STUB1, hsa-miR-150 | 0.777344 | 1.404375 | 0.654375 | 0.75 | combiAbMir |
| HSPD1, hsa-miR-574-5p | 0.775 | 1.3875 | 0.7625 | 0.625 | combiAbMir |
| STUB1, hsa-miR-342-3p | 0.774531 | 1.41125 | 0.66125 | 0.75 | combiAbMir |
| hsa-miR-122, hsa-miR-342-3p | 0.77 | 1.40 | 0.63 | 0.76 | microarray |
| STUB1, hsa-miR-574-5p | 0.769688 | 1.38 | 0.63 | 0.75 | combiAbMir |
| hsa-miR-150, hsa-miR-122 | 0.77 | 1.38 | 0.61 | 0.76 | microarray |
| hsa-miR-122, hsa-miR-3663-5p | 0.76 | 1.37 | 0.60 | 0.77 | qPCR |
| hsa-miR-150, hsa-miR-3663-5p | 0.75 | 1.35 | 0.50 | 0.85 | qPCR |
| hsa-miR-342-3p, hsa-miR-3663-5p | 0.72 | 1.30 | 0.60 | 0.69 | qPCR |
| TPM1, hsa-miR-122 | 0.694688 | 1.28125 | 0.65625 | 0.625 | combiAbMir |
| TPM1, HSPD1 | 0.69 | 1.28 | 0.84 | 0.44 | autoAb |
| TPM1, hsa-miR-3648 | 0.684219 | 1.24 | 0.74 | 0.5 | combiAbMir |
| HSPD1, hsa-miR-3648 | 0.672656 | 1.254375 | 0.754375 | 0.5 | combiAbMir |
| hsa-miR-150, hsa-miR-3194-5p | 0.67 | 1.28 | 0.43 | 0.85 | qPCR |
| hsa-miR-122, hsa-miR-3648 | 0.67 | 1.23 | 0.82 | 0.41 | microarray |
| STUB1, hsa-miR-122 | 0.661094 | 1.265 | 0.64 | 0.625 | combiAbMir |
| RAN, hsa-miR-3648 | 0.661094 | 1.255625 | 0.880625 | 0.375 | combiAbMir |
| TPM1, STUB1 | 0.66 | 1.25 | 0.81 | 0.44 | autoAb |
| hsa-miR-3663-5p, hsa-miR-3194-5p | 0.66 | 1.18 | 0.64 | 0.54 | qPCR |
| HSPD1, STUB1 | 0.65 | 1.19 | 0.64 | 0.56 | autoAb |
| hsa-miR-342-3p, hsa-miR-3194-5p | 0.64 | 1.18 | 0.33 | 0.85 | qPCR |
| STUB1, hsa-miR-3648 | 0.639844 | 1.20125 | 0.82625 | 0.375 | combiAbMir |
| TPM1, hsa-miR-1224-5p | 0.639531 | 1.195625 | 0.570625 | 0.625 | combiAbMir |
| HSPD1, hsa-miR-122 | 0.639063 | 1.1925 | 0.4425 | 0.75 | combiAbMir |
| hsa-miR-122, hsa-miR-3194-5p | 0.64 | 1.26 | 0.41 | 0.85 | qPCR |
| HSPD1, RAN | 0.63 | 1.17 | 0.84 | 0.33 | autoAb |
| hsa-miR-150, hsa-miR-122 | 0.63 | 1.21 | 0.36 | 0.85 | qPCR |
| hsa-miR-150, hsa-miR-342-3p | 0.63 | 1.21 | 0.36 | 0.85 | qPCR |
| TPM1, RAN | 0.63 | 1.18 | 0.73 | 0.44 | autoAb |
| hsa-miR-122, hsa-miR-342-3p | 0.62 | 1.20 | 0.35 | 0.85 | qPCR |
| RAN, hsa-miR-122 | 0.617813 | 1.159375 | 0.534375 | 0.625 | combiAbMir |
| STUB1, hsa-miR-1224-5p | 0.612188 | 1.173125 | 0.423125 | 0.75 | combiAbMir |
| hsa-miR-122, hsa-miR-1224-5p | 0.59 | 1.11 | 0.70 | 0.41 | microarray |
| HSPD1, hsa-miR-1224-5p | 0.587031 | 1.120625 | 0.620625 | 0.5 | combiAbMir |
| RAN, STUB1 | 0.59 | 1.15 | 0.82 | 0.33 | autoAb |
| hsa-miR-1224-5p, hsa-miR-3648 | 0.58 | 1.11 | 0.81 | 0.29 | microarray |
| RAN, hsa-miR-1224-5p | 0.537344 | 1.084375 | 0.834375 | 0.25 | combiAbMir |

**Table 12 (3-mers)**

| **Panel** | **AUC** | **S+S (i)** | **Sensitivity (ii)** | **Specificity (iii)** | **Assay (iv)** |
|---|---|---|---|---|---|
| RAN, hsa-miR-150, hsa-miR-574-5p | 0.923594 | 1.6575 | 0.7825 | 0.875 | combiAbMir |
| TPM1, hsa-miR-150, hsa-miR-574-5p | 0.923594 | 1.655 | 0.905 | 0.75 | combiAbMir |
| hsa-miR-150, hsa-miR-342-3p, hsa-miR-574-5p | 0.90 | 1.61 | 0.85 | 0.76 | microarray |
| STUB1, hsa-miR-150, hsa-miR-574-5p | 0.900781 | 1.61625 | 0.86625 | 0.75 | combiAbMir |
| hsa-miR-150, hsa-miR-122, hsa-miR-574-5p | 0.90 | 1.60 | 0.83 | 0.76 | microarray |
| HSPD1, hsa-miR-150, hsa-miR-574-5p | 0.897188 | 1.600625 | 0.850625 | 0.75 | combiAbMir |
| hsa-miR-122, hsa-miR-342-3p, hsa-miR-574-5p | 0.90 | 1.60 | 0.78 | 0.82 | microarray |
| TPM1, hsa-miR-342-3p, hsa-miR-574-5p | 0.892969 | 1.57125 | 0.69625 | 0.875 | combiAbMir |
| hsa-miR-150, hsa-miR-1224-5p, hsa-miR-574-5p | 0.89 | 1.59 | 0.83 | 0.76 | microarray |
| hsa-miR-150, hsa-miR-3648, hsa-miR-574-5p | 0.89 | 1.59 | 0.83 | 0.76 | microarray |
| RAN, hsa-miR-342-3p, hsa-miR-574-5p | 0.878906 | 1.55375 | 0.67875 | 0.875 | combiAbMir |
| hsa-miR-1224-5p, hsa-miR-342-3p, hsa-miR-574-5p | 0.88 | 1.57 | 0.81 | 0.76 | microarray |
| TPM1, hsa-miR-122, hsa-miR-574-5p | 0.872344 | 1.55 | 0.8 | 0.75 | combiAbMir |
| STUB1, hsa-miR-342-3p, hsa-miR-574-5p | 0.867031 | 1.535 | 0.785 | 0.75 | combiAbMir |
| RAN, hsa-miR-122, hsa-miR-574-5p | 0.864219 | 1.52875 | 0.77875 | 0.75 | combiAbMir |
| hsa-miR-342-3p, hsa-miR-3648, hsa-miR-574-5p | 0.86 | 1.54 | 0.77 | 0.76 | microarray |
| HSPD1, hsa-miR-342-3p, hsa-miR-574-5p | 0.862656 | 1.51875 | 0.76875 | 0.75 | combiAbMir |
| RAN, hsa-miR-3648, hsa-miR-574-5p | 0.860781 | 1.515625 | 0.765625 | 0.75 | combiAbMir |
| hsa-miR-122, hsa-miR-3648, hsa-miR-574-5p | 0.85 | 1.51 | 0.75 | 0.76 | microarray |
| TPM1, hsa-miR-3648, hsa-miR-574-5p | 0.8425 | 1.518125 | 0.768125 | 0.75 | combiAbMir |
| TPM1, RAN, hsa-miR-574-5p | 0.825156 | 1.45875 | 0.70875 | 0.75 | combiAbMir |
| hsa-miR-122, hsa-miR-1224-5p, hsa-miR-574-5p | 0.82 | 1.49 | 0.72 | 0.76 | microarray |
| HSPD1, hsa-miR-122, hsa-miR-574-5p | 0.821719 | 1.46125 | 0.71125 | 0.75 | combiAbMir |
| STUB1, hsa-miR-122, hsa-miR-574-5p | 0.819375 | 1.47125 | 0.72125 | 0.75 | combiAbMir |
| TPM1, RAN, hsa-miR-342-3p | 0.815781 | 1.4575 | 0.5825 | 0.875 | combiAbMir |
| HSPD1, RAN, hsa-miR-574-5p | 0.814063 | 1.455 | 0.83 | 0.625 | combiAbMir |
| RAN, hsa-miR-342-3p, hsa-miR-3648 | 0.811094 | 1.43875 | 0.68875 | 0.75 | combiAbMir |
| TPM1, STUB1, hsa-miR-574-5p | 0.810938 | 1.454375 | 0.829375 | 0.625 | combiAbMir |
| TPM1, RAN, hsa-miR-150 | 0.810625 | 1.475 | 0.85 | 0.625 | combiAbMir |
| TPM1, HSPD1, hsa-miR-342-3p | 0.809688 | 1.473125 | 0.723125 | 0.75 | combiAbMir |
| RAN, hsa-miR-1224-5p, hsa-miR-574-5p | 0.80875 | 1.45625 | 0.70625 | 0.75 | combiAbMir |
| hsa-miR-1224-5p, hsa-miR-3648, hsa-miR-574-5p | 0.81 | 1.44 | 0.74 | 0.71 | microarray |
| HSPD1, hsa-miR-342-3p, hsa-miR-3648 | 0.803438 | 1.44 | 0.69 | 0.75 | combiAbMir |
| RAN, hsa-miR-150, hsa-miR-342-3p | 0.802813 | 1.456875 | 0.581875 | 0.875 | combiAbMir |
| TPM1, hsa-miR-150, hsa-miR-342-3p | 0.802188 | 1.436875 | 0.686875 | 0.75 | combiAbMir |
| TPM1, HSPD1, hsa-miR-574-5p | 0.802031 | 1.435 | 0.81 | 0.625 | combiAbMir |
| TPM1, hsa-miR-342-3p, hsa-miR-3648 | 0.801094 | 1.439375 | 0.689375 | 0.75 | combiAbMir |
| TPM1, HSPD1, hsa-miR-150 | 0.800625 | 1.45375 | 0.82875 | 0.625 | combiAbMir |
| STUB1, hsa-miR-3648, hsa-miR-574-5p | 0.800469 | 1.425625 | 0.675625 | 0.75 | combiAbMir |
| hsa-miR-150, hsa-miR-1224-5p, hsa-miR-342-3p | 0.80 | 1.42 | 0.65 | 0.76 | microarray |
| HSPD1, RAN, hsa-miR-150 | 0.799844 | 1.44 | 0.69 | 0.75 | combiAbMir |
| HSPD1, hsa-miR-150, hsa-miR-342-3p | 0.799219 | 1.419375 | 0.669375 | 0.75 | combiAbMir |
| RAN, hsa-miR-122, hsa-miR-342-3p | 0.798281 | 1.43125 | 0.68125 | 0.75 | combiAbMir |
| TPM1, hsa-miR-1224-5p, hsa-miR-574-5p | 0.798125 | 1.451875 | 0.826875 | 0.625 | combiAbMir |
| RAN, STUB1, hsa-miR-574-5p | 0.7975 | 1.415625 | 0.665625 | 0.75 | combiAbMir |
| TPM1, STUB1, hsa-miR-342-3p | 0.797188 | 1.435625 | 0.685625 | 0.75 | combiAbMir |
| TPM1, hsa-miR-122, hsa-miR-342-3p | 0.796719 | 1.423125 | 0.548125 | 0.875 | combiAbMir |
| TPM1, hsa-miR-150, hsa-miR-3648 | 0.794063 | 1.4625 | 0.8375 | 0.625 | combiAbMir |
| TPM1, hsa-miR-1224-5p, hsa-miR-342-3p | 0.793906 | 1.44375 | 0.56875 | 0.875 | combiAbMir |
| HSPD1, RAN, hsa-miR-342-3p | 0.793281 | 1.446875 | 0.571875 | 0.875 | combiAbMir |
| RAN, hsa-miR-1224-5p, hsa-miR-342-3p | 0.793125 | 1.425625 | 0.550625 | 0.875 | combiAbMir |
| HSPD1, STUB1, hsa-miR-342-3p | 0.7925 | 1.4325 | 0.6825 | 0.75 | combiAbMir |
| HSPD1, hsa-miR-150, hsa-miR-1224-5p | 0.790781 | 1.419375 | 0.669375 | 0.75 | combiAbMir |
| STUB1, hsa-miR-342-3p, hsa-miR-3648 | 0.786875 | 1.40875 | 0.65875 | 0.75 | combiAbMir |
| HSPD1, hsa-miR-3648, hsa-miR-574-5p | 0.781563 | 1.389375 | 0.889375 | 0.5 | combiAbMir |
| STUB1, hsa-miR-1224-5p, hsa-miR-574-5p | 0.781406 | 1.404375 | 0.654375 | 0.75 | combiAbMir |
| STUB1, hsa-miR-122, hsa-miR-342-3p | 0.78125 | 1.420625 | 0.545625 | 0.875 | combiAbMir |
| RAN, hsa-miR-150, hsa-miR-1224-5p | 0.78125 | 1.39 | 0.765 | 0.625 | combiAbMir |
| TPM1, hsa-miR-150, hsa-miR-122 | 0.778438 | 1.436875 | 0.811875 | 0.625 | combiAbMir |
| TPM1, STUB1, hsa-miR-150 | 0.7775 | 1.424375 | 0.799375 | 0.625 | combiAbMir |
| hsa-miR-150, hsa-miR-342-3p, hsa-miR-3648 | 0.78 | 1.40 | 0.63 | 0.76 | microarray |
| hsa-miR-150, hsa-miR-122, hsa-miR-342-3p | 0.78 | 1.40 | 0.64 | 0.76 | microarray |
| STUB1, hsa-miR-1224-5p, hsa-miR-342-3p | 0.775938 | 1.395625 | 0.520625 | 0.875 | combiAbMir |
| RAN, hsa-miR-150, hsa-miR-3648 | 0.775156 | 1.38125 | 0.75625 | 0.625 | combiAbMir |
| TPM1, hsa-miR-150, hsa-miR-1224-5p | 0.774063 | 1.4225 | 0.7975 | 0.625 | combiAbMir |
| hsa-miR-122, hsa-miR-1224-5p, hsa-miR-342-3p | 0.77 | 1.38 | 0.61 | 0.76 | microarray |
| STUB1, hsa-miR-150, hsa-miR-342-3p | 0.770938 | 1.398125 | 0.648125 | 0.75 | combiAbMir |
| HSPD1, hsa-miR-150, hsa-miR-3648 | 0.769531 | 1.38375 | 0.75875 | 0.625 | combiAbMir |
| HSPD1, STUB1, hsa-miR-574-5p | 0.766719 | 1.355 | 0.73 | 0.625 | combiAbMir |
| RAN, STUB1, hsa-miR-342-3p | 0.764844 | 1.38375 | 0.63375 | 0.75 | combiAbMir |
| HSPD1, hsa-miR-1224-5p, hsa-miR-342-3p | 0.764531 | 1.37375 | 0.49875 | 0.875 | combiAbMir |
| HSPD1, hsa-miR-122, hsa-miR-342-3p | 0.763438 | 1.393125 | 0.518125 | 0.875 | combiAbMir |
| hsa-miR-1224-5p, hsa-miR-342-3p, hsa-miR-3648 | 0.76 | 1.37 | 0.72 | 0.65 | microarray |
| hsa-miR-122, hsa-miR-342-3p, hsa-miR-3648 | 0.76 | 1.36 | 0.71 | 0.65 | microarray |
| HSPD1, hsa-miR-150, hsa-miR-122 | 0.761094 | 1.36875 | 0.61875 | 0.75 | combiAbMir |
| HSPD1, STUB1, hsa-miR-150 | 0.760938 | 1.3825 | 0.7575 | 0.625 | combiAbMir |
| STUB1, hsa-miR-150, hsa-miR-122 | 0.760625 | 1.364375 | 0.739375 | 0.625 | combiAbMir |
| hsa-miR-150, hsa-miR-1224-5p, hsa-miR-3648 | 0.76 | 1.35 | 0.71 | 0.65 | microarray |
| RAN, hsa-miR-150, hsa-miR-122 | 0.755625 | 1.35875 | 0.73375 | 0.625 | combiAbMir |
| HSPD1, hsa-miR-1224-5p, hsa-miR-574-5p | 0.754688 | 1.361875 | 0.736875 | 0.625 | combiAbMir |
| RAN, STUB1, hsa-miR-150 | 0.754531 | 1.371875 | 0.746875 | 0.625 | combiAbMir |
| STUB1, hsa-miR-150, hsa-miR-1224-5p | 0.754219 | 1.370625 | 0.745625 | 0.625 | combiAbMir |
| STUB1, hsa-miR-150, hsa-miR-3648 | 0.748906 | 1.351875 | 0.726875 | 0.625 | combiAbMir |
| hsa-miR-150, hsa-miR-122, hsa-miR-1224-5p | 0.75 | 1.35 | 0.65 | 0.71 | microarray |
| hsa-miR-150, hsa-miR-122, hsa-miR-3648 | 0.75 | 1.35 | 0.71 | 0.65 | microarray |
| hsa-miR-150, hsa-miR-3663-5p, hsa-miR-3194-5p | 0.74 | 1.34 | 0.65 | 0.69 | qPCR |
| TPM1, HSPD1, hsa-miR-122 | 0.730938 | 1.346875 | 0.721875 | 0.625 | combiAbMir |
| hsa-miR-122, hsa-miR-3663-5p, hsa-miR-3194-5p | 0.72 | 1.32 | 0.47 | 0.85 | qPCR |
| hsa-miR-150, hsa-miR-122, hsa-miR-3663-5p | 0.72 | 1.31 | 0.46 | 0.85 | qPCR |
| hsa-miR-122, hsa-miR-342-3p, hsa-miR-3663-5p | 0.72 | 1.32 | 0.63 | 0.69 | qPCR |
| hsa-miR-150, hsa-miR-342-3p, hsa-miR-3663-5p | 0.72 | 1.31 | 0.61 | 0.69 | qPCR |
| hsa-miR-342-3p, hsa-miR-3663-5p, hsa-m iR-3194-5p | 0.70 | 1.28 | 0.59 | 0.69 | qPCR |
| TPM1, HSPD1, hsa-miR-3648 | 0.7 | 1.280625 | 0.780625 | 0.5 | combiAbMir |
| TPM1, RAN, hsa-miR-3648 | 0.697656 | 1.258125 | 0.758125 | 0.5 | combiAbMir |
| TPM1, hsa-miR-122, hsa-miR-3648 | 0.69375 | 1.2575 | 0.7575 | 0.5 | combiAbMir |
| HSPD1, RAN, hsa-miR-3648 | 0.688125 | 1.288125 | 0.913125 | 0.375 | combiAbMir |
| HSPD1, STUB1, hsa-miR-3648 | 0.686406 | 1.24875 | 0.74875 | 0.5 | combiAbMir |
| TPM1, HSPD1, RAN | 0.68 | 1.28 | 0.83 | 0.44 | autoAb |
| TPM1, HSPD1, STUB1 | 0.68 | 1.29 | 0.84 | 0.44 | autoAb |
| TPM1, STUB1, hsa-miR-122 | 0.680781 | 1.289375 | 0.789375 | 0.5 | combiAbMir |
| RAN, hsa-miR-122, hsa-miR-3648 | 0.68 | 1.263125 | 0.888125 | 0.375 | combiAbMir |
| TPM1, hsa-miR-1224-5p, hsa-miR-3648 | 0.68 | 1.235 | 0.735 | 0.5 | combiAbMir |
| STUB1, hsa-miR-122, hsa-miR-3648 | 0.679375 | 1.2575 | 0.7575 | 0.5 | combiAbMir |
| HSPD1, hsa-miR-122, hsa-miR-3648 | 0.674531 | 1.21875 | 0.71875 | 0.5 | combiAbMir |
| TPM1, hsa-miR-122, hsa-miR-1224-5p | 0.674375 | 1.268125 | 0.643125 | 0.625 | combiAbMir |
| TPM1, STUB1, hsa-miR-3648 | 0.664375 | 1.234375 | 0.734375 | 0.5 | combiAbMir |
| RAN, STUB1, hsa-miR-3648 | 0.663594 | 1.245625 | 0.870625 | 0.375 | combiAbMir |
| TPM1, HSPD1, hsa-miR-1224-5p | 0.661875 | 1.22125 | 0.72125 | 0.5 | combiAbMir |
| TPM1, RAN, hsa-miR-122 | 0.655625 | 1.24 | 0.74 | 0.5 | combiAbMir |
| HSPD1, RAN, hsa-miR-122 | 0.655625 | 1.18375 | 0.80875 | 0.375 | combiAbMir |
| hsa-miR-150, hsa-miR-342-3p, hsa-miR-3194-5p | 0.65 | 1.25 | 0.41 | 0.85 | qPCR |
| hsa-miR-150, hsa-miR-122, hsa-miR-342-3p | 0.65 | 1.21 | 0.36 | 0.85 | qPCR |
| HSPD1, RAN, STUB1 | 0.65 | 1.21 | 0.76 | 0.44 | autoAb |
| HSPD1, STUB1, hsa-miR-122 | 0.648281 | 1.20875 | 0.58375 | 0.625 | combiAbMir |
| hsa-miR-122, hsa-miR-1224-5p, hsa-miR-3648 | 0.64 | 1.17 | 0.82 | 0.35 | microarray |
| hsa-miR-150, hsa-miR-122, hsa-miR-3194-5p | 0.64 | 1.25 | 0.41 | 0.85 | qPCR |
| TPM1, STUB1, hsa-miR-1224-5p | 0.635313 | 1.204375 | 0.704375 | 0.5 | combiAbMir |
| HSPD1, STUB1, hsa-miR-1224-5p | 0.63375 | 1.199375 | 0.699375 | 0.5 | combiAbMir |
| RAN, hsa-miR-1224-5p, hsa-miR-3648 | 0.632656 | 1.191875 | 0.816875 | 0.375 | combiAbMir |
| RAN, STUB1, hsa-miR-122 | 0.629063 | 1.196875 | 0.571875 | 0.625 | combiAbMir |
| TPM1, RAN, STUB1 | 0.62 | 1.19 | 0.64 | 0.56 | autoAb |
| TPM1, RAN, hsa-miR-1224-5p | 0.619219 | 1.151875 | 0.651875 | 0.5 | combiAbMir |
| hsa-miR-122, hsa-miR-342-3p, hsa-miR-3194-5p | 0.62 | 1.22 | 0.37 | 0.85 | qPCR |
| STUB1, hsa-miR-122, hsa-miR-1224-5p | 0.614688 | 1.1825 | 0.5575 | 0.625 | combiAbMir |
| HSPD1, hsa-miR-122, hsa-miR-1224-5p | 0.614063 | 1.158125 | 0.408125 | 0.75 | combiAbMir |
| HSPD1, RAN, hsa-miR-1224-5p | 0.613281 | 1.1725 | 0.7975 | 0.375 | combiAbMir |
| STUB1, hsa-miR-1224-5p, hsa-miR-3648 | 0.609219 | 1.155 | 0.78 | 0.375 | combiAbMir |
| HSPD1, hsa-miR-1224-5p, hsa-miR-3648 | 0.604375 | 1.15 | 0.9 | 0.25 | combiAbMir |
| RAN, hsa-miR-122, hsa-miR-1224-5p | 0.599063 | 1.11625 | 0.61625 | 0.5 | combiAbMir |
| RAN, STUB1, hsa-miR-1224-5p | 0.574531 | 1.136875 | 0.761875 | 0.375 | combiAbMir |

**Table 13 (4-mers)**

| **Panel** | **AUC** | **S+S (i)** | **Sensitivity (ii)** | **Specificity (iii)** | **Assay (iv)** |
|---|---|---|---|---|---|
| TPM1, hsa-miR-150, hsa-miR-342-3p, hsa-miR-574-5p | 0.928594 | 1.6525 | 0.9025 | 0.75 | combiAbMir |
| TPM1, hsa-miR-150, hsa-miR-122, hsa-miR-574-5p | 0.928125 | 1.649375 | 0.899375 | 0.75 | combiAbMir |
| RAN, hsa-miR-150, hsa-miR-3648, hsa-miR-574-5p | 0.925469 | 1.64625 | 0.77125 | 0.875 | combiAbMir |
| TPM1, RAN, hsa-miR-150, hsa-miR-574-5p | 0.923125 | 1.646875 | 0.896875 | 0.75 | combiAbMir |
| TPM1, STUB1, hsa-miR-150, hsa-miR-574-5p | 0.919844 | 1.635 | 0.76 | 0.875 | combiAbMir |
| RAN, hsa-miR-150, hsa-miR-122, hsa-miR-574-5p | 0.914375 | 1.635 | 0.76 | 0.875 | combiAbMir |
| TPM1, hsa-miR-150, hsa-miR-3648, hsa-miR-574-5p | 0.913594 | 1.6125 | 0.8625 | 0.75 | combiAbMir |
| RAN, STUB1, hsa-miR-150, hsa-miR-574-5p | 0.913438 | 1.635625 | 0.760625 | 0.875 | combiAbMir |
| TPM1, hsa-miR-150, hsa-miR-1224-5p, hsa-miR-574-5p | 0.913438 | 1.631875 | 0.881875 | 0.75 | combiAbMir |
| HSPD1, RAN, hsa-miR-150, hsa-miR-574-5p | 0.912031 | 1.62375 | 0.74875 | 0.875 | combiAbMir |
| RAN, hsa-miR-150, hsa-miR-342-3p, hsa-miR-574-5p | 0.908594 | 1.619375 | 0.869375 | 0.75 | combiAbMir |
| RAN, hsa-miR-150, hsa-miR-1224-5p, hsa-miR-574-5p | 0.906719 | 1.618125 | 0.868125 | 0.75 | combiAbMir |
| TPM1, hsa-miR-122, hsa-miR-342-3p, hsa-miR-574-5p | 0.906563 | 1.61125 | 0.86125 | 0.75 | combiAbMir |
| TPM1, HSPD1, hsa-miR-150, hsa-miR-574-5p | 0.906406 | 1.6125 | 0.8625 | 0.75 | combiAbMir |
| HSPD1, hsa-miR-150, hsa-miR-342-3p, hsa-miR-574-5p | 0.903906 | 1.6075 | 0.8575 | 0.75 | combiAbMir |
| STUB1, hsa-miR-150, hsa-miR-342-3p, hsa-miR-574-5p | 0.902031 | 1.615625 | 0.865625 | 0.75 | combiAbMir |
| TPM1, RAN, hsa-miR-342-3p, hsa-miR-574-5p | 0.901406 | 1.584375 | 0.834375 | 0.75 | combiAbMir |
| hsa-miR-150, hsa-miR-122, hsa-miR-3648, hsa-miR-574-5p | 0.90 | 1.61 | 0.84 | 0.76 | microarray |
| HSPD1, hsa-miR-150, hsa-miR-122, hsa-miR-574-5p | 0.900625 | 1.593125 | 0.843125 | 0.75 | combiAbMir |
| HSPD1, hsa-miR-150, hsa-miR-1224-5p, hsa-miR-574-5p | 0.898125 | 1.605625 | 0.855625 | 0.75 | combiAbMir |
| hsa-miR-150, hsa-miR-122, hsa-miR-342-3p, hsa-miR-574-5p | 0.90 | 1.59 | 0.83 | 0.76 | microarray |
| TPM1, HSPD1, hsa-miR-342-3p, hsa-miR-574-5p | 0.895781 | 1.58625 | 0.83625 | 0.75 | combiAbMir |
| HSPD1, STUB1, hsa-miR-150, hsa-miR-574-5p | 0.895313 | 1.6 | 0.85 | 0.75 | combiAbMir |
| TPM1, STUB1, hsa-miR-342-3p, hsa-miR-574-5p | 0.893125 | 1.579375 | 0.829375 | 0.75 | combiAbMir |
| STUB1, hsa-miR-150, hsa-miR-3648, hsa-miR-574-5p | 0.891563 | 1.595 | 0.845 | 0.75 | combiAbMir |
| HSPD1, hsa-miR-122, hsa-miR-342-3p, hsa-miR-574-5p | 0.890938 | 1.6075 | 0.7325 | 0.875 | combiAbMir |
| STUB1, hsa-miR-150, hsa-miR-122, hsa-miR-574-5p | 0.890625 | 1.58375 | 0.83375 | 0.75 | combiAbMir |
| HSPD1, hsa-miR-150, hsa-miR-3648, hsa-miR-574-5p | 0.889063 | 1.5925 | 0.8425 | 0.75 | combiAbMir |
| STUB1, hsa-miR-122, hsa-miR-342-3p, hsa-miR-574-5p | 0.888594 | 1.585625 | 0.835625 | 0.75 | combiAbMir |
| RAN, hsa-miR-122, hsa-miR-342-3p, hsa-miR-574-5p | 0.886719 | 1.6 | 0.725 | 0.875 | combiAbMir |
| hsa-miR-150, hsa-miR-1224-5p, hsa-miR-3648, hsa-miR-574-5p | 0.89 | 1.57 | 0.81 | 0.76 | microarray |
| RAN, hsa-miR-342-3p, hsa-miR-3648, hsa-miR-574-5p | 0.884375 | 1.560625 | 0.810625 | 0.75 | combiAbMir |
| STUB1, hsa-miR-150, hsa-miR-1224-5p, hsa-miR-574-5p | 0.882344 | 1.575625 | 0.825625 | 0.75 | combiAbMir |
| hsa-miR-150, hsa-miR-342-3p, hsa-miR-3648, hsa-miR-574-5p | 0.88 | 1.57 | 0.80 | 0.76 | microarray |
| RAN, STUB1, hsa-miR-342-3p, hsa-miR-574-5p | 0.88125 | 1.555625 | 0.680625 | 0.875 | combiAbMir |
| hsa-miR-150, hsa-miR-122, hsa-miR-1224-5p, hsa-miR-574-5p | 0.88 | 1.56 | 0.80 | 0.76 | microarray |
| TPM1, hsa-miR-342-3p, hsa-miR-3648, hsa-miR-574-5p | 0.880313 | 1.555625 | 0.805625 | 0.75 | combiAbMir |
| TPM1, hsa-miR-122, hsa-miR-3648, hsa-miR-574-5p | 0.877813 | 1.5725 | 0.8225 | 0.75 | combiAbMir |
| TPM1, hsa-miR-1224-5p, hsa-miR-342-3p, hsa-miR-574-5p | 0.877813 | 1.556875 | 0.806875 | 0.75 | combiAbMir |
| hsa-miR-150, hsa-miR-1224-5p, hsa-miR-342-3p, hsa-miR-574-5p | 0.88 | 1.57 | 0.81 | 0.76 | microarray |
| hsa-miR-122, hsa-miR-342-3p, hsa-miR-3648, hsa-miR-574-5p | 0.88 | 1.56 | 0.80 | 0.76 | microarray |
| HSPD1, STUB1, hsa-miR-342-3p, hsa-miR-574-5p | 0.874688 | 1.54625 | 0.79625 | 0.75 | combiAbMir |
| RAN, hsa-miR-1224-5p, hsa-miR-342-3p, hsa-miR-574-5p | 0.872031 | 1.565 | 0.69 | 0.875 | combiAbMir |
| HSPD1, hsa-miR-1224-5p, hsa-miR-342-3p, hsa-miR-574-5p | 0.870156 | 1.5525 | 0.8025 | 0.75 | combiAbMir |
| HSPD1, RAN, hsa-miR-342-3p, hsa-miR-574-5p | 0.87 | 1.546875 | 0.671875 | 0.875 | combiAbMir |
| hsa-miR-122, hsa-miR-1224-5p, hsa-miR-342-3p, hsa-miR-574-5p | 0.87 | 1.55 | 0.73 | 0.82 | microarray |
| TPM1, RAN, hsa-miR-3648, hsa-miR-574-5p | 0.8625 | 1.5425 | 0.7925 | 0.75 | combiAbMir |
| TPM1, hsa-miR-122, hsa-miR-1224-5p, hsa-miR-574-5p | 0.861094 | 1.536875 | 0.786875 | 0.75 | combiAbMir |
| STUB1, hsa-miR-1224-5p, hsa-miR-342-3p, hsa-miR-574-5p | 0.859844 | 1.530625 | 0.780625 | 0.75 | combiAbMir |
| hsa-miR-1224-5p, hsa-miR-342-3p, hsa-miR-3648, hsa-miR-574-5p | 0.86 | 1.52 | 0.76 | 0.76 | microarray |
| STUB1, hsa-miR-342-3p, hsa-miR-3648, hsa-miR-574-5p | 0.855156 | 1.5025 | 0.7525 | 0.75 | combiAbMir |
| RAN, hsa-miR-122, hsa-miR-3648, hsa-miR-574-5p | 0.854219 | 1.5 | 0.75 | 0.75 | combiAbMir |
| TPM1, HSPD1, hsa-miR-122, hsa-miR-574-5p | 0.852969 | 1.5 | 0.75 | 0.75 | combiAbMir |
| HSPD1, hsa-miR-342-3p, hsa-miR-3648, hsa-miR-574-5p | 0.850625 | 1.50625 | 0.75625 | 0.75 | combiAbMir |
| TPM1, RAN, hsa-miR-122, hsa-miR-574-5p | 0.848281 | 1.4975 | 0.7475 | 0.75 | combiAbMir |
| TPM1, STUB1, hsa-miR-122, hsa-miR-574-5p | 0.847813 | 1.509375 | 0.759375 | 0.75 | combiAbMir |
| HSPD1, hsa-miR-122, hsa-miR-3648, hsa-miR-574-5p | 0.846719 | 1.496875 | 0.621875 | 0.875 | combiAbMir |
| STUB1, hsa-miR-122, hsa-miR-3648, hsa-miR-574-5p | 0.841406 | 1.49875 | 0.74875 | 0.75 | combiAbMir |
| HSPD1, RAN, hsa-miR-122, hsa-miR-574-5p | 0.839531 | 1.470625 | 0.720625 | 0.75 | combiAbMir |
| RAN, hsa-miR-122, hsa-miR-1224-5p, hsa-miR-574-5p | 0.837969 | 1.50375 | 0.75375 | 0.75 | combiAbMir |
| HSPD1, hsa-miR-122, hsa-miR-1224-5p, hsa-miR-574-5p | 0.837188 | 1.4925 | 0.6175 | 0.875 | combiAbMir |
| hsa-miR-122, hsa-miR-1224-5p, hsa-miR-3648, hsa-miR-574-5p | 0.83 | 1.50 | 0.73 | 0.76 | microarray |
| TPM1, HSPD1, RAN, hsa-miR-342-3p | 0.832656 | 1.505 | 0.755 | 0.75 | combiAbMir |
| HSPD1, RAN, hsa-miR-3648, hsa-miR-574-5p | 0.832656 | 1.440625 | 0.690625 | 0.75 | combiAbMir |
| TPM1, RAN, hsa-miR-150, hsa-miR-342-3p | 0.832188 | 1.486875 | 0.736875 | 0.75 | combiAbMir |
| RAN, hsa-miR-1224-5p, hsa-miR-3648, hsa-miR-574-5p | 0.830625 | 1.468125 | 0.718125 | 0.75 | combiAbMir |
| TPM1, RAN, hsa-miR-342-3p, hsa-miR-3648 | 0.828125 | 1.485 | 0.735 | 0.75 | combiAbMir |
| TPM1, STUB1, hsa-miR-3648, hsa-miR-574-5p | 0.827969 | 1.476875 | 0.726875 | 0.75 | combiAbMir |
| RAN, STUB1, hsa-miR-122, hsa-miR-574-5p | 0.8275 | 1.470625 | 0.720625 | 0.75 | combiAbMir |
| HSPD1, STUB1, hsa-miR-122, hsa-miR-574-5p | 0.826563 | 1.470625 | 0.720625 | 0.75 | combiAbMir |
| RAN, STUB1, hsa-miR-3648, hsa-miR-574-5p | 0.823125 | 1.428125 | 0.678125 | 0.75 | combiAbMir |
| TPM1, HSPD1, STUB1, hsa-miR-342-3p | 0.822656 | 1.483125 | 0.733125 | 0.75 | combiAbMir |
| TPM1, hsa-miR-1224-5p, hsa-miR-3648, hsa-miR-574-5p | 0.822031 | 1.4675 | 0.7175 | 0.75 | combiAbMir |
| TPM1, HSPD1, hsa-miR-122, hsa-miR-342-3p | 0.821563 | 1.485625 | 0.735625 | 0.75 | combiAbMir |
| TPM1, HSPD1, RAN, hsa-miR-150 | 0.818906 | 1.47375 | 0.84875 | 0.625 | combiAbMir |
| TPM1, HSPD1, hsa-miR-342-3p, hsa-miR-3648 | 0.817813 | 1.45375 | 0.70375 | 0.75 | combiAbMir |
| TPM1, HSPD1, hsa-miR-3648, hsa-miR-574-5p | 0.817031 | 1.44375 | 0.81875 | 0.625 | combiAbMir |
| TPM1, HSPD1, hsa-miR-150, hsa-miR-342-3p | 0.815938 | 1.4775 | 0.7275 | 0.75 | combiAbMir |
| TPM1, HSPD1, RAN, hsa-miR-574-5p | 0.815 | 1.430625 | 0.805625 | 0.625 | combiAbMir |
| TPM1, HSPD1, STUB1, hsa-miR-574-5p | 0.812656 | 1.433125 | 0.808125 | 0.625 | combiAbMir |
| HSPD1, RAN, hsa-miR-342-3p, hsa-miR-3648 | 0.810781 | 1.43875 | 0.68875 | 0.75 | combiAbMir |
| TPM1, HSPD1, hsa-miR-150, hsa-miR-122 | 0.810625 | 1.455625 | 0.830625 | 0.625 | combiAbMir |
| TPM1, RAN, STUB1, hsa-miR-150 | 0.810156 | 1.470625 | 0.845625 | 0.625 | combiAbMir |
| TPM1, RAN, hsa-miR-1224-5p, hsa-miR-342-3p | 0.806094 | 1.44875 | 0.69875 | 0.75 | combiAbMir |
| TPM1, RAN, hsa-miR-150, hsa-miR-1224-5p | 0.805469 | 1.469375 | 0.844375 | 0.625 | combiAbMir |
| RAN, hsa-miR-150, hsa-miR-342-3p, hsa-miR-3648 | 0.805 | 1.424375 | 0.674375 | 0.75 | combiAbMir |
| TPM1, STUB1, hsa-miR-122, hsa-miR-342-3p | 0.803438 | 1.449375 | 0.699375 | 0.75 | combiAbMir |
| RAN, hsa-miR-150, hsa-miR-122, hsa-miR-342-3p | 0.802188 | 1.434375 | 0.559375 | 0.875 | combiAbMir |
| STUB1, hsa-miR-122, hsa-miR-1224-5p, hsa-miR-574-5p | 0.801875 | 1.463125 | 0.713125 | 0.75 | combiAbMir |
| HSPD1, RAN, hsa-miR-150, hsa-miR-1224-5p | 0.800781 | 1.4575 | 0.7075 | 0.75 | combiAbMir |
| TPM1, hsa-miR-122, hsa-miR-342-3p, hsa-miR-3648 | 0.800781 | 1.434375 | 0.684375 | 0.75 | combiAbMir |
| TPM1, RAN, hsa-miR-1224-5p, hsa-miR-574-5p | 0.8 | 1.42625 | 0.80125 | 0.625 | combiAbMir |
| TPM1, HSPD1, hsa-miR-1224-5p, hsa-miR-342-3p | 0.799375 | 1.44375 | 0.69375 | 0.75 | combiAbMir |
| TPM1, RAN, STUB1, hsa-miR-574-5p | 0.798281 | 1.415 | 0.79 | 0.625 | combiAbMir |
| TPM1, HSPD1, hsa-miR-150, hsa-miR-3648 | 0.796875 | 1.45125 | 0.82625 | 0.625 | combiAbMir |
| HSPD1, RAN, hsa-miR-1224-5p, hsa-miR-574-5p | 0.795938 | 1.43125 | 0.80625 | 0.625 | combiAbMir |
| HSPD1, RAN, hsa-miR-150, hsa-miR-342-3p | 0.795625 | 1.449375 | 0.574375 | 0.875 | combiAbMir |
| TPM1, HSPD1, STUB1, hsa-miR-150 | 0.794844 | 1.44125 | 0.81625 | 0.625 | combiAbMir |
| HSPD1, hsa-miR-150, hsa-miR-342-3p, hsa-miR-3648 | 0.79375 | 1.419375 | 0.669375 | 0.75 | combiAbMir |
| HSPD1, STUB1, hsa-miR-3648, hsa-miR-574-5p | 0.792813 | 1.4025 | 0.7775 | 0.625 | combiAbMir |
| HSPD1, hsa-miR-150, hsa-miR-122, hsa-miR-342-3p | 0.792656 | 1.426875 | 0.551875 | 0.875 | combiAbMir |
| HSPD1, hsa-miR-1224-5p, hsa-miR-3648, hsa-miR-574-5p | 0.7925 | 1.416875 | 0.791875 | 0.625 | combiAbMir |
| RAN, STUB1, hsa-miR-342-3p, hsa-miR-3648 | 0.7925 | 1.41125 | 0.66125 | 0.75 | combiAbMir |
| TPM1, RAN, hsa-miR-150, hsa-miR-3648 | 0.792344 | 1.4325 | 0.8075 | 0.625 | combiAbMir |
| HSPD1, RAN, STUB1, hsa-miR-574-5p | 0.790938 | 1.400625 | 0.775625 | 0.625 | combiAbMir |
| TPM1, hsa-miR-150, hsa-miR-342-3p, hsa-miR-3648 | 0.790469 | 1.405625 | 0.780625 | 0.625 | combiAbMir |
| TPM1, HSPD1, hsa-miR-150, hsa-miR-1224-5p | 0.790313 | 1.421875 | 0.796875 | 0.625 | combiAbMir |
| TPM1, HSPD1, hsa-miR-1224-5p, hsa-miR-574-5p | 0.790313 | 1.415625 | 0.790625 | 0.625 | combiAbMir |
| HSPD1, STUB1, hsa-miR-150, hsa-miR-342-3p | 0.790156 | 1.43125 | 0.68125 | 0.75 | combiAbMir |
| HSPD1, RAN, STUB1, hsa-miR-342-3p | 0.788438 | 1.424375 | 0.549375 | 0.875 | combiAbMir |
| TPM1, STUB1, hsa-miR-150, hsa-miR-342-3p | 0.788281 | 1.41375 | 0.66375 | 0.75 | combiAbMir |
| TPM1, hsa-miR-150, hsa-miR-1224-5p, hsa-miR-342-3p | 0.7875 | 1.406875 | 0.781875 | 0.625 | combiAbMir |
| HSPD1, STUB1, hsa-miR-150, hsa-miR-122 | 0.787031 | 1.435625 | 0.685625 | 0.75 | combiAbMir |
| HSPD1, STUB1, hsa-miR-342-3p, hsa-miR-3648 | 0.785938 | 1.414375 | 0.664375 | 0.75 | combiAbMir |
| RAN, hsa-miR-150, hsa-miR-1224-5p, hsa-miR-342-3p | 0.785313 | 1.42 | 0.545 | 0.875 | combiAbMir |
| TPM1, STUB1, hsa-miR-342-3p, hsa-miR-3648 | 0.785313 | 1.40375 | 0.65375 | 0.75 | combiAbMir |
| HSPD1, hsa-miR-1224-5p, hsa-miR-342-3p, hsa-miR-3648 | 0.785156 | 1.410625 | 0.660625 | 0.75 | combiAbMir |
| hsa-miR-150, hsa-miR-122, hsa-miR-342-3p, hsa-miR-3648 | 0.78 | 1.39 | 0.75 | 0.65 | microarray |
| TPM1, STUB1, hsa-miR-1224-5p, hsa-miR-342-3p | 0.783906 | 1.424375 | 0.674375 | 0.75 | combiAbMir |
| STUB1, hsa-miR-1224-5p, hsa-miR-3648, hsa-miR-574-5p | 0.78375 | 1.404375 | 0.654375 | 0.75 | combiAbMir |
| HSPD1, hsa-miR-150, hsa-miR-122, hsa-miR-1224-5p | 0.783281 | 1.419375 | 0.669375 | 0.75 | combiAbMir |
| hsa-miR-150, hsa-miR-1224-5p, hsa-miR-342-3p, hsa-miR-3648 | 0.78 | 1.40 | 0.75 | 0.65 | microarray |
| TPM1, STUB1, hsa-miR-1224-5p, hsa-miR-574-5p | 0.782188 | 1.43125 | 0.68125 | 0.75 | combiAbMir |
| HSPD1, RAN, hsa-miR-1224-5p, hsa-miR-342-3p | 0.782188 | 1.4275 | 0.5525 | 0.875 | combiAbMir |
| TPM1, hsa-miR-150, hsa-miR-122, hsa-miR-342-3p | 0.782188 | 1.4075 | 0.6575 | 0.75 | combiAbMir |
| HSPD1, RAN, hsa-miR-122, hsa-miR-342-3p | 0.782188 | 1.405625 | 0.530625 | 0.875 | combiAbMir |
| TPM1, RAN, hsa-miR-122, hsa-miR-342-3p | 0.781875 | 1.418125 | 0.543125 | 0.875 | combiAbMir |
| RAN, STUB1, hsa-miR-1224-5p, hsa-miR-574-5p | 0.781719 | 1.413125 | 0.663125 | 0.75 | combiAbMir |
| HSPD1, RAN, hsa-miR-150, hsa-miR-3648 | 0.781719 | 1.396875 | 0.771875 | 0.625 | combiAbMir |
| TPM1, hsa-miR-1224-5p, hsa-miR-342-3p, hsa-miR-3648 | 0.780938 | 1.39625 | 0.77125 | 0.625 | combiAbMir |
| RAN, STUB1, hsa-miR-1224-5p, hsa-miR-342-3p | 0.780469 | 1.396875 | 0.646875 | 0.75 | combiAbMir |
| TPM1, RAN, STUB1, hsa-miR-342-3p | 0.780313 | 1.408125 | 0.658125 | 0.75 | combiAbMir |
| RAN, hsa-miR-122, hsa-miR-342-3p, hsa-miR-3648 | 0.779063 | 1.37625 | 0.75125 | 0.625 | combiAbMir |
| HSPD1, STUB1, hsa-miR-150, hsa-miR-1224-5p | 0.778125 | 1.40125 | 0.65125 | 0.75 | combiAbMir |
| TPM1, hsa-miR-122, hsa-miR-1224-5p, hsa-miR-342-3p | 0.777969 | 1.409375 | 0.659375 | 0.75 | combiAbMir |
| HSPD1, RAN, hsa-miR-150, hsa-miR-122 | 0.777188 | 1.395 | 0.77 | 0.625 | combiAbMir |
| HSPD1, hsa-miR-150, hsa-miR-1224-5p, hsa-miR-342-3p | 0.776875 | 1.395625 | 0.645625 | 0.75 | combiAbMir |
| TPM1, hsa-miR-150, hsa-miR-1224-5p, hsa-miR-3648 | 0.773281 | 1.415625 | 0.790625 | 0.625 | combiAbMir |
| RAN, hsa-miR-122, hsa-miR-1224-5p, hsa-miR-342-3p | 0.772344 | 1.40625 | 0.53125 | 0.875 | combiAbMir |
| HSPD1, RAN, STUB1, hsa-miR-150 | 0.772344 | 1.384375 | 0.634375 | 0.75 | combiAbMir |
| HSPD1, STUB1, hsa-miR-122, hsa-miR-342-3p | 0.772188 | 1.381875 | 0.506875 | 0.875 | combiAbMir |
| TPM1, STUB1, hsa-miR-150, hsa-miR-122 | 0.770469 | 1.426875 | 0.801875 | 0.625 | combiAbMir |
| HSPD1, hsa-miR-122, hsa-miR-342-3p, hsa-miR-3648 | 0.770156 | 1.370625 | 0.620625 | 0.75 | combiAbMir |
| RAN, hsa-miR-1224-5p, hsa-miR-342-3p, hsa-miR-3648 | 0.769688 | 1.38125 | 0.75625 | 0.625 | combiAbMir |
| RAN, hsa-miR-150, hsa-miR-1224-5p, hsa-miR-3648 | 0.769063 | 1.37375 | 0.74875 | 0.625 | combiAbMir |
| hsa-miR-150, hsa-miR-122, hsa-miR-1224-5p, hsa-miR-342-3p | 0.77 | 1.38 | 0.62 | 0.76 | microarray |
| TPM1, hsa-miR-150, hsa-miR-122, hsa-miR-1224-5p | 0.76875 | 1.40625 | 0.78125 | 0.625 | combiAbMir |
| HSPD1, STUB1, hsa-miR-1224-5p, hsa-miR-342-3p | 0.768125 | 1.398125 | 0.523125 | 0.875 | combiAbMir |
| RAN, STUB1, hsa-miR-122, hsa-miR-342-3p | 0.767969 | 1.37875 | 0.62875 | 0.75 | combiAbMir |
| HSPD1, hsa-miR-150, hsa-miR-122, hsa-miR-3648 | 0.767344 | 1.37625 | 0.75125 | 0.625 | combiAbMir |
| HSPD1, STUB1, hsa-miR-1224-5p, hsa-miR-574-5p | 0.766094 | 1.378125 | 0.628125 | 0.75 | combiAbMir |
| TPM1, RAN, hsa-miR-150, hsa-miR-122 | 0.765938 | 1.405 | 0.78 | 0.625 | combiAbMir |
| TPM1, STUB1, hsa-miR-150, hsa-miR-1224-5p | 0.764219 | 1.415 | 0.79 | 0.625 | combiAbMir |
| HSPD1, hsa-miR-122, hsa-miR-1224-5p, hsa-miR-342-3p | 0.763594 | 1.37375 | 0.49875 | 0.875 | combiAbMir |
| RAN, STUB1, hsa-miR-150, hsa-miR-1224-5p | 0.763281 | 1.385 | 0.76 | 0.625 | combiAbMir |
| HSPD1, hsa-miR-150, hsa-miR-1224-5p, hsa-miR-3648 | 0.758438 | 1.37 | 0.745 | 0.625 | combiAbMir |
| RAN, STUB1, hsa-miR-150, hsa-miR-342-3p | 0.757813 | 1.376875 | 0.501875 | 0.875 | combiAbMir |
| STUB1, hsa-miR-122, hsa-miR-1224-5p, hsa-miR-342-3p | 0.757344 | 1.351875 | 0.601875 | 0.75 | combiAbMir |
| RAN, STUB1, hsa-miR-150, hsa-miR-3648 | 0.755781 | 1.373125 | 0.748125 | 0.625 | combiAbMir |
| hsa-miR-122, hsa-miR-1224-5p, hsa-miR-342-3p, hsa-miR-3648 | 0.76 | 1.36 | 0.66 | 0.71 | microarray |
| TPM1, STUB1, hsa-miR-150, hsa-miR-3648 | 0.755469 | 1.3925 | 0.7675 | 0.625 | combiAbMir |
| RAN, hsa-miR-150, hsa-miR-122, hsa-miR-1224-5p | 0.753594 | 1.35125 | 0.72625 | 0.625 | combiAbMir |
| TPM1, hsa-miR-150, hsa-miR-122, hsa-miR-3648 | 0.753438 | 1.37625 | 0.75125 | 0.625 | combiAbMir |
| RAN, STUB1, hsa-miR-150, hsa-miR-122 | 0.7525 | 1.355 | 0.73 | 0.625 | combiAbMir |
| STUB1, hsa-miR-122, hsa-miR-342-3p, hsa-miR-3648 | 0.7525 | 1.34375 | 0.59375 | 0.75 | combiAbMir |
| STUB1, hsa-miR-150, hsa-miR-122, hsa-miR-342-3p | 0.75125 | 1.35375 | 0.60375 | 0.75 | combiAbMir |
| STUB1, hsa-miR-150, hsa-miR-1224-5p, hsa-miR-342-3p | 0.749688 | 1.34625 | 0.47125 | 0.875 | combiAbMir |
| HSPD1, STUB1, hsa-miR-150, hsa-miR-3648 | 0.74875 | 1.3525 | 0.7275 | 0.625 | combiAbMir |
| STUB1, hsa-miR-150, hsa-miR-122, hsa-miR-3648 | 0.74875 | 1.34375 | 0.71875 | 0.625 | combiAbMir |
| STUB1, hsa-miR-1224-5p, hsa-miR-342-3p, hsa-miR-3648 | 0.746406 | 1.344375 | 0.719375 | 0.625 | combiAbMir |
| STUB1, hsa-miR-150, hsa-miR-342-3p, hsa-miR-3648 | 0.742969 | 1.3325 | 0.5825 | 0.75 | combiAbMir |
| RAN, hsa-miR-150, hsa-miR-122, hsa-miR-3648 | 0.740938 | 1.323125 | 0.698125 | 0.625 | combiAbMir |
| hsa-miR-150, hsa-miR-122, hsa-miR-1224-5p, hsa-miR-3648 | 0.74 | 1.32 | 0.68 | 0.65 | microarray |
| STUB1, hsa-miR-150, hsa-miR-122, hsa-miR-1224-5p | 0.736719 | 1.325 | 0.575 | 0.75 | combiAbMir |
| STUB1, hsa-miR-150, hsa-miR-1224-5p, hsa-miR-3648 | 0.730313 | 1.31375 | 0.68875 | 0.625 | combiAbMir |
| hsa-miR-150, hsa-miR-122, hsa-miR-3663-5p, hsa-miR-3194-5p | 0.73 | 1.30 | 0.45 | 0.85 | qPCR |
| hsa-miR-150, hsa-miR-122, hsa-miR-342-3p, hsa-miR-3663-5p | 0.72 | 1.31 | 0.47 | 0.85 | qPCR |
| hsa-miR-150, hsa-miR-342-3p, hsa-miR-3663-5p, hsa-miR-3194-5p | 0.72 | 1.31 | 0.61 | 0.69 | qPCR |
| TPM1, RAN, hsa-miR-122, hsa-miR-3648 | 0.720156 | 1.309375 | 0.809375 | 0.5 | combiAbMir |
| TPM1, HSPD1, RAN, hsa-miR-122 | 0.718438 | 1.318125 | 0.693125 | 0.625 | combiAbMir |
| TPM1, HSPD1, RAN, hsa-miR-3648 | 0.715469 | 1.28125 | 0.78125 | 0.5 | combiAbMir |
| TPM1, RAN, hsa-miR-1224-5p, hsa-miR-3648 | 0.710156 | 1.280625 | 0.780625 | 0.5 | combiAbMir |
| TPM1, hsa-miR-122, hsa-miR-1224-5p, hsa-miR-3648 | 0.709063 | 1.27875 | 0.77875 | 0.5 | combiAbMir |
| hsa-miR-122, hsa-miR-342-3p, hsa-miR-3663-5p, hsa-miR-3194-5p | 0.71 | 1.29 | 0.60 | 0.69 | qPCR |
| TPM1, HSPD1, STUB1, hsa-miR-3648 | 0.704531 | 1.27625 | 0.77625 | 0.5 | combiAbMir |
| TPM1, HSPD1, hsa-miR-122, hsa-miR-3648 | 0.6975 | 1.283125 | 0.658125 | 0.625 | combiAbMir |
| TPM1, HSPD1, STUB1, hsa-miR-122 | 0.696719 | 1.298125 | 0.673125 | 0.625 | combiAbMir |
| HSPD1, RAN, hsa-miR-122, hsa-miR-3648 | 0.692656 | 1.270625 | 0.770625 | 0.5 | combiAbMir |
| TPM1, HSPD1, hsa-miR-1224-5p, hsa-miR-3648 | 0.688281 | 1.25875 | 0.75875 | 0.5 | combiAbMir |
| TPM1, STUB1, hsa-miR-122, hsa-miR-3648 | 0.687656 | 1.269375 | 0.769375 | 0.5 | combiAbMir |
| TPM1, HSPD1, RAN, STUB1 | 0.69 | 1.28 | 0.83 | 0.44 | autoAb |
| TPM1, HSPD1, hsa-miR-122, hsa-miR-1224-5p | 0.684063 | 1.28625 | 0.66125 | 0.625 | combiAbMir |
| HSPD1, RAN, hsa-miR-1224-5p, hsa-miR-3648 | 0.682656 | 1.234375 | 0.734375 | 0.5 | combiAbMir |
| TPM1, HSPD1, STUB1, hsa-miR-1224-5p | 0.682344 | 1.28625 | 0.78625 | 0.5 | combiAbMir |
| TPM1, RAN, STUB1, hsa-miR-3648 | 0.68 | 1.25 | 0.75 | 0.5 | combiAbMir |
| HSPD1, STUB1, hsa-miR-122, hsa-miR-3648 | 0.678125 | 1.249375 | 0.749375 | 0.5 | combiAbMir |
| RAN, STUB1, hsa-miR-122, hsa-miR-3648 | 0.669531 | 1.255 | 0.755 | 0.5 | combiAbMir |
| TPM1, RAN, STUB1, hsa-miR-122 | 0.669219 | 1.273125 | 0.773125 | 0.5 | combiAbMir |
| HSPD1, RAN, STUB1, hsa-miR-3648 | 0.669063 | 1.235 | 0.86 | 0.375 | combiAbMir |
| STUB1, hsa-miR-122, hsa-miR-1224-5p, hsa-miR-3648 | 0.66875 | 1.234375 | 0.734375 | 0.5 | combiAbMir |
| TPM1, HSPD1, RAN, hsa-miR-1224-5p | 0.666094 | 1.2425 | 0.7425 | 0.5 | combiAbMir |
| HSPD1, RAN, STUB1, hsa-miR-122 | 0.663281 | 1.2375 | 0.7375 | 0.5 | combiAbMir |
| TPM1, STUB1, hsa-miR-1224-5p, hsa-miR-3648 | 0.66125 | 1.2 | 0.7 | 0.5 | combiAbMir |
| TPM1, STUB1, hsa-miR-122, hsa-miR-1224-5p | 0.6575 | 1.270625 | 0.770625 | 0.5 | combiAbMir |
| TPM1, RAN, hsa-miR-122, hsa-miR-1224-5p | 0.651406 | 1.221875 | 0.596875 | 0.625 | combiAbMir |
| HSPD1, hsa-miR-122, hsa-miR-1224-5p, hsa-miR-3648 | 0.649219 | 1.1925 | 0.8175 | 0.375 | combiAbMir |
| hsa-miR-150, hsa-miR-122, hsa-miR-342-3p, hsa-miR-3194-5p | 0.65 | 1.25 | 0.41 | 0.85 | qPCR |
| HSPD1, STUB1, hsa-miR-122, hsa-miR-1224-5p | 0.642031 | 1.19375 | 0.56875 | 0.625 | combiAbMir |
| HSPD1, STUB1, hsa-miR-1224-5p, hsa-miR-3648 | 0.63125 | 1.16625 | 0.79125 | 0.375 | combiAbMir |
| HSPD1, RAN, hsa-miR-122, hsa-miR-1224-5p | 0.628438 | 1.1475 | 0.6475 | 0.5 | combiAbMir |
| RAN, STUB1, hsa-miR-1224-5p, hsa-miR-3648 | 0.622656 | 1.210625 | 0.835625 | 0.375 | combiAbMir |
| HSPD1, RAN, STUB1, hsa-miR-1224-5p | 0.618906 | 1.156875 | 0.781875 | 0.375 | combiAbMir |
| RAN, hsa-miR-122, hsa-miR-1224-5p, hsa-miR-3648 | 0.617656 | 1.18 | 0.805 | 0.375 | combiAbMir |
| TPM1, RAN, STUB1, hsa-miR-1224-5p | 0.614531 | 1.1775 | 0.8025 | 0.375 | combiAbMir |
| RAN, STUB1, hsa-miR-122, hsa-miR-1224-5p | 0.603125 | 1.141875 | 0.516875 | 0.625 | combiAbMir |

**Table 14 (5-mers)**

| **Panel** | **AUC** | **S+S (i)** | **Sensitivity (ii)** | **Specificity (iii)** | **Assay (iv)** |
|---|---|---|---|---|---|
| hsa-miR-150, hsa-miR-122, hsa-miR-1224-5p, hsa-miR-342-3p, hsa-miR-574-5p | 0.90 | 1.59 | 0.77 | 0.82 | microarray |
| hsa-miR-150, hsa-miR-122, hsa-miR-342-3p, hsa-miR-3648, hsa-miR-574-5p | 0.89 | 1.57 | 0.80 | 0.76 | microarray |
| hsa-miR-150, hsa-miR-122, hsa-miR-1224-5p, hsa-miR-3648, hsa-miR-574-5p | 0.88 | 1.57 | 0.80 | 0.76 | microarray |
| hsa-miR-122, hsa-miR-1224-5p, hsa-miR-342-3p, hsa-miR-3648, hsa-miR-574-5p | 0.88 | 1.57 | 0.81 | 0.76 | microarray |
| hsa-miR-150, hsa-miR-1224-5p, hsa-miR-342-3p, hsa-miR-3648, hsa-miR-574-5p | 0.88 | 1.56 | 0.80 | 0.76 | microarray |
| hsa-miR-150, hsa-miR-122, hsa-miR-1224-5p, hsa-miR-342-3p, hsa-miR-3648 | 0.77 | 1.38 | 0.73 | 0.65 | microarray |
| hsa-miR-150, hsa-miR-122, hsa-miR-342-3p, hsa-miR-3663-5p, hsa-miR-3194-5p | 0.71 | 1.28 | 0.44 | 0.85 | qPCR |

**Table 15 (6-mers)**

| **Panel** | **AUC** | **S+S (i)** | **Sensitivity (ii)** | **Specificity (iii)** | **Assay (iv)** |
|---|---|---|---|---|---|
| hsa-miR-150, hsa-miR-122, hsa-miR-1224-5p, hsa-miR-342-3p, hsa-miR-3648, hsa-miR-574-5p | 0.89 | 1.58 | 0.81 | 0.76 | microarray |

**Table 16**

| The levels of expression of the following 2 miRNAs are significantly different in ES samples compared to that in OvES, NE and NP samples in Study 2 (area A in Figure 9), as determined in study 2: | |
|---|---|
| **miRNA** | **Expression in ES samples compared to that in OvES, NE and NP samples** |
| ebv-miR-BART2-5p | Up |
| hsa-miR-564 | Down |

**Table 17**

| The levels of expression of the following miRNAs are significantly different in OvES samples compared to that in ES, NE and NP samples in Study 2 (area F in Figure 9), as determined in study 2: | |
|---|---|
| **miRNA** | **Expression in OvES samples compared to that in ES, NE and NP samples** |
| hsa-let-7d-5p/3p | Up |
| hsa-miR-29a | Down |
| hsa-miR-142-3p | Down |
| hsa-miR-197 | Up |
| hsa-miR-215 | Down |
| hsa-miR-219-1-3p | Up |
| hsa-miR-296-3p | Up |
| hsa-miR-320c | Down |
| hsa-miR-337-5p | Down |
| hsa-miR-369-3p | Down |
| hsa-miR-450b-5p | Down |
| hsa-miR-483-5p | Up |
| hsa-miR-507 | Down |
| hsa-miR-517c / hsa-miR-519a | Down |
| hsa-miR-519d | Up |
| hsa-miR-520g | Down |
| hsa-miR-576-5p | Up |
| hsa-miR-577 | Down |
| hsa-miR-604 | Up |
| hsa-miR-610 | Down |
| hsa-miR-619 | Down |
| hsa-miR-1256 | Down |
| hsa-miR-1278 | Down |
| hsa-miR-1297 | Down |
| hcmv-miR-US33-5p | Down |
| hsv1-miR-H1-5p | Up |

**Table 18**

| The level of expression of the following miRNA in OvES and ES samples is significantly different from that in NE and NP samples in Study 2 (area C in Figure 9), as determined in study 2: | |
|---|---|
| **miRNA** | **Expression in OvES and ES samples compared to that in NE and NP samples** |
| hsa-miR-877 | Down |

**Table 19**

| The levels of expression of the following miRNAs in OvES and NE samples are significantly different from that in ES and NP samples in Study 2 (area E in Figure 9), as determined in study 2: | |
|---|---|
| **miRNA** | **Expression in OvES and NE samples compared to that in ES and NP samples** |
| hsa-let-7e-5p/3p | Up |
| hsa-let-7f | Up |
| hsa-let-7g | Up |
| hsa-miR-9 | Up |
| hsa-miR-30b | Up |
| hsa-miR-34a | Up |
| hsa-miR-92b | Up |
| hsa-miR-96-5p/3p | Up |
| hsa-miR-106a-5p/3p | Up |
| hsa-miR-17-5p/3p | Up |
| hsa-miR-106b-5p/3p | Up |
| hsa-miR-127-3p | Up |
| hsa-miR-128 | Up |
| hsa-miR-154 | Up |
| hsa-miR-155 | Up |
| hsa-miR-183-5p/3p | Up |
| hsa-miR-194 | Up |
| hsa-miR-196b-5p/3p | Up |
| hsa-miR-203 | Up |
| hsa-miR-204 | Up |
| hsa-miR-221-5p/3p | Up |
| hsa-miR-223 | Up |
| hsa-miR-376a | Up |
| hsa-miR-376c | Up |
| hsa-miR-377 | Up |
| hsa-miR-379 | Up |
| hsa-miR-423-3p | Up |
| hsa-miR-424-5p/3p | Up |
| hsa-miR-425-5p/3p | Up |
| hsa-miR-454-5p/3p | Up |
| hsa-miR-486-3p | Up |
| hsa-miR-509-3p | Up |
| hsa-miR-514 | Up |
| hsa-miR-542-3p | Up |
| hsa-miR-545-5p/3p | Up |
| hsa-miR-626 | Up |
| hsa-miR-630 | Up |
| hsa-miR-663 | Up |
| hsa-miR-720 | Up |
| hsa-miR-758-5p/3p | Up |
| hsa-miR-876-3p | Up |
| hsa-miR-1185 | Up |
| hsa-miR-1260 | Up |
| hsa-miR-1266-5p/3p | Up |

**Table 20**

| The levels of expression of the following miRNAs are significantly different in OvES, ES and NP samples compared to that in NE samples in Study 2 (area B in Figure 9), as determined in study 2: | |
|---|---|
| **miRNA** | **Expression in OvES, ES and NP compared to that in NE samples** |
| hsa-miR-199a-3p | Up |
| hsa-miR-199b-3p | Up |
| hsa-miR-497 | Up |
| hsa-miR-625-5p/3p | Up |

**Table 21**

| The levels of expression of the following miRNAs are significantly different in OvES, ES and NE samples compared to that in NP samples in Study 2 (area D in Figure 9), as determined in study 2: | |
|---|---|
| **miRNA** | **Expression in OvES, ES and NE samples compared to that in NP samples** |
| hsa-miR-631 | Up |
| hsa-miR-635 | Up |

[1] Strathy et al, Fertil Steril. 1982;38(6):667-672.
[2] Nnoaham et al, Fertil Steril. 2011;96(2):366-373 e368.
[3] Hadfield et al, Hum Reprod. 1996;11(4):878-880.
[4] Endometriosis Research Center. Understanding endometriosis: past present and future. The National women's health information council; 2005.
[5] Nnoaham et al, Human Reproduction. 2010;25(suppl 1):i9-i11.
[6] Augoulea et al, Gynecol Endocrinol. 2009;25(2):75-81.
[7] Missmer SA & Cramer DW. Obstet Gynecol Clin North Am. 2003;30(1):1-19, vii.
[8] Moen MH & Schei B. Acta Obstet Gynecol Scand. 1997;76(6):559-562.
[9] Risk factors for pelvic endometriosis in women with pelvic pain or infertility. Gruppo Italiano per lo Studio dell' endometriosi. Eur J Obstet Gynecol Reprod Biol. 1999;83(2):195-199.
[10] Missmer et al, Fertil Steril. 2004;82(6):1501-1508.
[11] Parazzini et al, J Epidemiol Community Health. 1995;49(1):61-64.
[12] Missmer et al, Obstet Gynecol. 2004; 104(5 Pt 1):965-974.
[13] Moen MH & Magnus P. Acta Obstet Gynecol Scand. 1993;72(7):560-564.
[14] Moen MH. Acta Obstet Gynecol Scand. 1994;73(1):59-62.
[15] Vessey et al, BMJ. 1993;306(6871): 182-184.
[16] Vitonis et al, Epidemiology. 2010;21 (1): 16-23.
[17] Sangi-Haghpeykar H & Poindexter AN, 3rd. Obstet Gynecol. 1995;85(6):983-992.
[18] Koninckx etal, Hum Reprod. 1994;9(6):1001-1002.
[19] Sinaii et al, Hum Reprod. 2002; 17(10):2715-2724.
[20] Matalliotakis et al, J BUON. 2009;14(4):699-701.
[21] Kvaskoff M et al, Int J Epidemiol. 2009;38(4):1143-1153.
[22] Hornstein et al, Hum Reprod. 1997;12(1):143-145.
[23] Signorile et al, J Exp Clin Cancer Res. 2009;28:49.
[24] Sanfilippo et al, Am J Obstet Gynecol. 1986;154(1):39-43.
[25] Simpson et al, Obstet Gynecol Clin North Am. 2003;30(1):21-40, vii.
[26] Cullen TS. Adenomyoma of the Uterus. WB Saunders. Philadelphia; 1908.
[27] Levander G & Normann P. Acta Obstet Gynecol Scand. 1955;34(4):366-398.
[28] Javert CT. Cancer. 1949;2(3):399-410.
[29] Sampson JA. Am J Pathol. 1927;3(2):93-110 143.
[30] Gargett CE. Hum Reprod Update. 2007; 13(1):87-101.
[31] Donnez J et al, Obstet Gynecol Clin North Am. 2003;30(1):83-93, viii.
[32] Garai et al, Front Biosci. 2006;11:595-619.
[33] Noble et al, J Clin Endocrinol Metab. 1996;81(1):174-179.
[34] Taylor RN & Yu J, Torres PB, et al. Reprod Sci. 2009;16(2):140-146.
[35] Oosterlynck et al, Fertil Steril. 1991;56(1):45-51.
[36] Vinatier et al, Eur J Obstet Gynecol Reprod Biol. 2001;96(1):21-34.
[37] Tabibzadeh et al, Hum Reprod. 1996; 11(3):633-640.
[38] Lessey et al, J Clin Endocrinol Metab. 1994;79(2):643-649.
[39] Sharpe-Timms et al, Fertil Steril. 1998;69(6):1128-1134.
[40] McLaren et al, Hum Reprod. 1997;12(6):1307-1310.
[41] Al-Jefout et al, Hum Reprod. 2009;24(12):3019-3024.
[42] Kyama et al, Fertil Steril. 2011;95(4):1338-1343 e1331-1333.
[43] Kyama et al, Fertil Steril. 2006;86(1):203-209.
[44] Fassbender et al, Reprod Biol Endocrinol. 2010;8:123.
[45] Fassbender et al, Obstet Gynecol. 2012; 119(2 Pt 1):276-285.
[46] Jing J, et al, Fertil Steril. 2009;92(4):1221-1227.
[47] Liu et al, Fertil Steril. 2007;87(4):988-990.
[48] Seeber et al, Fertil Steril. 2010;93(7):2137-2144.
[49] Wolfler et al, Fertil Steril. 2009;91(6):2331-2337.
[50] Zhang et al, Chin Med J (Engl). 2009;122(4):373-376.
[51] Pappworth et al. (2009) Mol Immunol 46:1042-9.
[52] Guerra et al. (2012) Arthritis Res Ther. 29;14(3):21
[53] Vanderlugt&Miller (1996) CurrOpinlmmunol. 8:831-6.
[54] Cheung et al. (2000) Nucleic Acids Res. 28(1):361-3. http://alfred.med.yale.edu/alfred/
[55] McKusick (1998) Mendelian Inheritance in Man. A Catalog of Human Genes and Genetic Disorders. Baltimore: Johns Hopkins University Press, 1998 (12th edition). See also http://www.ncbi.nlm.nih.gov/omim/*.*
[56] Stenson et al. (2009) Genome Med 1:13.
[57] Stamm et al. (2006) Nucleic Acids Res 34: D46-D55.
[58] Griffiths-Jones (2004) Nucleic Acid Res 32(Database Issue):D109-11.
[59] Griffiths-Jones et al. (2006) Nucleic Acid Res 34 (Database Issue):D140-4.
[60] Griffiths-Jones et al. (2008) Nucleic Acid Res 36 (Database Issue):D154-8.
[61] Kozomara & Griffiths-Jones (2011) Nucleic Acid Res 39 (Database Issue):D152-7. http://www.mirbase.org/
[62] Maragkakis et al. (2009) Nucleic Acid Res 37 (Web Server Issue):W273-6.
[63] Maragkakis et al. (2011) Nucleic Acid Res 39 (Web Server Issue):W145-8. http://diana.cslab.ece.ntua.gr/DianaTools/index.php?r=microtv4/index
[64] Betel et al. (2008) Nucleic Acid Res 36 (Database Issue):D149-53. http://www.microrna.org/microrna/home.do
[65] Wang & El Naqa (2008) Bioinformatics 24:325-32.
[66] Wang (2008) RNA 14:1012-7. http://mirdb.org/miRDB/index.html
[67] Grimson et al. (2007) Mol Cell 27:91-105. http://www.targetscan.org/vert_50/
[68] Krek et al. (2005) Nat Genet 37:495-500. http://pictar.mdc-berlin.de/cgi-bin/PicTar_vertebrate.cgi
[69] Geysen etal. (1984) PNAS USA 81:3998-4002.
[70] Carter (1994) Methods Mol Biol 36:207-23.
[71] Jameson, BA ef al. 1988, CABIOS 4(1):181-186.
[72] Maksyutov & Zagrebelnaya (1993) Comput Appl Biosci 9(3):291-7.
[73] Hopp (1993) Peptide Research 6:183-190.
[74] Welling et al. (1985) FEBS Lett. 188:215-218.
[75] Bublil et al. (2007) Proteins 68(1):294-304.
[76] Sun et al. (2009) Nucleic Acids Res 37:W612-6.
[77] Raddrizzani & Hammer (2000) Brief Bioinform 1(2):179-89.
[78] Chen et al. (2007) Amino Acids 33(3):423-8.
[79] Reimer (2009) Methods Mol Biol 524:335-44.
[80] Boutell et al. (2004) Proteomics 4:1950-8.
[81] Tassinari et al. (2008) Curr Opin Mol Ther 10:107-15.
[82] Stoevesandt et al. (2009) Expert Rev Proteomics 6:145-57.
[83] Tao et al. (2007) Comb Chem High Throughput Screen 10:706-18.
[84] Gnjatic et al. (2009) J Immunol Methods 341:50-8.
[85] Hartmann et al. (2009) Anal Bioanal Chem 393:1407-16.
[86] Fall & Niessner (2009) Methods Mol Biol 509:107-22.
[87] WO01/57198.
[88] WO02/27327.
[89] Blackburn & Hart (2005) Methods Mol Biol. 310:197-216
[90] WO03/064656.
[91] WO2004/046730.
[92] Stahl et al. (2006) Immunol Lett 102:50-9.
[93] Quintana (2008) PNAS USA 105:18889-94.
[94] Koopmann & Blackburn (2003) Rapid Commun Mass Spectrom. 17:455-62.
[95] WO01/61040.
[96] Oleinikov et al. (2003) J Proteome Res. 2:313-9.
[97] Bolstad et al. (2003) Bioinformatics 19:185-93.
[98] Meyer et al. (2003) Neurocomputing 55:169-86.
[99] Koza (1992), Genetic Programming: On the Programming of Computers by Means of Natural Selection, MIT Press.
[100] Wang & Japkowicz (2008) Lecture Notes in Computer Science 4994/2008, 38-47.
[101] Elkon & Casali (2008) Nat Clin Pract Rheumatol. 4(9):491-8.
[102] Dudoit, S, et al. (2002) Statistica Sinica 12, 111-139
[103] Yang, YH, et al. Normalization for Two-color cDNA Microarray Data. Science and Statistics: A Festschrift for Terry Speed, Monograph Series.
[104] Yang, YH et al. in Normalization for cDNA microarray data: a robust composite method addressing single and multiple slide systematic variation.
[105] Chada et al. (2003) CurrOpin Drug DiscovDevel. 6(2):169-73.
[106] Chène(2003) Nature Reviews Cancer 3, 102-109.
[107] Wang &EI-Deiry (2008) CurrOpinOncol. 20(1):90-6.
[108] Gleave & Monia (2005) Nat Rev Cancer 5:468-79.
[109] Current Protocols in Molecular Biology (F.M. Ausubel et al., eds., 1987) Supplement 30
[110] Smith & Waterman (1981) Adv. Appl. Math.2: 482-489.
[111] Boutell et al. (2004) Proteomics 4:1950-8.
[112] Gnjatic et al. (2009) J Immunol Methods 341:50-8.
[113] Bolstad et al. (2003) Bioinformatics 19:185-93.
[114] Huber et al. (2002) Bioinformatics 18 suppl. 1 S96-S104.
[115] Pradervand et al. (2010) Biotechniques 48:219-22.
[116] WO93/22480.
[117] WO03/020415.
[118] WO2005/037425

## Claims

1. A method for providing a diagnostic indicator of whether a subject has endometriosis, comprising a step of determining the level of x different biomarkers in a sample from the subject, wherein *x* is 3 or more and wherein the *x* different biomarkers are hsa-miR-150 and ebv-miR-BART2-5p and one or more other biomarkers listed in Table 1.

2. The method of claim 1, wherein the one or more other biomarkers are selected from the group consisting of hsa-let-7f, hsa-let-7g, hsa-miR-1260, hsa-miR142-3p, hsa-miR-197, hsa-miR-215, hsa-miR-223, hsa-miR-30b, hsa-miR-320c, hsa-miR34a, hsa-miR-497, hsa-miR-630, hsa-miR-663 and hsa-miR-720.

3. The method of claim 1 or claim 2, wherein *x* is 5 or more.

4. The method of claim 1 or claim 2, wherein *x* is 10 or more.

5. The method of any preceding claim, wherein the method also includes a step of determining if a sample from the subject contains autoantibodies against CA125 and/or CA19-9.

6. The method of any preceding claim, wherein the method involves comparing levels of the biomarkers in the subject sample to levels in (i) a sample from a patient with endometriosis and/or (ii) a sample from a patient without endometriosis.

7. The method of any preceding claim, wherein the method involves analysing levels of the biomarkers in the sample with a classifier algorithm which uses the measured levels of to distinguish between patients with endometriosis and patients without endometriosis.

8. The method of any preceding claim, wherein the subject is (i) pre-symptomatic for endometriosis or (ii) already displaying clinical symptoms of endometriosis.

9. The method of any preceding claim, wherein the sample is a body fluid.

10. The method of claim 9, wherein the sample is cervical discharge or peritoneal fluid.

11. The method of any preceding claim, wherein the presence of antibodies is determined using an immunoassay.

12. The method of claim 9, wherein the immunoassay utilises an antigen comprising an amino acid sequence (i) having at least 90% sequence identity to an amino acid sequence disclosed in Table 1, and/or (ii) comprising at least one epitope from an amino acid sequence disclosed in Table 1.

13. The method of claim 11 or claim 12, wherein the immunoassay utilises a fusion polypeptide with a first region and a second region, wherein the first region can react with an auto-antibody in a sample and the second region can react with a substrate to immobilise the fusion polypeptide thereon.

14. The method of any preceding claim, wherein the subject sample is a human.

## Patentansprüche

1. Verfahren zur Bereitstellung eines diagnostischen Indikators dafür, ob bei einem Individuum Endometriose vorliegt, umfassend einen Schritt, bei dem der Spiegel von x unterschiedlichen Biomarkern in einer Probe von dem Individuum bestimmt wird, wobei x gleich 3 oder größer ist und wobei es sich bei den x unterschiedlichen Biomarkern um hsa-miR-150 und ebv-miR-BART2-5p und einen oder mehrere andere, in Tabelle 1 aufgeführte Biomarker handelt.

2. Verfahren nach Anspruch 1, wobei die ein oder mehreren anderen Biomarker ausgewählt sind aus der Gruppe bestehend aus hsa-let-7f, hsa-let-7g, hsa-miR-1260, hsa-miR142-3p, hsa-miR-197, hsa-miR-215, hsa-miR-223, hsa-miR-30b, hsa-miR-320c, hsa-miR34a, hsa-miR-497, hsa-miR-630, hsa-miR-663 und hsa-miR-720.

3. Verfahren nach Anspruch 1 oder Anspruch 2, wobei x gleich 5 oder größer ist.

4. Verfahren nach Anspruch 1 oder Anspruch 2, wobei x gleich 10 oder größer ist.

5. Verfahren nach einem vorhergehenden Anspruch, wobei das Verfahren auch einen Schritt enthält, bei dem bestimmt wird, ob eine Probe von dem Individuum Autoantikörper gegen CA125 und/oder CA19-9 enthält.

6. Verfahren nach einem vorhergehenden Anspruch, wobei das Verfahren Vergleichen von Spiegeln der Biomarker beim Probenindividuum mit Spiegeln in (i) einer Probe von einem Patienten mit Endometriose und/oder (ii) einer Probe von einem Patienten ohne Endometriose beinhaltet.

7. Verfahren nach einem vorhergehenden Anspruch, wobei das Verfahren Analysieren von Spiegeln der Biomarker in der Probe mit einem Klassifikator-Algorithmus beinhaltet, bei dem die gemessenen Spiegel von zur Unterscheidung zwischen Patienten mit Endometriose und Patienten ohne Endometriose verwendet werden.

8. Verfahren nach einem vorhergehenden Anspruch, wobei das Individuum (i) präsymptomatisch für Endometriose ist oder (ii) bereits klinische Symptome von Endometriose präsentiert.

9. Verfahren nach einem vorhergehenden Anspruch, wobei es sich bei der Probe um eine Körperflüssigkeit handelt.

10. Verfahren nach Anspruch 9, wobei es sich bei der Probe um Zervixausfluss oder Peritonealflüssigkeit handelt.

11. Verfahren nach einem vorhergehenden Anspruch, wobei das Vorliegen von Antikörpern unter Verwendung eines Immuntests bestimmt wird.

12. Verfahren nach Anspruch 9, wobei beim Immuntest ein Antigen benutzt wird, das eine Aminosäuresequenz umfasst, die (i) wenigstens 90% Sequenzidentität mit einer in Tabelle 1 dargestellten Aminosäuresequenz aufweist und/oder (ii) wenigstens ein Epitop aus einer in Tabelle 1 dargestellten Aminosäuresequenz umfasst.

13. Verfahren nach Anspruch 11 oder Anspruch 12, wobei beim Immuntest ein Fusionspolypeptid mit einer ersten Region und einer zweiten Region benutzt wird, wobei die erste Region mit einem Autoantikörper in einer Probe reagieren kann und die zweite Region mit einem Substrat zur Immobilisierung des Fusionspolypeptids daran reagieren kann.

14. Verfahren nach einem vorhergehenden Anspruch, wobei es sich bei dem Probenindividuum um einen Menschen handelt.

## Revendications

1. Procédé de fourniture d'un indicateur diagnostique du fait qu'un sujet a ou non une endométriose, comprenant une étape de détermination du taux de x biomarqueurs différents dans un échantillon provenant du sujet, dans lequel x est 3 ou plus et dans lequel les x biomarqueurs différents sont hsa-miR-150 et ebv-miR-BART2-5p et un ou plusieurs autres biomarqueurs répertoriés dans le tableau 1.

2. Procédé selon la revendication 1, dans lequel les un ou plusieurs autres biomarqueurs sont choisis dans le groupe constitué de hsa-let-7f, hsa-let-7g, hsa-miR-1260, hsa-miR142-3p, hsa-miR-197, hsa-miR-215, hsa-miR-223, hsa-miR-30b, hsa-miR-320c, hsa-miR34a, hsa-miR-497, hsa-miR-630, hsa-miR-663 et hsa-miR-720.

3. Procédé selon la revendication 1 ou la revendication 2, dans lequel x est 5 ou plus.

4. Procédé selon la revendication 1 ou la revendication 2, dans lequel x est 10 ou plus.

5. Procédé selon l'une quelconque des revendications précédentes, le procédé comprenant en outre une étape de détermination du fait qu'un échantillon provenant du sujet contient des autoanticorps contre CA125 et/ou CA19-9.

6. Procédé selon l'une quelconque des revendications précédentes, le procédé mettant en œuvre la comparaison des taux des biomarqueurs dans l'échantillon du sujet aux taux dans (i) un échantillon provenant d'un patient avec endométriose et/ou (ii) un échantillon provenant d'un patient sans endométriose.

7. Procédé selon l'une quelconque des revendications précédentes, le procédé mettant en œuvre l'analyse des taux des biomarqueurs dans l'échantillon avec un algorithme de classificateur qui utilise les taux mesurés afin de distinguer les patients avec endométriose et les patients sans endométriose.

8. Procédé selon l'une quelconque des revendications précédentes, dans lequel le sujet est (i) présymptomatique pour l'endométriose ou (ii) présente déjà des symptômes d'endométriose.

9. Procédé selon l'une quelconque des revendications précédentes, dans lequel l'échantillon est un fluide corporel.

10. Procédé selon la revendication 9, dans lequel l'échantillon est un écoulement cervical ou un fluide péritonéal.

11. Procédé selon l'une quelconque des revendications précédentes, dans lequel la présence d'anticorps est déterminée au moyen d'un immunoessai.

12. Procédé selon la revendication 9, dans lequel l'immunoessai utilise un antigène comprenant une séquence d'acides aminés (i) ayant au moins 90 % d'identité de séquence avec une séquence d'acides aminés décrite dans le tableau 1, et/ou (ii) comprenant au moins un épitope d'une séquence d'acides aminés décrite dans le tableau 1.

13. Procédé selon la revendication 11 ou la revendication 12, dans lequel l'immunoessai utilise un polypeptide de fusion avec une première région et une deuxième région, dans lequel la première région peut réagir avec un autoanticorps dans un échantillon et la deuxième région peut réagir avec un substrat pour immobiliser le polypeptide de fusion sur celui-ci.

14. Procédé selon l'une quelconque des revendications précédentes, dans lequel l'échantillon du sujet est d'un humain.
